(19) Europäisches Patentamt
European Patent Office
Office européen des brevets

(11) EP 3 560 494 A1

(12) # EUROPEAN PATENT APPLICATION
published in accordance with Art. 153(4) EPC

(43) Date of publication:
**30.10.2019 Bulletin 2019/44**

(21) Application number: **17884516.0**

(22) Date of filing: **20.12.2017**

(51) Int Cl.:
***A61K 31/4164*** *(2006.01)* ***A61K 31/4178*** *(2006.01)*
***A61K 31/422*** *(2006.01)* ***A61K 31/427*** *(2006.01)*
***A61K 31/437*** *(2006.01)* ***A61K 31/4375*** *(2006.01)*
***A61K 31/4439*** *(2006.01)* ***A61K 31/4709*** *(2006.01)*
***A61K 31/4725*** *(2006.01)* ***A61K 31/497*** *(2006.01)*
***A61K 31/5365*** *(2006.01)* ***A61K 45/00*** *(2006.01)*
***A61P 35/00*** *(2006.01)*

(86) International application number:
**PCT/JP2017/045836**

(87) International publication number:
**WO 2018/117196 (28.06.2018 Gazette 2018/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **20.12.2016 US 201662436671 P**

(71) Applicant: **Sumitomo Dainippon Pharma Co., Ltd.**
**Osaka-shi**
**Osaka 541-8524 (JP)**

(72) Inventors:
- **FUKUNAGA, Yuichi**
**Osaka-shi**
**Osaka 554-0022 (JP)**
- **YAMAKAWA, Erina**
**Osaka-shi**
**Osaka 554-0022 (JP)**
- **SUGARU, Eiji**
**Osaka-shi**
**Osaka 554-0022 (JP)**
- **IKEDA, Satoshi**
**Osaka-shi**
**Osaka 554-0022 (JP)**
- **OTSUBO, Tsuguteru**
**Osaka-shi**
**Osaka 554-0022 (JP)**
- **UMEHARA, Hiroki**
**Osaka-shi**
**Osaka 554-0022 (JP)**
- **LI, Chiang Jia**
**Cambridge, Massachusetts 02141 (US)**

(74) Representative: **Vossius & Partner**
**Patentanwälte Rechtsanwälte mbB**
**Siebertstrasse 3**
**81675 München (DE)**

(54) **DRUG TARGETING CANCER STEM CELL**

(57) The present invention provides an anti-tumor agent comprising a compound of formula (1) or a pharmaceutically acceptable salt thereof, wherein ring $Q^1$ is optionally-substituted $C_{6-10}$ aryl, etc.; $R^1$ and $R^2$ are independently hydrogen atom, etc.; $W^1$ is $C_{1-4}$ alkylene which may be optionally substituted with 1 to 3 fluorine atoms or $C_{3-7}$ cycloalkyl; $W^2$ is $-NR^{4a}C(O)-$, etc. wherein $R^{4a}$ is hydrogen atom or $C_{1-6}$ alkyl; ring $Q^2$ is optionally-substituted $C_{6-10}$ aryl, etc., in combination with at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof.

EP 3 560 494 A1

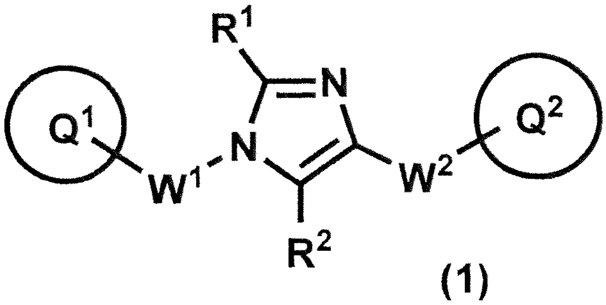

(1)

## Description

### TECHNICAL FIELD

The present invention relates to a pharmaceutical composition for treating or preventing cancer in which an agent whose target is cancer stem cells and various types of agents used for the treatment or prophylaxis of a disease such as cancer are used in combination.

### BACKGROUND ART

Conventional cancer treatments are sometimes not expected to bring in meaningful survival effects even if they can induce the regression of tumors, because of the persistent proliferation of malignant tumors, the metastasis or recurrence of cancer, and the resistance to anti-tumor agents. These days, it has been suggested that cancer stem cell (hereinafter also referred to as "CSC", as necessary) is one of the causes of the failure, which is closely involved in the factors such as the persistent proliferation of malignant tumor. CSCs have been identified in almost all types of major cancers in human such as breast cancer, colon cancer, lung cancer, and hematological malignancy (Non-Patent Document 1). Also, CSCs can be greatly different in the biological feature from normal cancer cells which differentiate from CSCs, and thus the development of an anti-tumor agent whose target is CSCs is expected to lead to a new strategy for cancer treatments (Non-Patent Document 2).

One of the features in CSCs is the self-renewal ability (Non-Patent Document 3). Reliable methods established for measuring the self-renewal ability of cells include, for example, a method for measuring the sphere-forming ability of cancer cells in non-adherent condition in the absence of serum (Non-Patent Document 4).

Other feature in CSCs is to show resistance to the existing anti-cancer agents. The combination of an agent whose target is CSCs and an existing anti-cancer agent is expected to produce a potent anti-cancer effect (Non-Patent Document 5).

Non-Patent Document 6 discloses that the combination of PF-03084014 having an N-imidazolylamide scaffold and docetaxel used as anti-cancer agent produces a potent anti-cancer effect.

Non-Patent Documents 7 and 8 disclose compounds such as 4-aminoimidazole derivative useful as anti-obesity agent.

However, it has not been reported that the compound of formula (1) of the present invention exhibits an anti-cancer effect, and enhances the anti-cancer effect in the combination with an existing agent.

### PRIOR ART DOCUMENTS

### NON-PATENT DOCUMENTS

Non-Patent Document 1: Boman et al., Journal of Clinical Oncology 26(17): 2795-2799. 2008
Non-Patent Document 2: Lobo et al., Annu Rev Cell Dev Biol 23:675-99. 2007
Non-Patent Document 3: Al-Hajj et al., Oncogene 23(43):7274-82. 2004
Non-Patent Document 4: Ponti et al., Cancer Res 65(13):5506-11. 2005
Non-Patent Document 5: Carmero et al. Cancer Treatment reviews 49:25-36. 2016
Non-Patent Document 6: Zhang et al., Stem Cells Translational Medicine 2:233-242. 2013
Non-Patent Document 7: The 27th medicinal chemistry symposium abstract, p.166-167
Non-Patent Document 8: Monthly Fine Chemicals, August 2009, Vol.38, No.8, p.12-24

### SUMMARY OF INVENTION

### (PROBLEM TO BE SOLVED BY THE INVENTION)

An object of the present invention is to provide a pharmaceutical composition for inhibiting the self-renewal ability of cancer stem cells which is important for the persistent proliferation of malignant tumors, the metastasis or recurrence of cancer, and the resistance to anti-tumor agents to produce a potent anti-cancer effect.

### (MEANS FOR SOLVING THE PROBLEMS)

The present inventors have extensively studied to reach the above object, and then have found that a compound

of the following formula (1) or a pharmaceutically acceptable salt thereof (hereinafter also referred to as "the present compound", as necessary) has an inhibitory effect on the sphere-forming ability of cancer cells (hereinafter also referred to as "the sphere-forming ability", as necessary). In addition, the present inventors have found that a combination of a compound of formula (1) or a pharmaceutically acceptable salt thereof and other agent achieves the enhanced inhibitory effect on the sphere-forming ability and enhances the anti-cancer effect of the compound of formula (1) in animal models. Based upon the new findings, the present invention has been completed.

The present invention provides inventions described below.

[1] An anti-tumor agent comprising a compound of formula (1) :

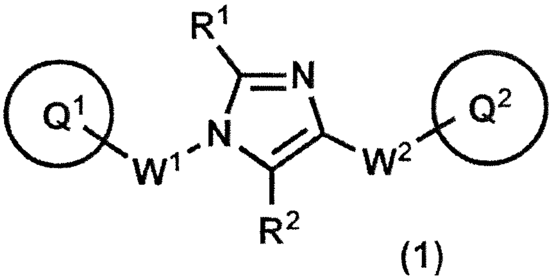

(1)

or a pharmaceutically acceptable salt thereof, wherein $Q^1$ is optionally-substituted $C_{6-10}$ aryl, optionally-substituted $C_{6-10}$ aryloxy, optionally-substituted $C_{6-10}$ arylthio, optionally-substituted $C_{3-10}$ cycloalkyl, or optionally-substituted 5- to 10-membered heteroaryl;

$R^1$ and $R^2$ are independently hydrogen atom, halogen atom, or $C_{1-6}$ alkyl which may be optionally substituted with the same or different 1 to 3 halogen atoms;

$W^1$ is $C_{1-4}$ alkylene which may be optionally substituted with 1 to 3 fluorine atoms or $C_{3-7}$ cycloalkyl;

$W^2-Q^2$ is $-NR^{3a}C(O)-Q^2$, $-NR^{3a}C(O)O-Q^2$, $-NR^{3a}C(O)OCH_2-Q^2$, - $NR^{3a}C(O)NR^{3b}-Q^2$, $-NR^{3a}C(O)NR^{3b}CH_2-Q^2$, $-NR^{3a}C(O)CH_2O-Q^2$, - $NR^{3a}C$ $(O)CH_2-Q^2$, $-NR^{3a}C(O)CH_2CH_2-Q^2$, $-C(O)NR^{3a}-Q^{-2}$, $-C(O)NR^{3a}CH_2-Q^2$, $-C(O)NR^{3a}CH_2CH_2Q^2$, or $-NR^{3a}C(O)-CR^{4c}=CR^{4d}-Q^2$ wherein $R^{3a}$ and $R^{3b}$ are independently hydrogen atom or $C_{1-6}$ alkyl; $R^{3c}$ and $R^{3d}$ are independently hydrogen atom, fluorine atom, or $C_{1-6}$ alkyl;

ring $Q^2$ is optionally-substituted $C_{6-10}$ aryl or optionally-substituted 5- to 10-membered heteroaryl, in combination with at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof.

[2] The anti-tumor agent according to [1], wherein $Q^1$ is phenyl which may be optionally substituted with the same or different 1 to 4 groups selected from the group consisting of halogen atom, and $C_{1-6}$ alkyl which may be optionally substituted with the same or different 1 to 3 halogen atoms;

$W^1$ is methylene;

$W^2-Q^2$ is $-NHC(O)-Q^2$, $-NHC(O)-CH=CH-Q^2$, $-C(O)NH-Q^2$, or - $NHC(O)CH_2O-Q^2$;

$R^1$ and $R^2$ are hydrogen atom; and

ring $Q^2$ is

(1) phenyl which may be optionally substituted with the same or different 1 to 4 groups selected from the group consisting of

(a) halogen atom,

(b) $C_{1-6}$ alkyl which may be optionally substituted with the same or different 1 to 3 groups selected from the group consisting of halogen atom, hydroxy, and $C_{1-6}$ alkoxy,

(c) $C_{1-6}$ alkoxy which may be optionally substituted with the same or different 1 to 3 groups selected from the group consisting of halogen atom, hydroxy, and $C_{1-6}$ alkoxy,

(d) $C_{3-7}$ cycloalkyl,

(e) $C_{2-6}$ alkenyl,

(f) cyano,

(g) amino which may be optionally substituted with the same or different 1 to 2 $C_{1-6}$ alkyl groups, and

(h) $C_{1-6}$ alkyl-carbonylamino,

(2) 5- or 6-membered heteroaryl which may be optionally substituted with the same or different 1 to 4 groups selected from the group consisting of (a)-(h) defined in the above (1), or

(3) a group of the following formula (11), (12), (13), (14), (15), or (16):

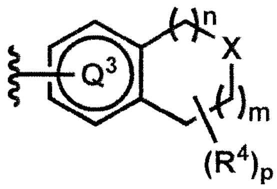
(11)

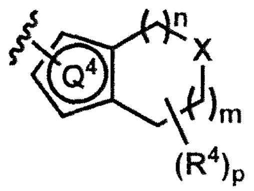
(12)

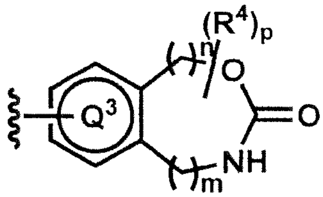
(13)

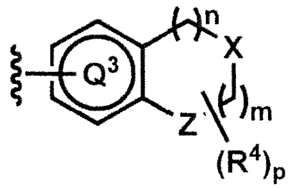
(14)

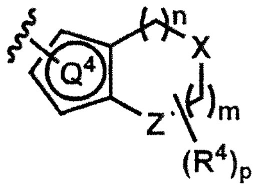
(15)

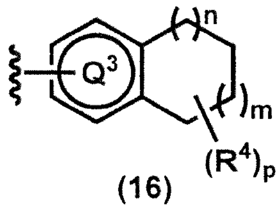
(16)

wherein ring $Q^3$ is optionally-substituted benzene ring, optionally-substituted pyridine ring, optionally-substituted pyrimidine ring, optionally-substituted pyridazine ring, or optionally-substituted pyrazine ring;
ring $Q^4$ is optionally-substituted 5-membered heteroaryl ring;
n and m are independently 0, 1, or 2, provided that n and m are not simultaneously 0;
X and Z are independently $NR^5$, $-NR^{3e}C(O)-$, $-C(O)NR^{3e}-$, or O wherein $R^5$ is hydrogen atom, $C_{1-6}$ alkyl which may be optionally substituted with the same or different 1 to 3 halogen atoms, or $C_{1-6}$ alkylcarbonyl; $R^{3e}$ is hydrogen atom or $C_{1-6}$ alkyl;
p is 1, 2, 3, 4, or 5;
$R^4$ is, independently when two or more exist, hydrogen atom, halogen atom, hydroxy, oxo, $C_{1-6}$ alkyl which may be optionally substituted with the same or different 1 to 3 halogen atoms, or $C_{1-6}$ alkoxy which may be optionally substituted with the same or different 1 to 3 halogen atoms.

[3] The anti-tumor agent according to [1] or [2], wherein ring $Q^2$ is

(1) phenyl which may be optionally substituted with the same or different 1 to 2 groups selected from the group consisting of $C_{1-6}$ alkoxy which may be optionally substituted with hydroxy, and $C_{1-6}$ alkyl-carbonylamino,
(2) a group of fromula (2):

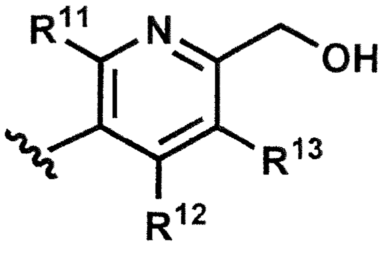
(2)

wherein $R^{11}$, $R^{12}$, and $R^{13}$ are independently

(a) hydrogen atom,
(b) halogen atom,
(c) $C_{1-6}$ alkyl which may be optionally substituted with 1 to 3 fluorine atoms, or
(d) amino which may be optionally substituted with the same or different 1 to 2 $C_{1-6}$ alkyl groups, or

(3) a group of formula (21):

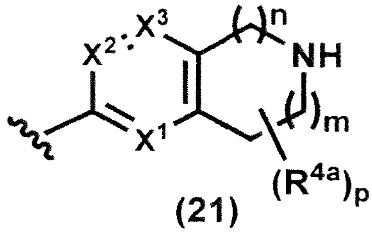

(21)

wherein $X^1$ is N or $CR^{14}$;
$X^2$ is N or $CR^{15}$;
$X^3$ is N or $CR^{16}$;
provided that $X^1$, $X^2$ and $X^3$ are not simultaneously N;
$R^{14}$, $R^{15}$, and $R^{16}$ are independently

(a) hydrogen atom,
(b) halogen atom,
(c) $C_{1-6}$ alkyl which may be optionally substituted with the same or different 1 to 3 halogen atoms, or
(d) $C_{1-6}$ alkoxy which may be optionally substituted with the same or different 1 to 3 halogen atoms;

n and m are independently 0, 1, or 2, provided that n and m are not simultaneously 0;
p is 1, 2, 3, 4, or 5;
$R^{4a}$ is, independently when two or more exist, hydrogen atom, halogen atom, or $C_{1-6}$ alkyl which may be optionally substituted with the same or different 1 to 3 halogen atoms.
[4] The anti-tumor agent according to [3], wherein $R^{11}$ and $R^{12}$ are hydrogen atom;
$R^{13}$ is hydrogen atom, $C_{1-4}$ alkyl which may be optionally substituted with 1 to 3 fluorine atoms, or amino;
$R^{14}$, $R^{15}$, and $R^{16}$ are independently hydrogen atom or fluorine atom;
n is 1;
m is 0 or 1;
p is 1 or 2;
$R^{4a}$ is, independently when two or more exist, hydrogen atom or methyl.
[5] The anti-tumor agent according to [3] or [4], wherein $W^2$-$Q^2$ is -NHC(O)-$Q^2$, or -C(O)NH-$Q^2$; and ring $Q^2$ is a group of formula (2) or (21).
[6] The anti-tumor agent according to any one of [3]-[5], wherein $W^2$-$Q^2$ is -NHC(O)-$Q^2$; and ring $Q^2$ is a group of formula (2).
[7] The anti-tumor agent according to [1], wherein the compound of formula (1) or a pharmaceutically acceptable salt is selected from the following compounds:

(2E)-3-[4-(acetylamino)phenyl]-N-{1-[3-(trifluoromethyl)benzyl]-1H-imidazol-4-yl}prop-2-enamide (Example 1-1),
(2E)-*N*-(1-(3-chlorobenzyl)-1H-imidazol-4-yl)-3-(pyridin-3-yl)prop-2-enamide (Example 9-1),
N-[1-(3-chlorobenzyl)-1H-imidazol-4-yl]-3,4 - dimethoxybenzamide (Example 10-1),
N-[1-(3,4-difluorobenzyl)-1H-imidazol-4-yl]-3,4-dimethoxybenzamide (Example 11-1),
N-[1-(2,4-difluorobenzyl)-1H-imidazol-4-yl]-3,4-dimethoxybenzamide (Example 12-1),
3,4-dimethoxy-N-[1-(3,4,5-trifluorobenzyl)-1H-imidazol-4-yl]benzamide (Example 13-1),
6-(hydroxymethyl)-N-{1-[3-(trifluoromethyl)benzyl]-1H-imiaazol-4-yl}nicotinamide (Example 22),
5-(difluoromethyl)-6-(hydroxymethyl)-N-{1-[3-(trifluoromethyl)benzyl]-1H-imidazol-4-yl}nicotinamide (Example 23),
5-(difluoromethyl)-6-(hydroxymethyl)-N-[1-(3,4,5-trifluorobenzyl)-1H-imidazol-4-yl]nicotinamide (Example 24),
N-(5,6,7,8-tetrahydro-2,7-naphthyridin-3-yl)-1-(3,4,5-trifluorobenzyl)-1H-imidazole-4-carboxamide (Example 32),
8-fluoro-N-[1-(3,4,5-trifluorobenzyl)-1H-imidazol-4-yl]-1,2,3,4-tetrahydroquinoline-6-carboxamide (Example 34),
N-[1-(3,4,5-trifluorobenzyl)-1H-imidazol-4-yl]-1,2,3,4-tetrahydroquinoline-6-carboxamide (Example 35),
N-{1-[3-(trifluoromethyl)benzyl]-1H-imidazol-4-yl}-5,6,7,8-tetrahydro-1,6-naphthyridine-2-carboxamide (Example 39),
N-[1-(3,4,5-trifluorobenzyl)-1H-imidazol-4-yl]-5,6,7,8-tetrahydro-1,6-naphthyridine-2-carboxamide (Example 40),

N-[1-(3,4,5-trifluorobenzyl)-1H-imidazol-4-yl]-5,6,7,8-tetrahydro-1,7-naphthyridine-3-carboxamide (Example 41),
N-(1-[3-(trifluoromethyl)benzyl]-1H-imidazol-4-yl)-5,6,7,8-tetrahydro-1,7-naphthyridine-3-carboxamide (Example 42),
6-(hydroxymethyl)-5-methyl-N-[1-(3,4,5-trifluorobenzyl)-1H-imidazol-4-yl]nicotinamide (Example 55),
5-amino-6-(hydroxymethyl)-N-{1-[3-(trifluoromethyl)benzyl]-1H-imidazol-4-yl}nicotinamide (Example 58), and
5-amino-6-(hydroxymethyl)-N-[1-(3,4,5-trifluorobenzyl)-1H-imidazol-4-yl]nicotinamide (Example 59).

[8] The anti-tumor agent according to [1], wherein the compound of formula (1) or a pharmaceutically acceptable salt is selected from the following compounds:

(2E)-3-[4-(acetylamino)phenyl]-N-(1-[3-(trifluoromethyl)benzyl]-1H-imidazol-4-yl)prop-2-enamide (Example 1-1),
N-[1-(3-chlorobenzyl)-1H-imidazol-4-yl]-3,4-dimethoxybenzamide (Example 10-1),
3,4-dimethoxy-N-[1-(3,4,5-trifluorobenzyl)-1H-imidazol-4-yl]benzamide (Example 13-1),
5-(difluoromethyl)-6-(hydroxymethyl)-N-[1-(3,4,5-trifluorobenzyl)-1H-imidazol-4-yl]pyridine-3-carboxamide (Example 24), and
N-{1-[3-(trifluoromethyl)benzyl]-1H-imidazol-4-yl}-5,6,7,8-tetrahydro-1,7-naphthyridine-3-carboxamide (Example 42).

[9] The anti-tumor agent according to any one of [1]-[8], wherein the anti-cancer agent is at least one selected from the group consisting of a chemotherapeutic agent, a hormonal therapeutic agent, an angiogenesis inhibitor, an immunotherapeutic agent, a kinase inhibitor, an antibody medicine, a proteasome inhibitor, a HDAC inhibitor, a PARP inhibitor, a thalidomide analog, a retinoic acid analog, and a pharmaceutically acceptable salt thereof.
[10] The anti-tumor agent according to [9], wherein the chemotherapeutic agent is at least one selected from the group consisting of an alkylating agent, an anti-metabolite, an anti-cancer antibiotic, a microtubule inhibitor, a topoisomerase inhibitor, a platinum agent, and a pharmaceutically acceptable salt thereof.
[11] The anti-tumor agent according to [9] or [10], wherein the chemotherapeutic agent is at least one selected from the group consisting of an alkylating agent and a pharmaceutically acceptable salt thereof.
[12] The anti-tumor agent according to [9] or [10], wherein the chemotherapeutic agent is at least one selected from the group consisting of an anti-metabolite and a pharmaceutically acceptable salt thereof.
[13] The anti-tumor agent according to [9] or [10], wherein the chemotherapeutic agent is at least one selected from the group consisting of an anti-cancer antibiotic and a pharmaceutically acceptable salt thereof.
[14] The anti-tumor agent according to [9] or [10], wherein the chemotherapeutic agent is at least one selected from the group consisting of a microtubule inhibitor and a pharmaceutically acceptable salt thereof.
[15] The anti-tumor agent according to [9] or [10], wherein the chemotherapeutic agent is at least one selected from the group consisting of a topoisomerase inhibitor and a pharmaceutically acceptable salt thereof.
[16] The anti-tumor agent according to [9] or [10], wherein the chemotherapeutic agent is at least one selected from the group consisting of a platinum agent and a pharmaceutically acceptable salt thereof.
[17] The anti-tumor agent according to any one of [9]-[16], wherein the hormonal therapeutic agent is at least one selected from the group consisting of an estrogen receptor modulator, an androgen receptor modulator, an LH-RH agonist, an LH-RH antagonist, an aromatase inhibitor, an androgen synthesis inhibitor, and a pharmaceutically acceptable salt thereof.
[18] The anti-tumor agent according to any one of [91-[17], wherein the hormonal therapeutic agent is at least one selected from the group consisting of an estrogen receptor modulator, an androgen receptor modulator, and a pharmaceutically acceptable salt thereof.
[19] The anti-tumor agent according to any one of [9]-[16], wherein the anti-cancer agent is at least one selected from the group consisting of a chemotherapeutic agent and a pharmaceutically acceptable salt thereof.
[20] The anti-tumor agent according to any one of [9] and [17] - [18], wherein the anti-cancer agent is at least one selected from the group consisting of a hormonal therapeutic agent and a pharmaceutically acceptable salt thereof.
[21] The anti-tumor agent according to [9], wherein the anti-cancer agent is at least one selected from the group consisting of an angiogenesis inhibitor and a pharmaceutically acceptable salt thereof.
[22] The anti-tumor agent according to [9], wherein the anti-cancer agent is at least one selected from the group consisting of an immunotherapeutic agent and a pharmaceutically acceptable salt thereof.
[23] The anti-tumor agent according to [9], wherein the anti-cancer agent is at least one selected from the group consisting of a kinase inhibitor and a pharmaceutically acceptable salt thereof.
[24] The anti-tumor agent according to [9], wherein the anti-cancer agent is at least one selected from the group consisting of an antibody medicine.

[25] The anti-tumor agent according to any one of [1] - [24], wherein the anti-diabetic agent is at least one selected from the group consisting of a biguanide drug, a thiazolidine derivative, and a pharmaceutically acceptable salt thereof.

[26] The anti-tumor agent according to any one of [1] - [25], wherein the agent for treating dyslipidemia is at least one selected from the group consisting of an HMG-CoA reductase inhibitor, a cholesterol absorption inhibitor, and a pharmaceutically acceptable salt thereof.

[27] The anti-tumor agent according to any one of [1] - [24], wherein the agent selected from the group consisting of an anti-cancer agent, an anti-diabetic agent, an agent for treating dyslipidemia, an agent: for treating multiple sclerosis, a steroidal anti-inflammatory agent, a non-steroidal anti-inflammatory agent, an anti-fungal agent, and a pharmaceutically acceptable salt thereof is an anti-cancer agent.

[28] The anti-tumor agent according to any one of [1] - [8] and [25], wherein the agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof is an anti-diabetic agent.

[29] The anti-tumor agent according to any one of [1] - [8] and [26], wherein the agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof is an agent for treating dyslipidemia.

[30] An anti-tumor agent comprising the compound of formula (1) or a pharmaceutically acceptable salt thereof according to any one of [1] - [8] as an active ingredient, wherein the anti-tumor agent is administered in combination with at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof.

[31] A preparation comprising the compound of formula (1) according to any one of [1] - [8] or a pharmaceutically acceptable salt thereof, and at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof, as a combination preparation for administering the compound and the agent simultaneously, separately, or with time-interval in a cancer therapy.

[32] Use of the compound of formula (1) or a pharmaceutically acceptable salt thereof according to any one of [1] - [8] for the manufacture of a medicament for treating cancer in combination with at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof.

[33] A method for treating cancer which comprises administering a therapeutically effective amount of a combination of the compound of formula (1) according to any one of [1] - [8] or a pharmaceutically acceptable salt thereof and at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof to a patient in need thereof.

[34] A medicament for treating a tumor with gene mutation in Wnt/β-catenin pathway, comprising the compound of formula (1) according to any one of [1] - [8] or a pharmaceutically acceptable salt thereof.

[35] The medicament according to [34], wherein the gene mutation in Wnt/β-catenin pathway is at least one selected from the group consisting of APC gene mutation, CTNNB1 gene mutation, AXIN1 gene mutation, and AXIN2 gene mutation.

[36] The anti-tumor agent according to [11], wherein the alkylating agent is at least one selected from the group consisting of bendamustine, busulfan, carmustine, cyclophosphamide, streptozocin, dacarbazine, procarbazine, ifosfamide, melphalan, nimustine, ranimustine, and temozolomide.

[37] The anti-tumor agent according to [12], wherein the anti-metabolite is at least one selected from the group consisting of azacitidine, capecitabine, cladribine, clofarabine, cytarabine, doxifluridine, enocitabine, fludarabine, 5-fluorouracil drug (such as fluorouracil, tegafur, trifluridine), gemcitabine, hydroxycarbamide, mercaptopurine, methotrexate, nelarabine, pemetrexed, forodesine, and pentostatin.

[38] The anti-tumor agent according to [12], wherein the anti-metabolite is at least one selected from the group consisting of azacitidine, capecitabine, cladribine, clofarabine, cytarabine, doxifluridine, enocitabine, fludarabine, 5-fluorouracil drug (such as fluorouracil, tegafur, trifluridine), gemcitabine, hydroxycarbamide, mercaptopurine, methotrexate, nelarabine, and pemetrexed.

[39] The anti-tumor agent according to [12], wherein the anti-metabolite is at least one selected from the group consisting of gemcitabine and 5-fluorouracil drug.

[40] The anti-tumor agent according to [13], wherein the anti-cancer antibiotic is at least one selected from the group consisting of aclarubicin, actinomycin D, amrubicin, bleomycin, daunorubicin, doxorubicin, epirubicin, idarubicin, mitomycin C, peplomycin, and pirarubicin.

[41] The anti-tumor agent according to [14], wherein the microtubule inhibitor is at least one selected from the group consisting of docetaxel, paclitaxel, cabazitaxel, eribulin, vinblastine, vincristine, vindesine, and vinorelbine.

[42] The anti-tumor agent according to [14], wherein the microtubule inhibitor is at least one selected from the group consisting of docetaxel, paclitaxel, eribulin, vinblastine, vincristine, vindesine, and vinorelbine.

[43] The anti-tumor agent according to [14], wherein the microtubule inhibitor is at least one selected from the group consisting of docetaxel and paclitaxel.

[44] The anti-tumor agent according to [14], wherein the microtubule inhibitor is docetaxel.

[45] The anti-tumor agent according to [15], wherein the topoisomerase inhibitor is at least one selected from the group consisting of etoposide, irinotecan, nogitecan, and sobuzoxane.

[46] The anti-tumor agent according to [16], wherein the platinum agent is at least one selected from the group consisting of carboplatin, cisplatin, oxaliplatin, nedaplatin, and miriplatin.

[47] The anti-tumor agent according to [18], wherein the estrogen receptor modulator is at least one selected from the group consisting of tamoxifen, toremifene, raloxifene, and fulvestrant.

[48] The anti-tumor agent according to [18], wherein the androgen receptor modulator is at least one selected from the group consisting of chlormadinone, bicalutamide, flutamide, and enzalutamide.

[49] The anti-tumor agent according to [18], wherein the androgen receptor modulator is at least one selected from the group consisting of bicalutamide, flutamide, and enzalutamide.

[50] The anti-tumor agent according to [18], wherein the androgen receptor modulator is at least one selected from the group consisting of bicalutamide and enzalutamide.

[51] The anti-tumor agent according to [18], wherein the androgen receptor modulator is bicalutamide.

[52] The anti-tumor agent according to [21], wherein the angiogenesis inhibitor is at least one selected from the group consisting of bevacizumab, ramucirumab, and aflibercept.

[53] The anti-tumor agent according to [21], wherein the angiogenesis inhibitor is at least one selected from the group consisting of bevacizumab and ramucirumab.

[54] The anti-tumor agent according to [22], wherein the immunotherapeutic agent is at least one selected from the group consisting of krestin, picibanil, ubenimex, lentinan, interferon, interleukin, macrophage colony stimulating factor, granulocyte colony stimulating factor, erythropoietin, an anti-CTLA-4 antibody (e.g., ipilimumab, tremelimumab), an anti-PD-1 antibody (e.g., nivolumab, pembrolizumab), an anti-PD-LI antibody (e.g., avelumab, atezolizumab), and a Toll-like receptor agonist, and is preferably an anti-PD-1 antibody.

[55] The anti-tumor agent according to [23], wherein the kinase inhibitor is at least one selected from the group consisting of alectinib, afatinib, axitinib, bosutinib, crizotinib, vemurafenib, dabrafenib, dasatinib, erlotinib, everolimus, gefitinib, ibrutinib, imatinib, lapatinib, ruxolitinib, nilotinib, osimertinib, pazopanib, regorafenib, lenvatinib, sorafenib, sunitinib, ceritinib, vandetanib, temsirolimus, and trametinib.

[56] The anti-tumor agent according to [23], wherein the kinase inhibitor is at least one selected from the group consisting of afatinib, axitinib, bosutinib, crizotinib, dabrafenib, dasatinib, erlotinib, everolimus, gefitinib, ibrutinib, imatinib, lapatinib, ruxolitinib, nilotinib, osimertinib, pazopanib, regorafenib, lenvatinib, sorafenib, sunitinib, ceritinib, vandetanib, temsirolimus, and trametinib.

[57] The anti-tumor agent according to [23], wherein the kinase inhibitor is at least one selected from the group consisting of sunitinib, sorafenib, regorafenib, pazopanib, dasatinib, imatinib, and lapatinib.

[58] The anti-tumor agent according to [23], wherein the kinase inhibitor is at least one selected from the group consisting of sunitinib, sorafenib, pazopanib, dasatinib, imatinib, and lapatinib.

[59] The anti-tumor agent according to [24], wherein the antibody medicine is at least one selected from the group consisting of cetuximab, gemtuzumab ozogamicin, mogamulizumab, ofatumumab, panitumumab, pertuzumab, rituximab, trastuzumab, alemtuzumab, daratumumab, elotuzumab, brentuximab, and necitumumab.

[60] The anti-tumor agent according to [24], wherein the antibody medicine is at least one selected from the group consisting of cetuximab, gemtuzumab ozogamicin, mogamulizumab, ofatumumab, panitumumab, pertuzumab, rituximab, trastuzumab, and alemtuzumab.

[61] The anti-tumor agent according to [9], wherein the proteasome inhibitor is at least one selected from the group consisting of bortezomib, carfilzomib, and ixazomib.

[62] The anti-tumor agent according to [9], wherein the HDAC inhibitor is at least one selected from the group consisting of vorinostat, entinostat, belinostat, panobinostat, and romidepsin.

[63] The anti-tumor agent according to [9], wherein the HDAC inhibitor is at least one selected from the group consisting of vorinostat, entinostat, belinostat, and panobinostat.

(EFFECTS OF THE INVENTION)

The pharmaceutical composition of the present invention has an inhibitory effect on the sphere-forming ability of cancer cells, and inhibits the self-renewal ability of cancer stem cells which is important for the persistent proliferation of malignant tumor, the metastasis or recurrence of cancer, and the resistance to anti-tumor agents. Thus, the pharmaceutical composition of the present invention can be a novel and useful agent for treating cancer.

BRIEF DESCRIPTION OF THE DRAWINGS

Fig. 1-1 shows the result of the effect of the combination of the sphere-forming ability inhibitor and 5-FU in human colon cancer cell line HCT116 tumor-bearing mouse model.
Fig. 1-2 shows the result of the effect of the combination of the sphere-forming ability inhibitor and 5-FU in human colon cancer cell line HCT116 tumor-bearing mouse model.
Fig. 1-3 shows the result of the effect of the combination of the sphere-forming ability inhibitor and irinotecan in human colon cancer cell line HCT116 tumor-bearing mouse model.
Fig. 1-4 shows the result of the effect of the combination of the sphere-forming ability inhibitor and irinotecan in human colon cancer cell line HCT116 tumor-bearing mouse model.
Fig. 1-5 shows the result of the effect of the combination of the sphere-forming ability inhibitor and cisplatin in human lung cancer cell line H460 tumor-bearing mouse model.
Fig. 1-6 shows the result of the effect of the combination of the sphere-forming ability inhibitor and sunitinib in human colon cancer cell line HCT116 tumor-bearing mouse model.
Fig. 1-7 shows the result of the effect of the combination of the sphere-forming ability inhibitor and bevacizumab in human colon cancer cell line Colo205 tumor-bearing mouse model.
Fig. 1-8 shows the result of the effect of the combination of the sphere-forming ability inhibitor and fluvastatin in human colon cancer cell line HCT116 tumor-bearing mouse model.
Fig. 1-9 shows the result of the effect of the combination of the sphere-forming ability inhibitor and anti-mouse PD-1 antibody in human colon cancer cell line CT26 tumor-bearing mouse model.
Fig. 1-10 shows the result of the effect of the combination of the sphere-forming ability inhibitor and irinotecan in human colon cancer cell line HCT116 tumor-bearing mouse model.
Fig. 1-11 shows the result of the effect of the combination of the sphere-forming ability inhibitor and docetaxel in human lung cancer cell line H460 tumor-bearing mouse model.
Fig. 1-12 shows the result of the effect of the combination of the sphere-forming ability inhibitor and regorafenib in human colon cancer cell line HCT116 tumor-bearing mouse model.
Fig. 1-13 shows the result of the effect of the combination of the sphere-forming ability inhibitor and anti-mouse PD-1 antibody in human colon cancer cell line CT26 tumor-bearing mouse model.

DESCRIPTION OF EMBODIMENTS

Hereinafter, the present invention is explained in detail. The number of carbon atoms in the definition of the "substituent" used herein may be expressed as, for example, "$C_{1-6}$". Specifically, the term "$C_{1-6}$ alkyl" is used for the same meaning as alkyl group having 1 to 6 carbon atoms.

Specific examples of "halogen atom" used herein include fluorine atom, chlorine atom, bromine atom, and iodine atom. The halogen atom is preferably fluorine atom and chlorine atom.

The term "$C_{1-6}$ alkyl" used herein means a straight or branched, saturated hydrocarbon group having 1 to 6 carbon atoms. The group is preferably "$C_{1-4}$ alkyl". Specific examples of the "$C_{1-6}$ alkyl" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

The term "$C_{2-6}$ alkenyl" used herein means a straight or branched, unsaturated hydrocarbon group having 2 to 6 carbon atoms and 1 to 3 carbon-carbon double bonds. The group is preferably "$C_{2-4}$ alkenyl". Specific examples of the "$C_{2-6}$ alkenyl" include ethenyl, propenyl, butenyl, pentenyl, and hexenyl.

The term "$C_{1-4}$ alkylene" used herein means a straight or branched, divalent saturated hydrocarbon group having 1 to 4 carbon atoms, or a divalent saturated hydrocarbon group containing a cyclic structure having 3 to 4 carbon atoms.

Specific examples of the straight or branched "$C_{1-4}$ alkylene" include methylene, ethylene, propylene, butylene, 1-methylmethylene, 1-ethylmethylene, 1-propylmethylene, 1-methylethylene, 2-methylethylene, and 1-ethylethylene. Preferred examples thereof include methylene and ethylene.

Specific examples of the "$C_{1-4}$ alkylene" containing a cyclic structure include the following groups:

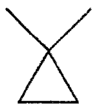 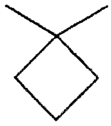 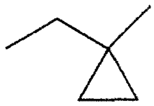 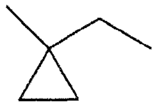 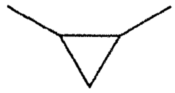

The "$C_{1-6}$ alkyl" moiety of the term "$C_{1-6}$ alkoxy" used herein is as defined in the above "$C_{1-6}$ alkyl". The group is preferably "$C_{1-4}$ alkoxy". Specific examples of the "$C_{1-6}$ alkoxy" include methoxy, ethoxy, propoxy, isopropoxy, butoxy, isobutoxy, sec-butoxy, and tert-butoxy.

The term "$C_{3-10}$ cycloalkyl" used herein means a 3-to 10-membered monocyclic or polycyclic, saturated or partially-unsaturated hydrocarbon group. The group is preferably "$C_{3-7}$ cycloalkyl". Specific examples of the "$C_{3-10}$ cycloalkyl" include cyclopropyl, cyclobutyl, cyclopentyl, cyclohexyl, cycloheptyl, cyclopentenyl, cyclohexenyl, decalinyl, adamantyl, and norbornyl.

The term "$C_{6-10}$ aryl" used herein means an aromatic hydrocarbon group having 6 to 10 carbon atoms. The group is preferably "$C_6$ aryl" (phenyl). Specific examples of the "$C_{6-10}$ aryl" include phenyl, 1-naphthyl, and 2-naphthyl.

The "$C_{6-10}$ aryl" also encompasses a fused ring group of phenyl with a 5- to 7-membered non-aromatic ring which contains the same or different one or more (e.g., 1 to 4) heteroatoms selected from nitrogen atom, sulfur atom, or oxygen atom, or a 5- to 7-membered saturated or partially-unsaturated hydrocarbon ring (cyclopentane or cyclohexane). The polycyclic "$C_{6-10}$ aryl" in which an aromatic ring and a non-aromatic ring are fused has the bond for a "group" in only the aromatic ring.

Specific examples of the group include the groups of the following formulae. The bond across a ring in the following formulae means that a "group" is linked at any replaceable position in the ring.

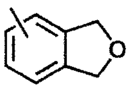 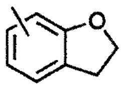 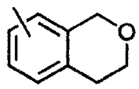 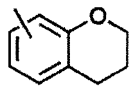 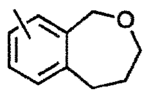 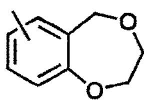 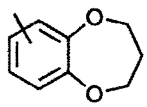

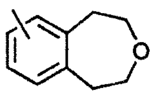 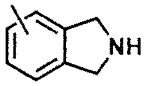 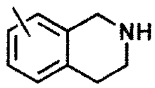 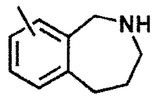 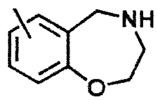 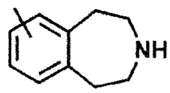

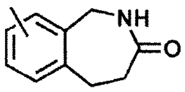 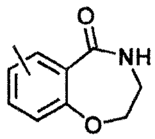 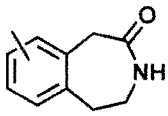 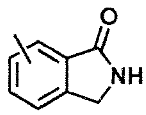 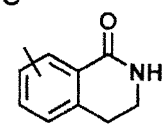

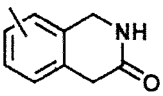 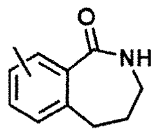 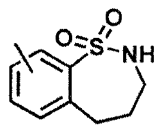 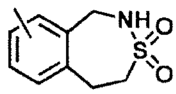 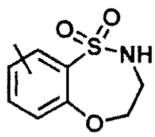

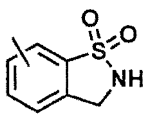 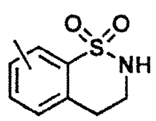 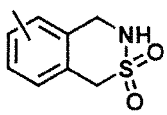 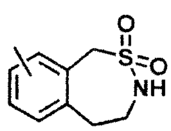 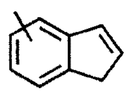

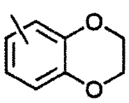 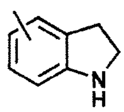 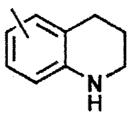 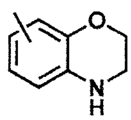 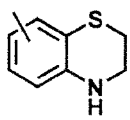 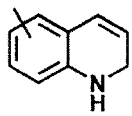

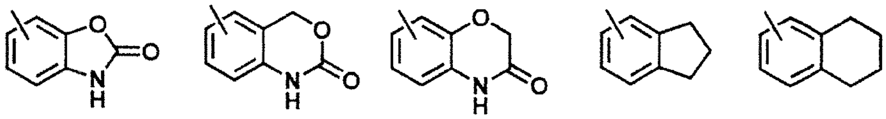

Examples of the term "5- to 10-membered heteroaryl" used herein include a 5- to 10-membered mono- or bicyclic aromatic heterocyclic group which contains the same or different one or more (e.g., 1 to 4) heteroatoms selected from the group consisting of nitrogen atom, sulfur atom, and oxygen atom. The bicyclic heteroaryl also encompasses a fused ring group of a monocyclic heteroaryl group mentioned above with an aromatic ring (such as benzene and pyridine) or a non-aromatic ring (such as cyclohexane, pyrrolidine, piperidine, tetrahydrofuran, tetrahydropyran, and 1,4-dioxane). Specific examples of the "heteroaryl" include the groups of the following formulae:

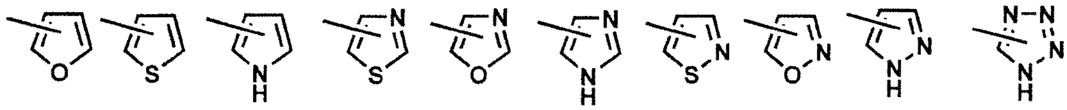

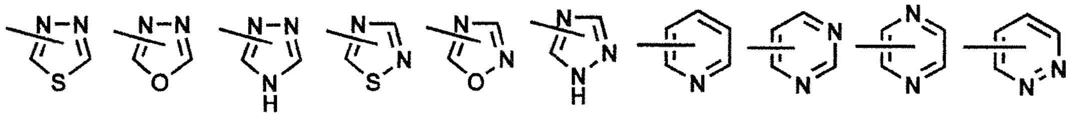

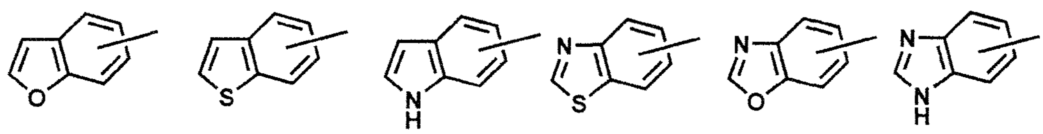

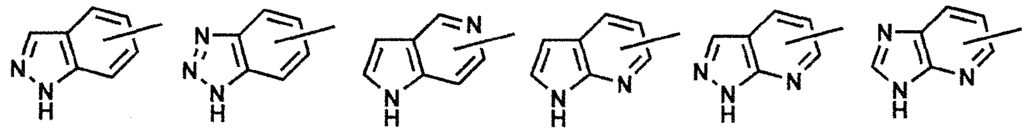

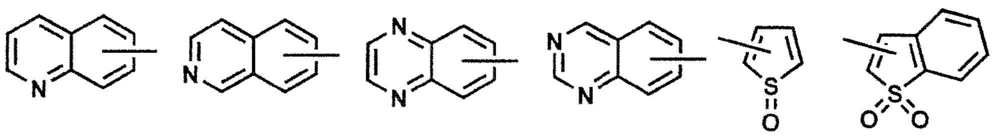

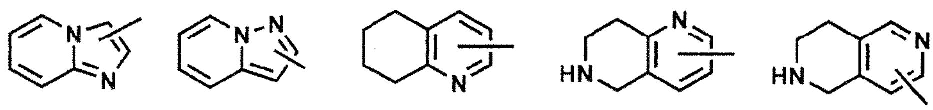

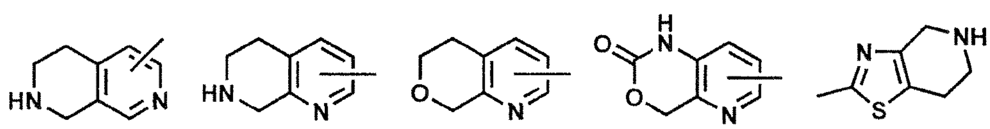

The bond across a ring in the above formulae means that a "group" is linked at any replaceable position in the ring. For example, when a group is the heteroaryl group of the following formula:

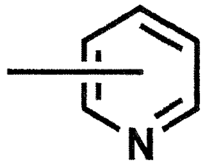

,

the group means 2-pyridyl, 3-pyridyl, or 4-pyridyl.

Furthermore, when a "heteroaryl" is a bicyclic group, for example, the group of the following formula:

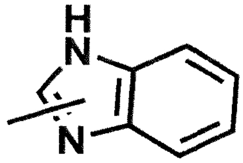

,

the group may be 1-benzimidazolyl, 2-benzimidazolyl, or 4-, 5-, 6- or 7-benzimidazolyl.

The polycyclic heteroaryl in which an aromatic ring and a non-aromatic ring (such as cyclohexane and piperidine) are fused has the bond for a "group" in only the aromatic ring. For example, when the "polycyclic heteroaryl" is the group of the following formula:

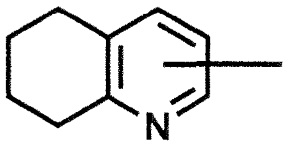

,

the bond means that a "group" is linked at the 2-, 3-, or 4-position.

In the groups of formulae (11)-(16) defined in the above [2], the two atoms indicated by arrows, which are shared between ring $Q^2$ or ring $Q^3$ and another ring fused with the ring, are carbon.

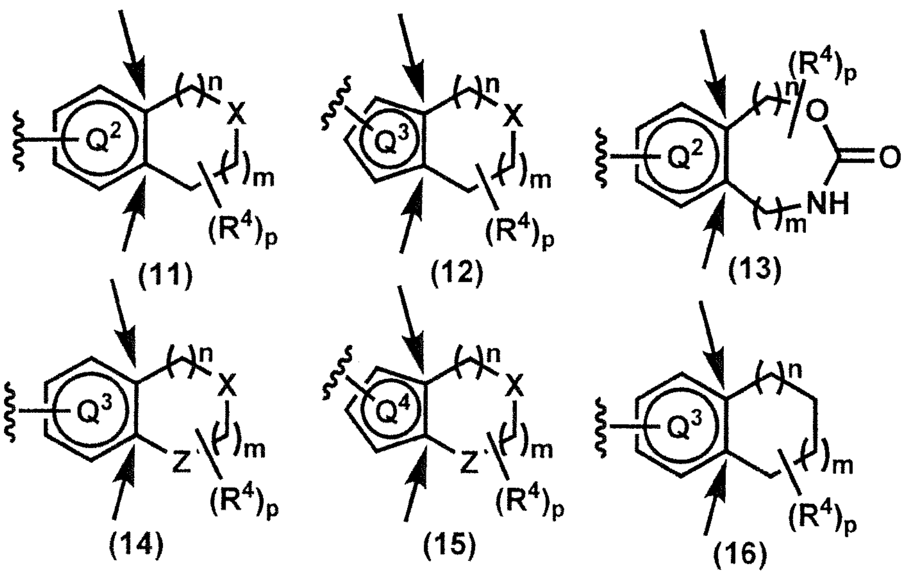

(11) (12) (13)

(14) (15) (16)

The "$C_{1-6}$ alkyl" moiety of the term "$C_{1-6}$ alkyl-carbonylamino" used herein is as defined in the above "$C_{1-6}$ alkyl". Preferred examples thereof include "$C_{1-4}$ alkyl-carbonylamino", more preferably methylcarbonylamino (acetylamino).

Examples of the substituent in the term "optionally-substituted $C_{6-10}$ aryl", "optionally-substituted $C_{6-10}$ aryloxy", "optionally-substituted $C_{6-10}$ arylthio", "optionally-substituted $C_{3-10}$ cycloalkyl", "optionally-substituted 5- to 10-membered heteroaryl", "optionally-substituted benzene ring", "optionally-substituted pyridine ring", "optionally-substituted pyrimidine

ring", "optionally-substituted pyridazine ring", "optionally-substituted pyrazine ring" include

(a) halogen atom,
(b) $C_{1-6}$ alkyl which may be optionally substituted with the same or different 1 to 3 groups selected from the group consisting of halogen atom, hydroxy, and $C_{1-6}$ alkoxy,
(c) $C_{1-6}$ alkoxy which may be optionally substituted with the same or different 1 to 3 groups selected from the consisting of halogen atom, hydroxy, and $C_{1-6}$ alkoxy,
(d) cyano,
(e) phenyl which may be optionally substituted with the same or different 1 to 4 groups selected from the group consisting of halogen atom, $C_{1-6}$ alkyl, and $C_{1-6}$ alkoxy,
(f) 5- or 6-membered heteroaryl which may be optionally substituted with the same or different 1 to 4 groups selected from the group consisting of halogen atom, $C_{1-6}$ alkyl, and $C_{1-6}$ alkoxy,
(g) phenoxy which may be optionally substituted with the same or different 1 to 4 groups selected from the group consisting of halogen atom, $C_{1-6}$ alkyl, and $C_{1-6}$ alkoxy,
(h) hydroxy,
(i) amino which may be optionally substituted with the same or different 1 to 2 $C_{1-6}$ alkyl groups, and
(j) aminocarbonyl wherein the amino moiety thereof may be optionally substituted with the same or different 1 to 2 $C_{1-6}$ alkyl groups.

The substituent is preferably halogen atom, $C_{1-6}$ alkyl which may be optionally substituted with the same or different 1 to 3 groups selected from the group consisting of halogen atom, hydroxy, and $C_{1-6}$ alkoxy, $C_{1-6}$ alkoxy which may be optionally substituted with the same or different 1 to 3 groups selected from the group consisting of halogen atom, hydroxy, and $C_{1-6}$ alkoxy, or cyano.

The substituent is more preferably halogen atom, or $C_{1-6}$ alkyl which may be optionally substituted with 1 to 3 fluorine atoms.

In the polycyclic aryl or heteroaryl in which an aromatic ring and a non-aromatic ring are fused, the above substituent(s) may be introduced on either the aromatic ring or the non-aromatic ring.

In the present compound of formula (1), $W^1$, $W^2$, $R^1$, $R^2$, ring $Q^1$, and ring $Q^2$ are preferably those shown below, but the technical scope of the present invention should not be limited to the following compounds.

$W^1$ is preferably methylene.

$W^2$-$Q^2$ is preferably -NHC(O)-$Q^2$, -NHC(O)-CH=CH-$Q^2$, - C(O)NH-$Q^2$, or -NHC(O)$CH_2$O-$Q^2$; more preferably -NHC(O)-$Q^2$ or - NHC(O)-CH=CH-$Q^2$; and furthermore preferably -NHC(O)-$Q^2$.

Preferably, $R^1$ and $R^2$ independently include hydrogen atom, chlorine atom, or methyl. $R^1$ and $R^2$ are more preferably hydrogen atom.

Ring $Q^1$ preferably includes phenyl which may be optionally substituted with the same or different 1 to 4 groups selected from the group consisting of

(1) halogen atom,
(2) $C_{1-6}$ alkyl which may be optionally substituted with the same or different 1 to 3 groups selected from the group consisting of halogen atom, hydroxy, and $C_{1-6}$ alkoxy,
(3) $C_{1-6}$ alkoxy which may be optionally substituted with the same or different 1 to 3 groups selected from the group consisting halogen atom, hydroxy, $C_{1-6}$alkoxy, and phenyl,
(4) amino which may be optionally substituted with the same or different 1 to 2 $C_{1-6}$ alkyl groups,
(5) $C_{6-10}$ aryl which may be optionally substituted with the same or different 1 to 4 groups selected from the group consisting of halogen atom, $C_{1-6}$ alkyl, and $C_{1-6}$ alkoxy,
(6) $C_{6-10}$ aryloxy which may be optionally substituted with the same or different 1 to 4 groups selected from the group consisting of halogen atom, $C_{1-6}$ alkyl, and $C_{1-6}$ alkoxy,
(7) 5- to 10-membered heteroaryl which may be optionally substituted with the same or different 1 to 4 groups selected from the group consisting of halogen atom, $C_{1-6}$ alkyl, and $C_{1-6}$ alkoxy, and
(8) $C_{1-6}$ alkoxy-carbonyl.

Ring $Q^1$ is more preferably phenyl which may be optionally substituted with the same or different 1 to 4 groups selected from the group consisting of halogen atom, and $C_{1-6}$ alkyl which may be optionally substituted with the same or different 1 to 3 halogen atoms; and furthermore preferably phenyl substituted with the same or different 1 to 3 halogen atoms, or trifluoromethylphenyl.

Ring $Q^2$ is preferably

(1) phenyl which may be optionally substituted with the same or different 1 to 4 groups selected from the group

consisting of

(a) halogen atom,
(b) $C_{1-6}$ alkyl which may be optionally substituted with the same or different 1 to 3 groups selected from the group consisting of halogen atom, hydroxy, and $C_{1-6}$ alkoxy,
(c) $C_{1-6}$ alkoxy which may be optionally substituted with the same or different 1 to 3 groups selected from the group consisting of halogen atom, hydroxy, and $C_{1-6}$ alkoxy,
(d) $C_{3-7}$ cycloalkyl,
(e) $C_{2-6}$ alkenyl,
(f) cyano,
(g) amino which may be optionally substituted with the same or different 1 to 2 $C_{1-6}$ alkyl group, and
(h) $C_{1-6}$ alkyl-carbonylamino,

(2) 5- or 6-membered heteroaryl which may be optionally substituted with the same or different 1 to 4 groups selected from the group consisting of (a)-(h) defined in the above (1), or
(3) a group of the following formula (11), (12), (13), (14), (15), or (16):

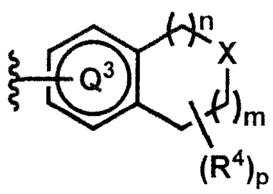
(11)

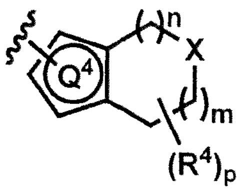
(12)

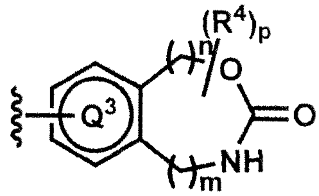
(13)

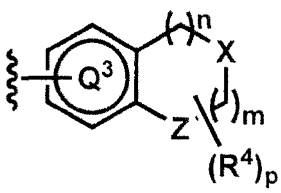
(14)

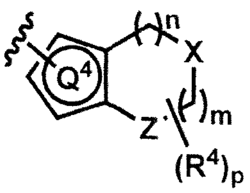
(15)

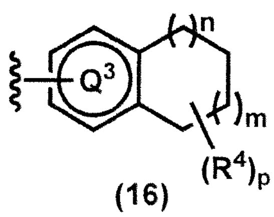
(16)

wherein ring $Q^3$ is optionally-substituted benzene ring, optionally-substituted pyridine ring, optionally-substituted pyrimidine ring, optionally-substituted pyridazine ring, or optionally-substituted pyrazine ring;
ring $Q^4$ is optionally-substituted 5-membered heteroaryl ring;
n and m are independently 0, 1, or 2, provided that n and m are not simultaneously 0;
X and Z are independently $NR^5$, $-NR^{3e}C(O)-$, $-C(O)NR^{3e}-$, or O wherein $R^5$ is hydrogen atom, $C_{1-6}$ alkyl which may be optionally substituted with the same or different 1 to 3 halogen atoms, or $C_{1-6}$ alkylcarbonyl; $R^{3e}$ is hydrogen atom or $C_{1-6}$ alkyl;
p is 1, 2, 3, 4, or 5;
$R^4$ is, independently when two or more exist, hydrogen atom, halogen atom, hydroxy, oxo, $C_{1-6}$ alkyl which may be optionally substituted with the same or different 1 to 3 halogen atoms, or $C_{1-6}$ alkoxy which may be optionally substituted with the same or different 1 to 3 halogen atoms.
Ring $Q^3$ is preferably benzene ring or pyridine ring.
Ring $Q^4$ is preferably imidazole ring, oxazole ring, or thiazole ring; and more preferably thiazole ring.

Ring $Q^2$ is more preferably

(1) phenyl which may be optionally substituted with the same or different 1 to 2 groups selected from the group consisting of $C_{1-6}$ alkoxy which may be optionally substituted with hydroxy, and $C_{1-6}$ alkyl-carbonylamino,
(2) a group of the following formula (2):

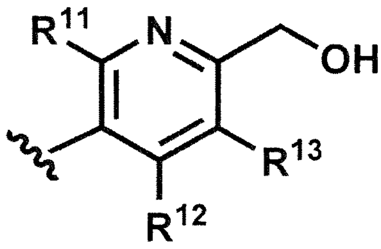

(2)

wherein $R^{11}$, $R^{12}$, and $R^{13}$ are independently

(a) hydrogen atom,
(b) halogen atom,
(c) $C_{1-6}$ alkyl which may be optionally substituted with 1 to 3 fluorine atoms, or
(d) amino which may be optionally substituted with the same or different 1 to 2 $C_{1-6}$ alkyl groups, or

(3) a group of formula (21):

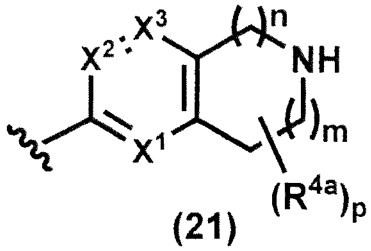

(21)

wherein $X^1$ is N or $CR^{14}$;
$X^2$ is N or $CR^{15}$;
$X^3$ is N or $CR^{16}$;
provided that $X^1$, $X^2$ and $X^3$ are not simultaneously N;
$R^{14}$, $R^{15}$, and $R^{16}$ are independently

(a) hydrogen atom,
(b) halogen atom,
(c) $C_{1-6}$ alkyl which may be optionally substituted with the same or different 1 to 3 halogen atoms, or
(d) $C_{1-6}$ alkoxy which may be optionally substituted with the same or different 1 to 3 halogen atoms;

n and m are independently 0, 1, or 2, provided that n and m are not simultaneously 0;
p is 1, 2, 3, 4, or 5;
$R^{4a}$ is, independently when two or more exist, hydrogen atom, halogen atom, or $C_{1-6}$ alkyl which may be optionally substituted with the same or different 1 to 3 halogen atoms.

Ring $Q^2$ is furthermore preferably

(1) acetylaminophenyl,
(2) 6-hydroxymethylpyridin-3-yl wherein the pyridine moiety thereof may be optionally further substituted with $C_{1-4}$ alkyl which may be optionally substituted with 1 to 3 fluorine atoms, or amino,
(3) a group of the following formula (21):

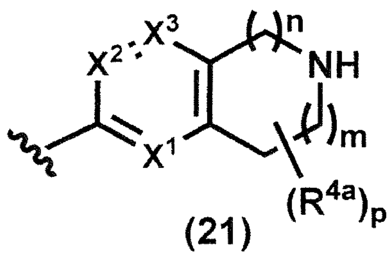

(21)

wherein $X^1$ is N, CH, or CF;
$X^2$ is N, CH, or CF;
$X^3$ is N, CH, or CF;
provided that $X^1$, $X^2$ and $X^3$ are not simultaneously N;
n is 1;
m is 0 or 1;
p is 1 or 2;
$R^{4a}$ is, independently when two or more exist, hydrogen atom or methyl.

Ring $Q^2$ is most preferably 5-difluoromethyl-6-hydroxymethylpyridin-3-yl.

The present compound may be in the forms of a hydrate and/or a solvate. Thus, the present compound also encompasses the hydrate and/or the solvate such as ethanol solvate. Furthermore, the present compound encompasses all types of crystal forms of the present compound.

Specific examples of the pharmaceutically acceptable salt of the compound of formula (1) include an inorganic acid salt such as hydrochloride, hydrobromide, sulfate, phosphate, and nitrate; and an organic acid salt such as acetate, propionate, oxalate, succinate, lactate, malate, tartrate, citrate, maleate, fumarate, methanesulfonate, p-toluenesulfonate, benzenesulfonate, and ascorbate.

The compound of formula (1) may be in the form of a tautomer. Thus, the present compound also encompasses the tautomer of the compound of formula (1).

The compound of formula (1) may contain one or more asymmetric carbon atoms. Thus, the present compound encompasses not only racemic forms of the compound of formula (1) but also optically-active forms thereof. When the compound of formula (1) contains two or more asymmetric carbon atoms, the compound can result in various stereoisomerisms. Thus, the present compound also encompasses the stereoisomer of the compound and a mixture or isolate thereof.

Also, the compound of formula (1) encompasses the compound wherein one or more of $^1H$ are replaced with $^2H(D)$ (i.e. deuterated form).

Hereinafter, the preparations of the compound of formula (1) are illustrated with some examples, but the invention should not be limited thereto.

The compound of formula (1) can be prepared according to processes shown below and according to the processes in combination with known compounds and known synthesis processes.

As appropriate, each compound used as a starting compound may be used in the salt form. The shown processes are just examples to prepare the compounds, and may be optionally modified by those skilled in the organic synthesis field.

In each process shown below, any functional groups which need to be protected may be optionally protected and then deprotected after the reaction or reactions are completed to give the desired compound even though the use of protective groups is not specifically described.

The protective group used herein includes any conventional groups described in various literatures, for example, T. W. Greene and P. G. M. Wuts, "Protective Groups in Organic Synthesis", 3rd Ed., John Wiley and Sons, inc., New York (1999). In more detail, specific examples of the protective groups for amino group include benzyloxycarbonyl, tert-butoxycarbonyl, acetyl, and benzyl, and specific examples of the protective groups for hydroxy group include trialkylsilyl, acetyl, and benzyl.

The protective groups can be introduced and cleaved according to commonly-used methods in synthetic organic chemistry (e.g., the method described in T. W. Greene and P. G. M. Wuts, "Protective Groups in Organic Synthesis", 3rd Ed., John Wiley and Sons, inc., New York (1999)) and similar methods thereto.

Preparation 1

One of the compounds of formula (1), the compound of formula (1-7) is prepared by linking each fragment in positions a and b, respectively.

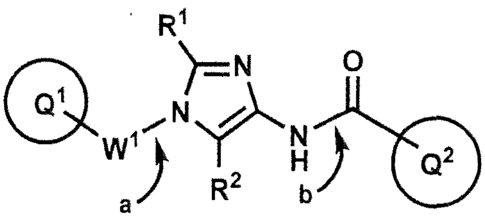

(1-7)

wherein $W^1$, $R^1$, $R^2$, ring $Q^1$, and ring $Q^2$ are as defined in the above [1].

The processes for forming each bond in positions a and b can be illustrated as follows, but the order of procedure for forming each bond may be optionally changed:

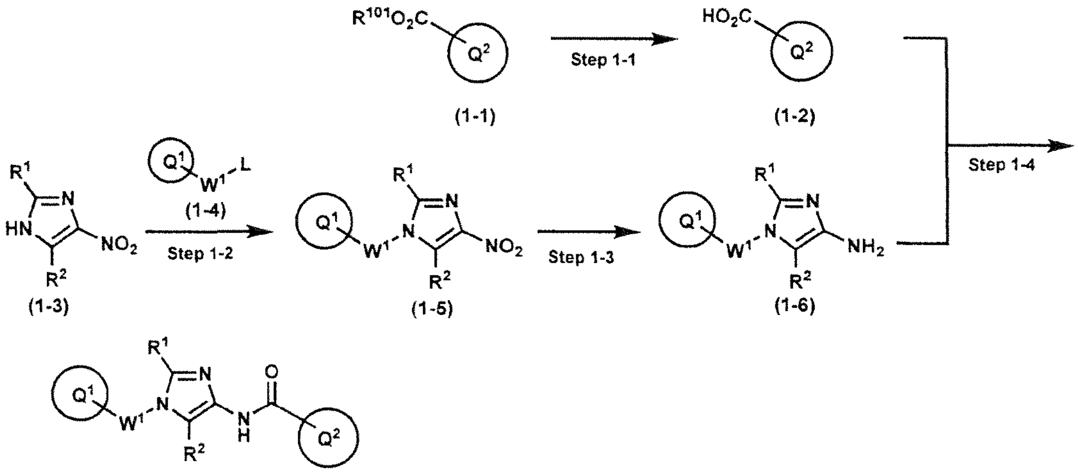


(1-7)

wherein $W^1$, $R^1$, $R^2$, ring $Q^1$, and ring $Q^2$ are as defined in the above [1]; $R^{101}$ is $C_{1-6}$ alkyl; L is a leaving group (such as iodine atom, bromine atom, chlorine atom, and substituted sulfonyloxy (e.g., methanesulfonyloxy and p-toluenesulfonyloxy)).

Compound (1-1) may be a commercially available product or be prepared according to known synthesis processes (e.g., New Version of Heterocyclic Compound (advanced level) edited by Kodansha Scientific Ltd.).

Step 1-1: Preparation process of compound (1-2)

Compound (1-2) is prepared by hydrolyzing compound (1-1) according to a similar process to a known process (e.g., Protective Groups in Organic Synthesis 3rd Edition (John Wiley & Sons, Inc.), Comprehensive Organic Transformation, by R. C. Larock, VCH publisher Inc., 1989).

Step 1-2: Preparation process of compound (1-5)

Compound (1-5) is prepared by the alkylation reaction of compounds (1-3) and (1-4) in an inert solvent in the presence of a base.

Specific examples of the base include an organic base such as triethylamine, diisopropylethylamine, and pyridine; an inorganic base such as potassium carbonate, sodium carbonate, cesium carbonate, potassium hydrogen carbonate, sodium hydrogen carbonate, potassium dihydrogen phosphate, dipotassium hydrogen phosphate, potassium phosphate, sodium dihydrogen phosphate, disodium hydrogen phosphate, sodium phosphate, potassium hydroxide, sodium hydroxide, and sodium hydride; and a metal alkoxide such as sodium methoxide and potassium tert-butoxide.

Specific examples of the inert solvent include a halogenated hydrocarbon such as chloroform and dichloromethane; an aromatic hydrocarbon such as toluene; an ether-type solvent such as diethyl ether, tetrahydrofuran (THF), and 1,4-dioxane; an aprotic polar solvent such as acetonitrile, acetone, methyl ethyl ketone, N,N-dimethylformamide, N-methyl-2-pyrrolidinone, and dimethyl sulfoxide; a basic solvent such as pyridine; and a mixture thereof.

The reaction temperature is typically 0°C to 150°C, preferably 20°C to 100°C, but is not limited thereto. The reaction time is typically 30 minutes to 48 hours, preferably 30 minutes to 10 hours.

Step 1-3: Preparation process of compound (1-6)

Compound (1-6) is prepared by reducing the nitro group in compound (1-5). For example, reductions under an acidic condition with a metal such as zinc, iron, and tin or a metal salt such as tin (II) chloride; reductions with a sulfide such as sodium hypodisulfite ($Na_2S_2O_4$) ; and catalytic hydrogenations with a metal catalyst such as palladium/carbon, Raney nickel, platinum oxide/carbon, and rhodium/carbon under hydrogen atmosphere may be used.

In the reduction with a metal or a metal salt, the amount of the metal or metal salt to be used is typically about 1 mole to 100 moles, preferably about 10 moles to 30 moles per mole of compound (1-5). Also, the amount of the acid to be used is typically about 1 mole to 100 moles, preferably about 10 moles to 30 moles per mole of compound (1-5). The reduction is typically carried out in a solvent which has no negative effect on the reaction (e.g., ethanol). The reaction temperature is typically 0°C to 100°C, but is not limited thereto. The reaction time is typically 30 minutes to 8 hours.

In the catalytic hydrogenation reaction, the amount of the metal catalyst to be used for compound (1-5) is typically 0.1% by weight to 1000% by weight, preferably 1% by weight to 100% by weight. The reaction may be carried out in a solvent such as an alcohol such as methanol; an ether such as tetrahydrofuran; and an ester such as ethyl acetate. The hydrogen pressure is typically 1 atm to 100 atms, preferably 1 atm to 5 atms. The reaction temperature is typically 0°C to 120°C, preferably 20°C to 80°C, but is not limited thereto. The reaction time is typically 30 minutes to 72 hours, preferably 1 hour to 48 hours.

Also, the reaction may be carried out in the presence of an acid catalyst, as appropriate. For example, an organic acid such as formic acid, acetic acid, and trifluoroacetic acid, and an inorganic acid such as sulfuric acid, hydrochloric acid, and hydrobromic acid are used as the acid catalyst. The amount of the acid to be used is 0.1 mole or more per mole of compound (1-5).

Step 1-4: Preparation process of compound (1-7)

Compound (1-7) is prepared by reacting compound (1-2) with compound (1-6) in an inert solvent in the presence of a condensation agent.

The reaction may be carried out in the presence of a base, as appropriate. The reaction temperature is typically about -20°C to the boiling point of the used solvent, but is not limited thereto. The reaction time is typically 10 minutes to 48 hours, which may vary according to various conditions such as a reaction temperature, a condensation agent, a starting material, and a solvent to be used.

Specific examples of the condensation agent include dicyclohexylcarbodiimide (DCC), diisopropylcarbodiimide (DIPC), 1-ethyl-3-(3-dimethylaminopropyl)-carbodiimide (WSC), benzotriazol-1-yloxytris(dimethylamino)phosphonium hexafluorophosphate (BOP), diphenylphosphoryl azide (DPPA), N,N'-carbonyldiimidazole (CDI), O-(benzotriazol-1-yl)-N,N,N',N'-tetramethyluronium hexafluorophosphate (HBTU), 0-(7-azabenzotriazol-1-yl)-N,N,N',N'-tetramethyluronium hexafluorophosphate (HATU), and diphenyl chlorophosphate. As appropriate, the reaction may be carried out with the addition of an additive such as N-hydroxysuccinimide (HOSu), 1-hydroxybenzotriazole (HOBt), and 3-hydroxy-4-oxo-3,4-dihydro-1,2,3-benzotriazine (HOOBt).

Specific examples of the base include an organic base such as triethylamine, diisopropylethylamine, and pyridine; an inorganic base such as potassium carbonate, sodium carbonate, cesium carbonate, potassium hydrogen carbonate, sodium hydrogen carbonate, potassium dihydrogen phosphate, dipotassium hydrogen phosphate, potassium phosphate, sodium dihydrogen phosphate, disodium hydrogen phosphate, sodium phosphate, potassium hydroxide, sodium hydroxide, and sodium hydride; and a metal alkoxide such as sodium methoxide and potassium tert-butoxide.

Specific example of the inert solvent include a halogenated hydrocarbon such as chloroform and dichloromethane; an aromatic hydrocarbon such as toluene; an ether-type solvent such as diethyl ether, tetrahydrofuran (THF), and 1,4-dioxane; an aprotic polar solvent such as acetonitrile, acetone, methyl ethyl ketone, dimethylformamide, N-methyl-2-pyrrolidinone, and dimethyl sulfoxide; a basic solvent such as pyridine; and a mixture thereof.

Compound (1-7) is also prepared by reacting compound (1-6) with an acid halide or an acid anhydride derived from compound (1-2) in an inert solvent in the presence of a base.

Preparation 2

One of the compounds of formula (1), the compound of formula (2-4) is prepared according to, for example, the following process.

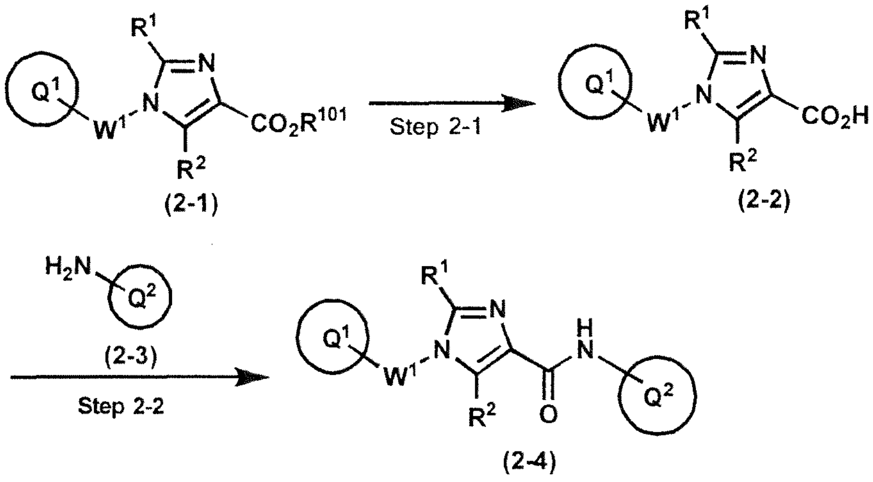


(2-1) (2-2) (2-4)

wherein $W^1$, $R^1$, $R^2$, ring $Q^1$, and ring $Q^2$ are as defined in the above [1] ; and $R^{101}$ is $C_{1-6}$ alkyl.

Compound (2-1) may be a commercially available product or be prepared according to known synthesis processes (e.g., WO 2014/125444).

Step 2-1: Preparation process of compound (2-2)

Compound (2-2) is prepared by hydrolyzing compound (2-1) according to a similar process to a known process (e.g., Protective Groups in Organic Synthesis 3rd Edition (John Wiley & Sons, Inc.), Comprehensive Organic Transformation, by R. C. Larock, VCH publisher Inc., 1989).

Step 2-2: Preparation process of compound (2-4)

Compound (2-4) is prepared from compounds (2-2) and (2-3) according to the process of Step 1-4.

<u>Preparation 3</u>

One of the compounds of formula (1), the compound of formula (1-7) is prepared according to, for example, the following process:

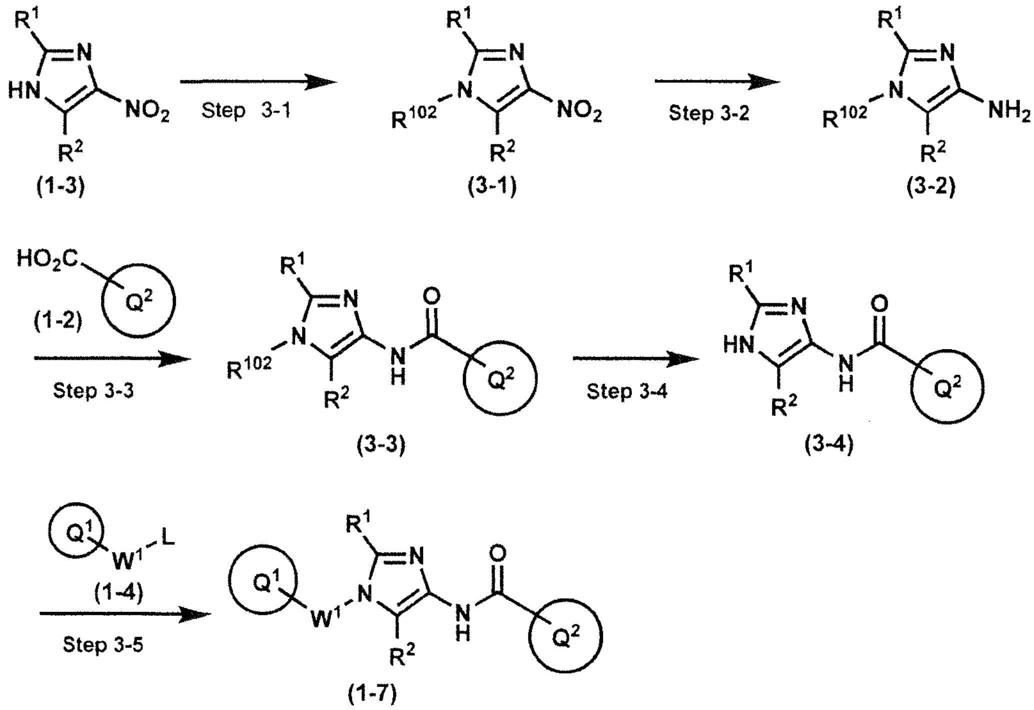


(1-3) (3-1) (3-2) (1-2) (3-3) (3-4) (1-4) (1-7)

wherein $W^1$, $R^1$, $R^2$, ring $Q^1$, and ring $Q^2$ are as defined in the above [1]; $R^{102}$ is a protective group; L is a leaving group (such as iodine atom, bromine atom, chlorine atom, and substituted sulfonyloxy (e.g., methanesulfonyloxy and p-toluenesulfonyloxy)) .

Step 3-1: Preparation process of compound (3-1)

Compound (3-1) is prepared by introducing a protectve group into nitrogen atom in imidazole group in compound (1-3) in an inert solvent. Examples of the protective group include 2-(trimethylsilyl)ethoxymethyl, benzyloxycarbonyl, tert-butoxycarbonyl, acetyl, and benzyl.

For example, when 2-(trimethylsilyl)ethoxymethyl group is introduced, compound (3-1) is prepared by reacting compound (1-3) with 2-(trimethylsilyl)ethoxymethyl chloride in an inert solvent in the presence of a base.

Examples of the base include potassium carbonate, sodium carbonate, cesium carbonate, potassium tert-butoxide, sodium hydride, sodium bis(trimethylsilyl)amide, lithium bis(trimethylsilyl)amide, potassium bis(trimethylsilyl)amide, and lithium diisoproylamide.

Examples of the inert solvent include DMF, THF, acetonitrile, and a mixture thereof.

The reaction temperature is typically 0°C to 150°C, preferably 0°C to 100°C, but is not limited thereto. The reaction time is typically 10 minutes to 24 hours, preferably 20 minutes to 6 hours.

Step 3-2: Preparation process of compound (3-2)

Compound (3-2) is prepared from compound (3-1) according to the process of Step 1-3.

Step 3-3: Preparation process of compound (3-3)

Compound (3-3) is prepared from compounds (3-2) and (1-2) according to the process of Step 1-4.

Step 3-4: Preparation process of compound (3-4)

Compound (3-4) is prepared by cleaving the protective group in nitrogen atom of imidazole group in compound (3-3) in an inert solvent.

For example, when 2-(trimethylsilyl)ethoxymethyl group is cleaved, compound (3-4) is prepared by reacting compound (3-3) with an acid or a fluorinating reagent.

Examples of the acid include TFA, formic acid, hydrochloric acid, sulfuric acid, p-toluenesulfonic acid, methanesulfonic acid, and (±) 10-camphorsulfonic acid.

Examples of the fluorinating reagent include hydrofluoric acid and tetrabutylammonium fluoride.

Examples of the solvent used include dichloromethane, 1,2-dichloroethane, 1,4-dioxane, THF, toluene, ethyl acetate, methanol, ethanol, 2-propanol, and a mixture thereof.

The reaction temperature is typically 0°C to 150°C, preferably 0°C to 50°C, but is not limited thereto. The reaction time is typically 5 minutes to 24 hours, preferably 1 hour to 9 hours.

Step 3-5: Preparation process of compound (1-7)

Compound (1-7) is prepared from compounds (3-4) and (1-4) according to the process of Step 1-2.

<u>Preparation 4</u>

One of the compounds of formula (1), the compound of formula (4-4) is prepared according to, for example, the following process:

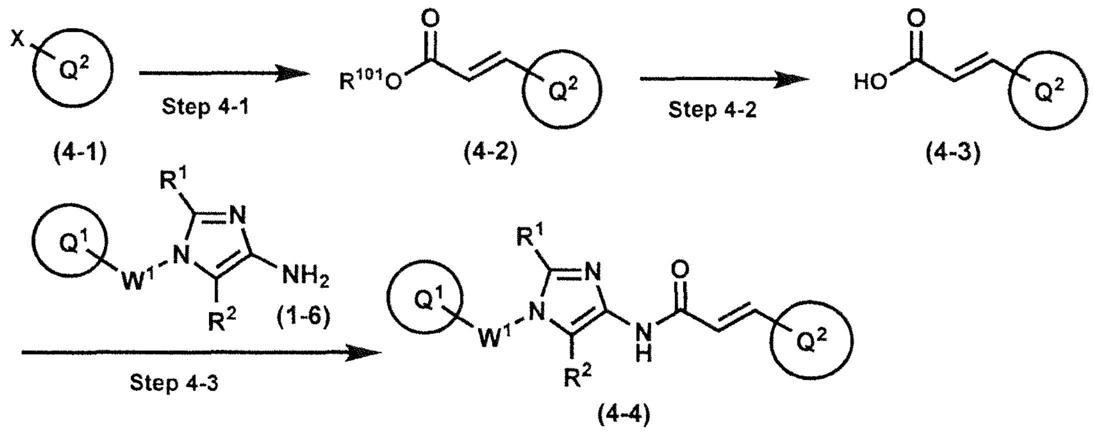

(4-1) (4-2) (4-3) (1-6) (4-4)

wherein $W^1$, $R^1$, $R^2$, ring $Q^1$, and ring $Q^2$ are as defined in the above [1]; $R^{101}$ is $C_{1-6}$ alkyl; and X is halogen atom.

Step 4-1: Preparation process of compound (4-2)

Compound (4-2) is prepared by reacting compound (4-1) with acrylate in an inert solvent in the presence of a palladium catalyst and a base.

Specific examples of the palladium catalyst include tetrakis(triphenylphosphine)palladium (0), dichlorodi(tri(o-tolylphosphine))palladium, bis(dibenzylideneacetone)palladium (0), tris(dibenzylideneacetone)dipalladium (0), bis(tri-tert-butylphosphine)palladium (0), [1,1'-bis(diphenylphosphino)ferrocene]palladium (II) dichloride.

Specific examples of the base include an inorganic base such as potassium carbonate, sodium carbonate, cesium carbonate, potassium phosphate, potassium hydroxide, and sodium hydroxide, triethylamine, and diisopropylethylamine.

Examples of the inert solvent include THF, acetonitrile, propionitrile, toluene, 1,2-dimethoxyethane, 1,4-dioxane, DMF, water, and a mixture thereof.

The reaction temperature is typically 50°C to 150°C, preferably 80°C to 120°C, but is not limited thereto. The reaction may be carried out under microwave irradiation. The reaction time is typically 1 hour to 24 hours, preferably 2 hours to 12 hours.

Step 4-2: Preparation process of compound (4-3)

Compound (4-3) is prepared by hydrolyzing compound (4-2) according to a similar process to a known process (e.g., Protective Groups in Organic Synthesis 3rd Edition (John Wiley & Sons, Inc.), Comprehensive Organic Transformation, by R. C. Larock, VCH publisher Inc., 1989).

Step 4-3: Preparation process of compound (4-4)

Compound (4-4) is prepared from compounds (4-3) and (1-6) according to the process of Step 1-4.

Preparation 5

One of the compounds of formula (1-1), the compound of formula (5-5) is prepared according to, for example, the following process:

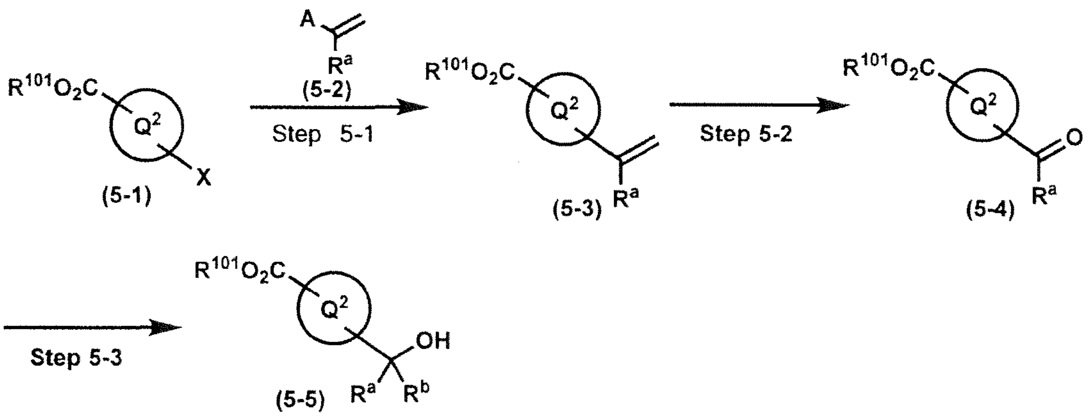


wherein ring $Q^2$ is as defined in the above [1]; A is boronic acid or boronate; $R^{101}$ is $C_{1-6}$ alkyl; $R^a$ and $R^b$ are independently the same or different hydrogen atom or methyl; X is halogen atom, and L is a leaving group (such as iodine atom, bromine atom, chlorine atom, and substituted sulfonyl (e.g., methanesulfonyl and p-toluenesulfonyl)).

Step 5-1: Preparation process of compound (5-3)

Compound (5-3) is prepared by reacting compound (5-1) with compound (5-2) in an inert solvent in the presence of a palladium catalyst and a base.

Specific examples of the palladium catalyst include tetrakis(triphenylphosphine)palladium (0), bis(dibenzylideneacetone)palladium (0), tris(dibenzylideneacetone)dipalladium (0), bis(tri-tert-butylphosphine)palladium (0), [1,1'-bis(diphenylphosphino)ferrocene]palladium (II) dichloride.

Specific examples of the base include potassium carbonate, sodium carbonate, cesium carbonate, potassium phosphate, potassium hydroxide, and sodium hydroxide.

Examples of the inert solvent include THF, toluene, 1,2-dimethoxyethane, 1,4-dioxane, DMF, water, and a mixture thereof.

The reaction temperature is typically 50°C to 150°C, preferably 80°C to 120°C, but is not limited thereto. The reaction may be carried out under microwave irradiation. The reaction time is typically 1 hour to 24 hours, preferably 2 hours to 12 hours.

Step 5-2: Preparation process of compound (5-4)

Compound (5-4) is prepared by reacting compound (5-3) with osmium tetroxide solution (immoblized catalyst, including microencapsulated osmium tetroxide) or potassium osmate (IV) dihydrate in the presence of sodium periodate.

Examples of the solvent used include acetone, 1,4-dioxane, THF, tert-butanol, water, and a mixture thereof.

The reaction temperature is typically 0°C to 100°C, preferably 25°C to 50°C, but is not limited thereto. The reaction time is typically 1 hour to 72 hours, preferably 1 hour to 24 hours.

Also, compound (5-4) is prepared by treating compound (5-3) with oxygen currents including ozone and then reacting the treated compound with a reducing agent such as dimethyl sulfide in a solvent such as dichloromethane, ethyl acetate, and methanol. The reaction temperature is typically -78°C to room temperature, but is not limited thereto. The reaction time is typically 1 hour to 72 hours, preferably 6 hours to 24 hours.

Step 5-3: Preparation process of compound (5-5)

Compound (5-5) is prepared by reacting compound (5-4) with a hydride reducing agent or an organometallic reagent.

Specific examples of the hydride reducing agent include sodium borohydride and sodium cyanoborohydride.

The solvent used in the reaction with the hydride reducing agent includes methanol, ethanol, dichloromethane, toluene, and a mixture thereof.

The reaction temperature is typically -78°C to 50°C, preferably 0°C to 25°C, but is not limited thereto. The reaction time is typically 5 minutes to 12 hours, preferably 30 minutes to 6 hours.

Specific examples of the organometallic reagent include methylmagnesium bromide, methylmagnesium iodide, and methyllithium.

Examples of the solvent used in the reaction with the organometallic reagent include THF, diethyl ether, and a mixture thereof.

The reaction temperature is typically -78°C to 25°C, preferably -40°C to 0°C, but is not limited thereto. The reaction time is typically 5 minutes to 12 hours, preferably 30 minutes to 6 hours.

Preparation 6

The compound of formula (6-5) is prepared according to, for example, the following process.

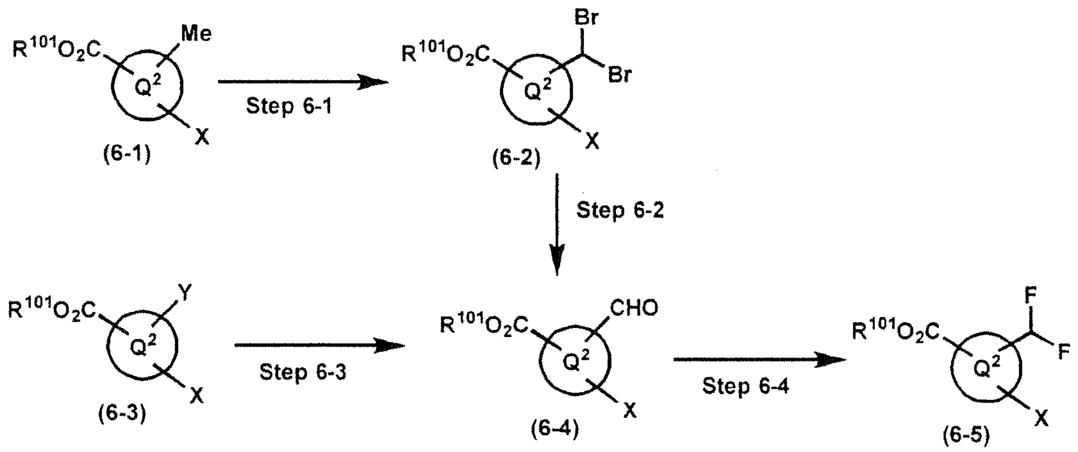


wherein ring $Q^2$ is as defined in the above [1]; $R^{101}$ is $C_{1-6}$ alkyl group; X is halogen atom; and Y is bromine atom or iodine atom.

Step 6-1: Preparation process of compound (6-2)

Compound (6-2) is prepared by reacting compound (6-1) with a brominating agent in an inert solvent in the presence of a radical initiator.

Specific examples of the radical initiator include azobisisobutyronitrile (AIBN) and benzoyl peroxide (BPO).

Specific examples of the brominating agent include N-bromosuccinimide and bromine.

Examples of the inert solvent include carbon tetrachloride, chlorobenzene, and a mixture thereof.

The reaction temperature is typically 50°C to 150°C, preferably 80°C to 120°C, but is not limited thereto. The reaction time is typically 3 hours to 48 hours, preferably 4 hours to 12 hours.

Step 6-2: Preparation process of compound (6-4)

Compound (6-4) is prepared by reacting compound (6-2) with silver nitrate in an inert solvent.

Specific examples of the inert solvent include acetonitrile, THF, 1,4-dioxane, and a mixture thereof under moisture conditions.

The reaction temperature is typically 50°C to 150°C, preferably 80°C to 120°C, but is not limited thereto. The reaction time is typically 3 hours to 48 hours, preferably 4 hours to 12 hours.

Step 6-3: Preparation process of compound (6-4)

Compound (6-4) is also prepared by reacting compound (6-3) with an organometallic reagent and then treating the resulting compound with a formylating agent.

Examples of the organometallic reagent include isopropylmagnesium chloride-lithium chloride complex, isopropylmagnesium chloride, and n-butyllithium.

Examples of the solvent used include THF, diethyl ether, toluene, and a mixture thereof.

Examples of the formylating agent include DMF and N-formylmorpholine.

The reaction temperature is typically -78°C to 50°C, preferably -30°C to 25°C, but is not limited thereto. The reaction time is typically 30 minutes to 24 hours, preferably 1 hour to 6 hours.

Step 6-4: Preparation process of compound (6-5)

Compound (6-5) is prepared by reacting compound (6-4) with a deoxofluorinating agent in an inert solvent.

Specific examples of the deoxofluorinating agent include diethylaminosulfur trifluoride (DAST), bis(2-methoxyethyl)aminosulfur trifluoride (Deoxo-Fluor®), XtalFluor-E®, XtalFluor-M®, and 4-tert-butyl-2,6-dimethylphenylsulfur trifluoride (Fluolead®). As appropriate, compounds such as diazabicycloundecene (DBU), triethylamine trihydrofluoride, and triethylamine dihydrofluoride may be used as a promoter.

Specific examples of the inert solvent include dichloromethane, 1,2-dichloroethane, toluene, THF, and a mixture thereof.

The reaction temperature is typically -20°C to 50°C, preferably 0°C to 25°C, but is not limited thereto. The reaction time is typically 10 minutes to 12 hours, preferably 30 minutes to 3 hours.

Compound (6-5) is also prepared by reacting compound (6-4) with sulfur tetrafluoride.

<u>Preparation 7</u>

One of the compounds of formula (1), the compound of formula (7-3) is prepared according to, for example, the following process.

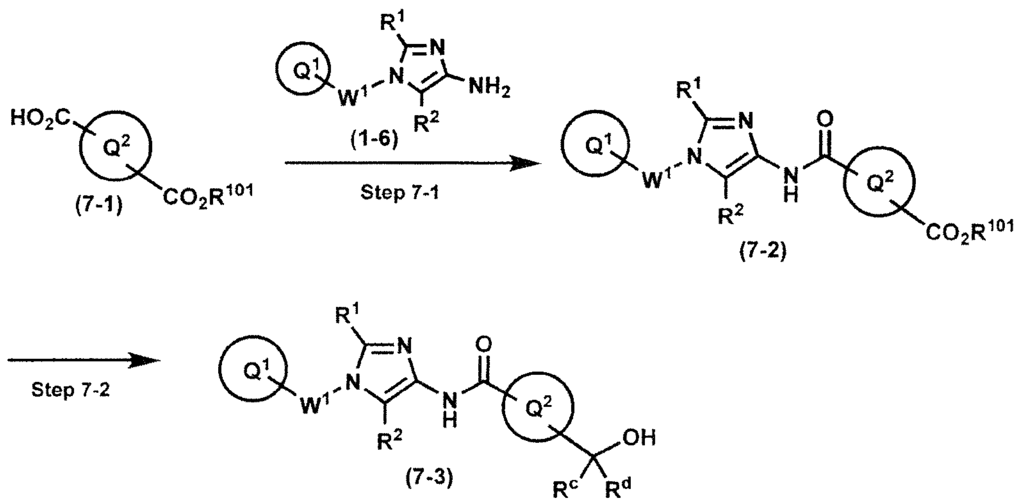

(7-1) (1-6) (7-2) (7-3)

wherein $W^1$, $R^1$, $R^2$, ring $Q^1$, and ring $Q^2$ are as defined in the above [1]; $R^{101}$ is $C_{1-6}$ alkyl; and $R^c$ and $R^d$ are independently the same or different hydrogen atom, deuterium atom, or methyl group.

Step 7-1: Preparation process of compound (7-2)

Compound (7-2) is prepared from compounds (7-1) and (1-6) according to the process of Step 1-4.

Step 7-2: Preparation process of compound (7-3)

Compound (7-3) is prepared by reacting compound (7-2) with a hydride reducing agent or an organometallic reagent in an inert solvent.

Specific examples of the hydride reducing agent include sodium borohydride, lithium borohydride, lithium aluminum hydride, sodium cyanoborohydride, lithium triethylborohydride, diisobutylaluminium hydride, sodium bis(2-methoxyethoxy)aluminium hydride, lithium borodeuteride, and lithium aluminum deuteride. Examples of the solvent used include methanol, ethanol, dichloromethane, toluene, and a mixture thereof.

The reaction temperature is typically -78°C to 25°C, preferably 0°C to 25°C, but is not limited thereto. The reaction time is typically 5 minutes to 12 hours, preferably 30 minutes to 6 hours.

Specific examples of the organometallic reagent include methylmagnesium bromide, methylmagnesium iodide, and methyllithium.

Examples of the solvent used in the reaction with the organometallic reagent include THF, diethyl ether, and

a mixture thereof.

The reaction temperature is typically -78°C to 25°C, preferably 0°C to 25°C, but is not limited thereto. The reaction time is typically 5 minutes to 12 hours, preferably 30 minutes to 6 hours.

Preparation 8

One of the compounds of formula (1), the compound of formula (8-5) is prepared according to, for example, the following process:

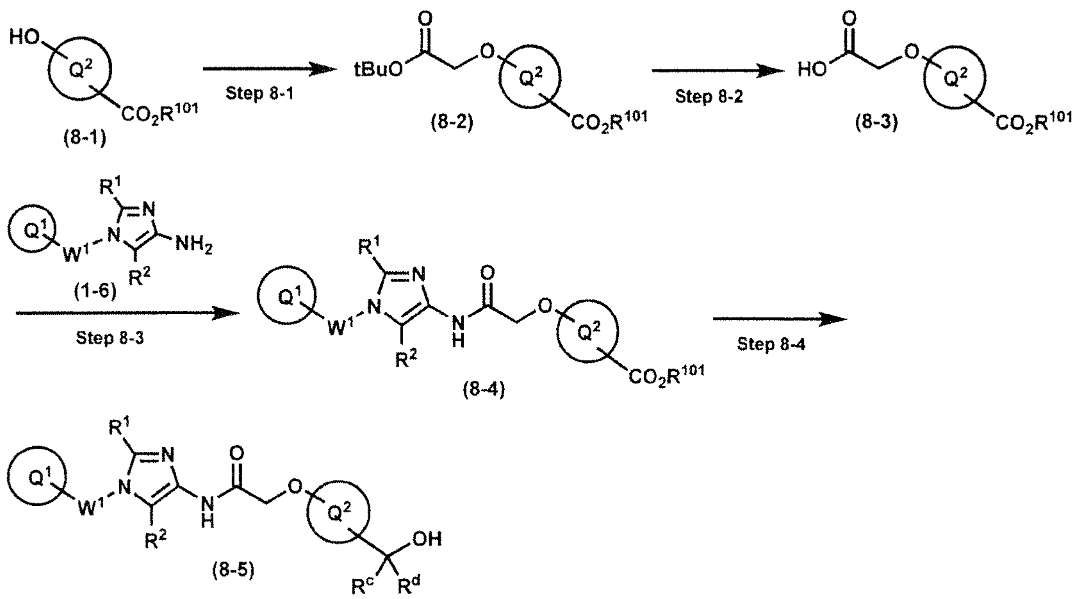


wherein $W^1$, $R^1$, $R^2$, ring $Q^1$, and ring $Q^2$ are as defined in [1]; $R^{101}$ is $C_{1-6}$ alkyl; and $R^c$ and $R^d$ are independently the same or different hydrogen atom, deuterium atom, or methyl group.

Step 8-1: Preparation process of compound (8-2)

Compound (8-2) is prepared by reacting compound (8-1) with haloacetate in an inert solvent in the presence of a base.

Specific examples of the haloacetate include tert-butyl chloroacetate, tert-butyl bromoacetate, and tert-butyl iodoacetate.

Examples of the base include potassium carbonate, sodium carbonate, cesium carbonate, potassium tert-butoxide, sodium hydride, sodium bis(trimethylsilyl)amide, lithium bis(trimethylsilyl)amide, potassium bis(trimethylsilyl)amide, and lithium diisopropylamide.

Examples of the inert solvent include DMF, THF, acetonitrile, and a mixture thereof.

The reaction temperature is typically 25°C to 150°C, preferably 70°C to 100°C, but is not limited thereto. The reaction time is typically 10 minutes to 12 hours, preferably 20 minutes to 6 hours.

Step 8-2: Preparation process of compound (8-3)

Compound (8-3) is prepared by cleaving tert-butylester group in compound (8-2) under an acid condition.

Examples of the acid used in the deprotection step include hydrochloric acid, sulfuric acid, HBr, HI, and TFA.

Examples of the solvent used include methanol, ethanol, dichloromethane, 1,2-dichloroethane, THF, 1,4-dioxane, ethyl acetate, a mixture thereof.

The reaction temperature is typically 0°C to 100°C, preferably 25°C to 50°C, but is not limited thereto. The reaction time is typically 1 hour to 24 hours, preferably 2 hours to 12 hours.

Step 8-3: Preparation process of compound (8-4)

Compound (8-4) is prepared from compounds (8-3) and (1-6) according to the process of Step 1-4.

Step 8-4: Preparation process of compound (8-5)

Compound (8-5) is prepared from compound (8-4) according to the process of Step 7-2.

Preparation 9

The compound of formula (9-4) is prepared according to, for example, the following process:

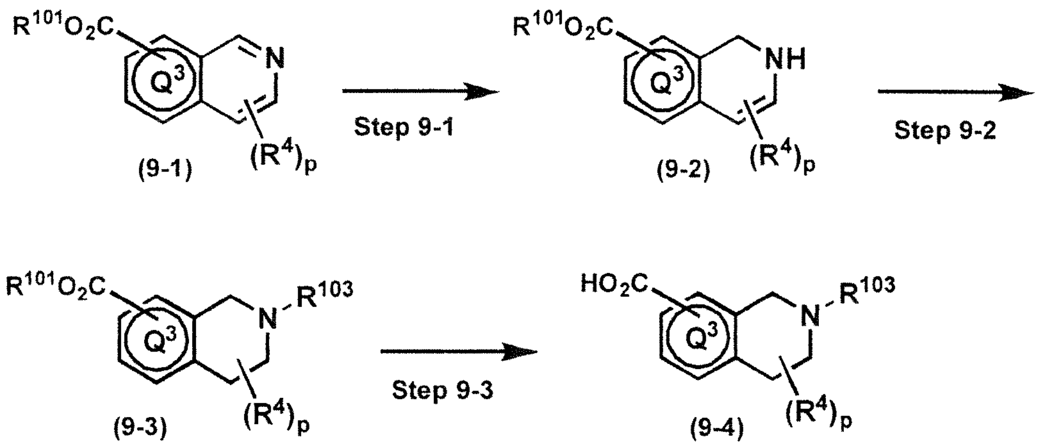


wherein $R^4$, p, and ring $Q^3$ are as defined in the above [2]; $R^{101}$ is $C_{1-6}$ alkyl; and $R^{103}$ is Cbz, Boc, benzyl, 4-methoxybenzyl, or Fmoc.

Compound (9-1) may be a commercially available product.

Step 9-1: Preparation process of compound (9-2)

Compound (9-2) is prepared by reacting compound (9-1) with a hydride reducing agent in an inert solvent.

Specific examples of the hydride reducing agent include sodium borohydride, sodium cyanoborohydride, borane, and hydride aluminium hydride.

Examples of the solvent used in the reaction with the hydride reducing agent include methanol, ethanol, dichloromethane, toluene, tetrahydrofuran, and a mixture thereof.

The reaction temperature is typically -78°C to 100°C, preferably 0°C to 50°C, but is not limited thereto. The reaction time is typically 5 minutes to 12 hours, preferably 30 minutes to 6 hours.

Step 9-2: Preparation process of compound (9-3)

Compound (9-3) is prepared by reducing olefin in compound (9-2) with a reagent for introducing a protective group. For example, reactions such as catalytic hydrogenation reaction with a metal catalyst such as palladium/carbon, Raney nickel, platinum oxide/carbon, and rhodium/carbon under hydrogen atmosphere in the presence of $Boc_2O$ are used.

In the catalytic hydrogenation reaction, the amount of the metal catalyst to be used for compound (9-2) is typically 0.1% by weight to 1000% by weight, preferably 1% by weight to 100% by weight. The reaction may be carried out in a solvent such as an alcohol such as methanol; an ether such as tetrahydrofuran; and an ester such as ethyl acetate. The hydrogen pressure is typically 1 atm to 100 atms, preferably 1 atm to 5 atms. The reaction temperature is typically 0°C to 120°C, preferably 20°C to 80°C, but is not limited thereto. The reaction time is typically 30 minutes to 72 hours, preferably 1 hour to 48 hours.

When $R^{103}$ is benzyl group, 4-methoxybenzyl group, etc., compound (9-3) can be directly prepared through a pyridinium salt intermediate of compound (9-1). For example, compound (9-3) is prepared by reducing the pyridinium salt of compound (9-1) synthesized by reacting compound (9-1) with a reagent such as benzyl bromide. Reduction reactions such as reduction with a hydride reducing agent and catalytic hydrogenation with a metal catalyst such as palladium/carbon, Raney nickel, platinum oxide/carbon, and rhodium/carbon under hydrogen atmosphere are used.

Step 9-3: Preparation process of compound (9-4)

Compound (9-4) is prepared by hydrolyzing compound (9-3) according to a similar process to a known process (e.g., Protective Groups in Organic Synthesis 3rd Edition (John Wiley & Sons, Inc.), Comprehensive Organic Transformation, by R. C. Larock et al., VCH publisher Inc., 1989).

Preparation 10

The compound of formula (10-5) is prepared according to, for example, the following process.

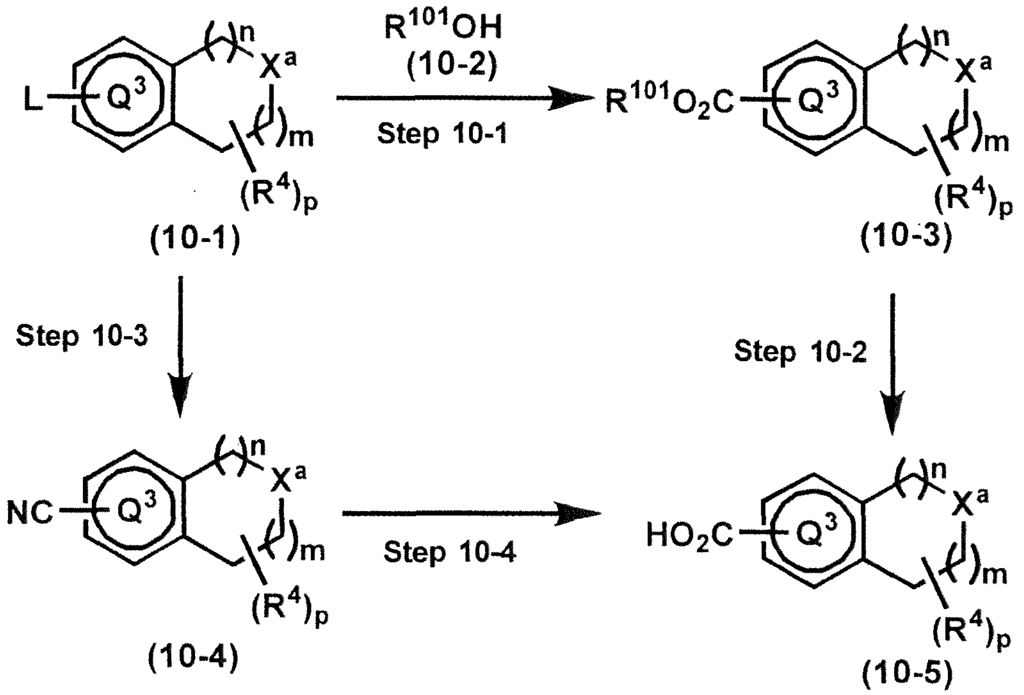


wherein $R^4$, n, m, p, and ring $Q^3$ are as defined in the above [2]; $R^{101}$ is $C_{1-6}$ alkyl; $X^a$ is O or $NR^{103}$; $R^{103}$ is Cbz, Boc, benzyl, 4-methoxybenzyl, or Fmoc; L is a leaving group (such as iodine atom, bromine atom, chlorine atom, and substituted sulfonyl (e.g., methanesulfonyl and p-toluenesulfonyl)).

Compound (10-1) may be a commercially available product or be prepared according to known synthesis processes (e.g., WO 2009/056556, WO 2006/065215).

Step 10-1: Preparation process of compound (10-3)

Compound (10-3) is prepared by introducing ester group to compound (10-1) under carbon monoxide atmosphere in the presence of a palladium catalyst, phosphorus ligand, an alcohol of formula (10-2) in an inert solvent.

The pressure of carbon monoxide is selected according to various conditions such as a reaction temperature, a starting material, and a solvent to be used, as appropriate, and is typically 1 atm to 100 atms, preferably 1 atm to 5 atms. The reaction temperature is typically about -20°C to the boiling point of the used solvent, preferably room temperature to the boiling point of the used solvent. The reaction time is typically 10 minutes to 48 hours, which may vary according to various conditions such as a reagent, a reaction temperature, a starting material, and a solvent to be used.

Examples of the palladium catalyst include tetrakis(triphenylphosphine)palladium and di-tert-butylphosphinepalladium.

Examples of the inert solvent include N,N-dimethylformamide, N-methyl-2-pyrrolidinone, 1,4-dioxane and a mixture thereof.

In addition, an organic base such as N,N-diisopropylethylamine and triethylamine may be added thereto, as appropriate.

Step 10-2: Preparation process of compound (10-5)

Compound (10-5) is prepared by hydrolyzing compound (2-3) according to a similar process to a known process (e.g., Protective Groups in Organic Synthesis 3rd Edition (John Wiley & Sons, Inc.), Comprehensive Organic Transformation, by R. C. Larock et al., VCH publisher Inc., 1989).

Step 10-3: Preparation process of compound (10-4)

Compound (10-4) is prepared by the cyanation of compound (10-1) in an inert solvent in the presence of a palladium catalyst, phosphorus ligand, and a cyanating agent.
The reaction temperature is typically about -20°C to the boiling point of the used solvent, preferably room temperature to the boiling point of the used solvent. The reaction may be carried out under microwave irradiation. The reaction time is typically 10 minutes to 48 hours, which may vary according to various conditions such as a reaction temperature, a reagent, a starting material, and a solvent to be used.
Examples of the cyanating agent include sodium cyanide, potassium cyanide, zinc cyanide, and copper (I) cyanide, preferably zinc cyanide.
Examples of the palladium catalyst include tetrakis(triphenylphosphine)palladium and di-tert-butylphosphinepalladium.
Examples of the inert solvent include N,N-dimethylformamide, N-methyl-2-pyrrolidinone, 1,4-dioxane and a mixture thereof.

Step 10-4: Preparation process of compound (10-5)

Compound (10-5) is prepared by hydrolyzing cyano group in compound (10-4) in an appropriate solvent in the presence of a base.
The reaction temperature is typically about -20°C to the boiling point of the used solvent, preferably room temperature to the boiling point of the used solvent. The reaction time is typically 10 minutes to 48 hours, which may vary according to various conditions such as a reaction temperature, a starting material, and a solvent to be used.
Examples of the base include sodium hydroxide, potassium hydroxide.
Examples of the solvent used include methanol, ethanol, 2-propanol, acetone, tetrahydrofuran, 1,4-dioxane, water, and a mixture thereof.
The intermediates and desired compounds in the above preparations may be isolated and purified by a conventional purification method in organic synthetic chemistry such as neutralization, filtration, extraction, washing, drying, concentration, recrystallization, and each type of chromatography. The intermediates may be also used in the next reaction without any specific purification.
An optically-active product of the present compound can be prepared from an optically-active starting material or intermediate, or by the optical resolution of the racemate of a final product. The optical resolution method includes a physical separation method with optically-active column, and a chemical separation method such as a fractional crystallization method. A diastereomer of the present compound can be prepared by, for example, a fractional crystallization method.
The pharmaceutically acceptable salt of the compound of formula (1) can be prepared by, for example, mixing the compound of formula (1) with a pharmaceutically acceptable acid in a solvent such as water, methanol, ethanol, and acetone.
The applicable cancer type for the anti-tumor agent of the present invention includes hematopoietic tumor and solid cancer, but is not limited thereto. Specific examples of the hematopoietic tumor include acute leukemia, chronic lymphatic leukemia, chronic myelocytic leukemia, polycythemia vera, malignant lymphoma, and myeloma, and specific examples of the solid cancer include brain tumor, head and neck cancer, esophageal cancer, thyroid cancer, small cell lung cancer, non-small cell lung cancer, breast cancer, stomach cancer, gallbladder or bile duct cancer, liver cancer, pancreatic cancer, colon cancer, rectal cancer, ovarian cancer, chorioepithelioma, endometrial cancer, cervical cancer, urothelial cancer, renal cell cancer, prostate cancer, testicular tumor, Wilms' tumor, malignant melanoma, neuroblastoma, osteosarcoma, Ewing's sarcoma, and soft tissue sarcoma.
The anti-tumor agent is used for the prophylaxis and/or treatment of a cancer, and is expected to produce the reduction or disappearance of carcinoma or inhibit the growth of carcinoma down to a certain level. The "prophylaxis" used herein means the administration of the active ingredient of the present invention to a healthy subject who does not develop a disease. For example, the prophylaxis is intended to prevent the development of a disease. The "treatment" used herein means the administration of the active ingredient of the present invention to a person diagnosed with the development of a disease by a doctor (i.e. a patient). For example, the treatment is intended to alleviate a disease or symptom thereof, inhibit the growth of carcinoma, or improve the condition of a patient to the previous condition before

a disease is developed. Also, even if an anti-tumor agent is administered for the purpose of preventing the worsening of a disease or symptom thereof or the growth of carcinoma, the administration is referred to as "treatment" when the subject to be administered is a patient.

The preparation comprising a compound of formula (1) or a pharmaceutically acceptable salt thereof in the pharmaceutical composition as an active ingredient shows a potent anti-tumor effect both alone and in combination with other agent used for the treatment or prophylaxis of diseases such as cancer (e.g., an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent).

Examples of the anti-cancer agent used in combination include a chemotherapeutic agent, a hormonal therapeutic agent, an angiogenesis inhibitor, an immunotherapeutic agent, a kinase inhibitor, an antibody medicine, a proteasome inhibitor, a HDAC inhibitor, a PARP inhibitor, a thalidomide analog, and a retinoic acid analog.

Examples of the chemotherapeutic agent include an alkylating agent, an anti-metabolite, an anti-cancer antibiotic, a microtubule inhibitor, a topoisomerase inhibitor, a platinum agent, and a chemotherapeutic agent other than the foregoings.

Examples of the alkylating agent include bendamustine, busulfan, carmustine, cyclophosphamide, streptozocin, dacarbazine, procarbazine, ifosfamide, melphalan, nimustine, ranimustine, and temozolomide. The alkylating agent is preferably temozolomide.

Examples of the anti-metabolite include azacitidine, capecitabine, cladribine, clofarabine, cytarabine, doxifluridine, enocitabine, fludarabine, 5-fluorouracil drug (e.g., fluorouracil, tegafur, trifluridine), gemcitabine, hydroxycarbamide, mercaptopurine, methotrexate, nelarabine, pemetrexed, forodesine, and pentostatin. The anti-metabolite is preferably gemcitabine and 5-fluorouracil drug.

Examples of the anti-cancer antibiotic include aclarubicin, actinomycin D, amrubicin, bleomycin, daunorubicin, doxorubicin, epirubicin, idarubicin, mitomycin C, peplomycin, and pirarubicin.

Examples of the microtubule inhibitor include docetaxel, paclitaxel, cabazitaxel, eribulin, vinblastine, vincristine, vindesine, and vinorelbine. The microtubule inhibitor is preferably docetaxel and paclitaxel.

Examples of the topoisomerase inhibitor include etoposide, irinotecan, nogitecan, and sobuzoxane. The topoisomerase inhibitor is preferably irinotecan.

Examples of the platinum agent include carboplatin, cisplatin, oxaliplatin, nedaplatin, and miriplatin. The platinum agent is preferably cisplatin.

Examples of the chemotherapeutic agent other than the foregoings include mitoxantrone, trabectedin, and L-asparaginase.

Example of the hormonal therapeutic agent include an estrogen receptor modulator, an androgen receptor modulator, a LH-RH agonist, a LH-RH antagonist, an aromatase inhibitor, an androgen synthesis inhibitor, estramustine, medroxyprogesterone, mepitiostane, and octreotide.

Examples of the estrogen receptor modulator include tamoxifen, toremifene, raloxifene, and fulvestrant. The estrogen receptor modulator is preferably tamoxifen, toremifene, and raloxifene.

Examples of the androgen receptor modulator include chlormadinone, bicalutamide, flutamide, and enzalutamide. The androgen receptor modulator is preferably bicalutamide and enzalutamide.

Examples of the LH-RH agonist include goserelin, buserelin, and leuprorelin.

Examples of the LH-RH antagonist include degarelix.

Examples of the aromatase inhibitor include anastrozole, letrozole, and exemestane.

Examples of the androgen synthesis inhibitor include abiraterone.

Examples of the angiogenesis inhibitor include bevacizumab, ramucirumab, and aflibercept. The angiogenesis inhibitor is preferably bevacizumab.

Examples of the immunotherapeutic agent include krestin, picibanil, ubenimex, lentinan, interferon, interleukin, macrophage colony stimulating factor, granulocyte colony stimulating factor, erythropoietin, an anti-CTLA-4 antibody (e.g., ipilimumab, tremelimumab), an anti-PD-1 antibody (e.g., nivolumab, pembrolizumab), an anti-PD-Ll antibody (e.g., avelumab, atezolizumab), and a Toll-like receptor agonist. The immunotherapeutic agent is preferably an anti-PD-1 antibody.

Examples of the kinase inhibitor include alectinib, afatinib, axitinib, bosutinib, crizotinib, vemurafenib, dabrafenib, dasatinib, erlotinib, everolimus, gefitinib, ibrutinib, imatinib, lapatinib, ruxolitinib, nilotinib, osimertinib, pazopanib, regorafenib, lenvatinib, sorafenib, sunitinib, ceritinib, vandetanib, temsirolimus, and trametinib. The kinase inhibitor is preferably sunitinib, sorafenib, regorafenib, pazopanib, dasatinib, imatinib, and lapatinib.

Examples of the antibody medicine include cetuximab, gemtuzumab ozogamicin, mogamulizumab, ofatumumab, panitumumab, pertuzumab, rituximab, trastuzumab, alemtuzumab, daratumumab, elotuzumab, brentuximab, and necitumumab.

Examples of the proteasome inhibitor include bortezomib, carfilzomib, and ixazomib.

Examples of the HDAC inhibitor include vorinostat, entinostat, belinostat, panobinostat, and romidepsin.

Examples of the PARP inhibitor include olaparib, iniparib, and veliparib.

Examples of the thalidomide analog include thalidomide, lenalidomide, and pomalidomide.

Examples of the retinoic acid analog include tretinoin, bexarotene, and tamibarotene.

Examples of the anti-diabetic agent include biguanide drug, and thiazolidine derivative.

Examples of the biguanide drug include metformin, buformin, and phenformin.

Examples of the thiazolidine derivative include pioglitazone, and rosiglitazone.

Examples of the agent for treating dyslipidemia include an HMG-CoA reductase inhibitor and a cholesterol absorption inhibitor.

Examples of the HMG-CoA reductase inhibitor include rosuvastatin, pitavastatin, atorvastatin, cerivastatin, fluvastatin, simvastatin, pravastatin, and lovastatin. The HMG-CoA reductase inhibitor is preferably fluvastatin, simvastatin, atorvastatin, and lovastatin.

Examples of the cholesterol absorption inhibitor include ezetimibe.

Examples of the agent for treating multiple sclerosis include fingolimod and natalizumab. The agent is preferably fingolimod.

Examples of the steroidal anti-inflammatory agent include hydrocortisone, prednisolone, triamcinolone, and dexamethasone.

Examples of the non-steroidal anti-inflammatory agent include aspirin, ethenzamide, diflunisal, loxoprofen, ibuprofen, diclofenac, indomethacin, meloxicam, etodolac, and celecoxib.

Examples of the anti-fungal agent include isoconazole, bifonazole, lanoconazole, ketoconazole, luliconazole, clotrimazole, terbinafine, butenafine, neticonazole, miconazole, amphotericin B, fluconazole, fosfluconazole, micafungin, and salinomycin.

The anti-cancer agent, anti-diabetic agent, agent for treating dyslipidemia, agent for treating multiple sclerosis, steroidal anti-inflammatory agent, non-steroidal anti-inflammatory agent, and anti-fungal agent used in combination may be in the form of a pharmaceutically acceptable salt thereof. Examples thereof include an inorganic acid salt such as hydrochloride, hydrobromide, sulfate, phosphate, and nitrate; and an organic acid salt such as acetate, propionate, oxalate, succinate, lactate, malate, tartrate, citrate, maleate, fumarate, methanesulfonate, p-toluenesulfonate, benzenesulfonate, and ascorbate.

The anti-cancer agent, anti-diabetic agent, agent for treating dyslipidemia, agent for treating multiple sclerosis, steroidal anti-inflammatory agent, non-steroidal anti-inflammatory agent, anti-fungal agent or a pharmaceutically acceptable salt thereof used in combination may be in the form of a hydrate. Also, when the agents can form a solvate, they may be in the form of a solvate. The solvate may be in the form or a hydrate or a non-hydrate. The solvate is preferably a hydrate. Examples of the solvent in the non-hydrate include an alcohol (e.g., methanol, ethanol, n-propanol), and dimethylformamide.

The commercially-available anti-cancer agent, anti-diabetic agent, agent for treating dyslipidemia, agent for treating multiple sclerosis, steroidal anti-inflammatory agent, non-steroidal anti-inflammatory agent, anti-fungal agent, or a pharmaceutically acceptable salt thereof for combination is easily available from manufacturers such as chemical manufacturer

The compound of formula (1) or a pharmaceutically acceptable salt thereof and an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, or a pharmaceutically acceptable salt thereof may be used in any combination. For example, one or more of the compound of formula (1) or a pharmaceutically acceptable salt thereof and one or more agents selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof may be used in any combination.

In addition, an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof may be combined two or more, respectively. For example, the compound of formula (1) or a pharmaceutically acceptable salt thereof and one or more agents selected from the group consisting of two or more anti-cancer agents, two or more anti-diabetic agents, two or more agents for treating dyslipidemia, two or more agents for treating multiple sclerosis, two or more steroidal anti-inflammatory agents, two or more non-steroidal anti-inflammatory agents, two or more anti-fungal agents, and pharmaceutically acceptable salts thereof may be used in any combination.

The pharmaceutical composition of the present invention may be formulated into a suitable dosage form and administered orally or parenterally. Examples of the dosage form include a tablet, a capsule, a powder, a granule, a solution, a suspension, an injection, a patch, and a poultice, but the dosage form is not limited thereto. The preparation is formulated using pharmaceutically acceptable additive(s) according to a known method.

As appropriate, an additive such as an excipient, a disintegrant, a binder, a fluidizer, a lubricant, a coating

agent, a solubilizer, a solubilizing agent, a thickening agent, a dispersant, a stabilizing agent, a sweetening agent, and a flavor may be used. Specific examples thereof include lactose, mannitol, crystalline cellulose, low substituted hydroxypropylcellulose, corn starch, partly pregelatinized starch, carmellose calcium, croscarmellose sodium, hydroxypropylcellulose, hydroxypropylmethylcellulose, polyvinylalcohol, magnesium stearate, sodium stearyl fumarate, polyethylene glycol, propylene glycol, titanium oxide, and talc.

The administration timing of the compound of formula (1) or a pharmaceutically acceptable salt thereof and a combined agent is not limited, and they may be administered simultaneously or administered with time-interval to a subject. The compound of formula (1) or a pharmaceutically acceptable salt thereof may be administered before, simultaneously with, or after the administration of a combined agent. In addition, the present compound and a combined agent may be used in the form of a combination drug. The dosage of the combined agent may be optionally determined based on the dosage in the clinical use. Also, the mixing ratio of the present compound and a combined agent may be optionally determined depending on the subject to be administered, the administration route, the disease to be treated, the symptom, and a combination thereof. For example, when the subject is human, the combined medicine may be used in an amount of 0.01 to 100 parts by weight relative to 1 part by weight of the present compound. In addition, a drug (a combined agent) such as an antiemetic, a sleep inducing agent, and an anticonvulsant may be used in combination with the present compound to inhibit side effects thereof.

The dosage can vary according to each compound and various conditions such as patient's disease, age, body weight, sex, symptom, and administration route. Typically, the present compound is administered to an adult (body weight: 50 kg) at a dose of 0.1 to 1000 mg/day, preferably at a dose of 0.1 to 300 mg/day, which may be administered once a day or 2 or 3 times a day. In addition, the present compound may be administered once in several days to several weeks.

In the present invention, there is also provided a medicine comprising a combination agent of at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof and the compound of formula (1) or a pharmaceutically acceptable salt thereof, and a label, instructions and/or a package insert that indicate the direction for the combination agent; or

a medicine comprising at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof, the compound of formula (1) or a pharmaceutically acceptable salt thereof, and a label, instructions and/or a package insert that indicate the direction for them in a use for the treatment of cancer.

In such case, an agent comprising the compound of formula (1) or a pharmaceutically acceptable salt thereof and an agent comprising an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, or a pharmaceutically acceptable salt thereof may be covered by the medicine of the present invention where each of them is a separate preparation or, preferably, a separate unit dosage form.

With regard to the instructions for use of the combination of both drugs, examples thereof are information concerning use method and dose such as administering amount and frequency of each drug per day, administering route. When the pharmaceutical medicine of the present invention contains only one of the drugs, information concerning another drug to be used together may be mentioned in the package insert.

The pharmaceutical composition has a potent inhibitory effect on the self-renewal ability of CSCs, and thus is expected to be used as a novel anti-tumor agent which can inhibit persistent proliferation, metastasis and recurrence of malignant tumors derived from CSCs.

In the present invention, the interaction between drugs used in combination was determined according to the following calculation procedure.

The cell survival rate or sphere formation rate at the addition of Drug A was defined as La, and the cell survival rate or sphere formation rate at the addition of Drug B was defined as Lb. In such case, it is found that the theoretical cell survival rate or sphere formation rate of the combination of Drug A and Drug B is calculated by multiplying La and Lb together. That is, the theoretical cell survival rate or sphere formation rate of the combination (Lc) is calculated according to the following formula.

$$(Lc) = (La) \times (Lb)$$

On the other hand, the measured cell survival rate or sphere formation rate of the combination of Drug A and Drug B (Ld) is calculated by measuring the cell survival rate or sphere formation rate of the combination. The interaction between drugs used in combination can be determined based on the value calculated by dividing (Lc) by (Ld). That is,

when (Ld)/(Lc) is 1, Drugs A and B do not interact with each other. Thus, it is considered that they are produce an additive effect.

When (Ld)/(Lc) is less than 1 (preferably, less than 0.8), it means that the actual effect is higher than theoretical effect. Thus, the interaction of Drugs A and B is considered as being synergistic.

When (Ld)/(Lc) is more than 1, it means that the actual effect is lower than theoretical effect. Thus, the interaction of Drugs A and B is considered as being antagonistic.

Also, the present compound with sphere-forming ability of cancer cells had a remarkably potent inhibitory effect on the sphere-forming ability of cancer cells with gene muations in Wnt/β-catenin pathway indicated by the following examples.

Examples of the gene mutation in Wnt/β-catenin pathway include APC gene mutation (including, but is limited to, amino acid mutation p.Q789*, p.Q1338*, p.T1556fs*3, p.R2333K, p.K1561N, p.E853*, p.M1431fs*42, p.Q1429*; p.R1450*, p.R876*, p.R213*, p.R1114*, p.E1309fs*4, p.Q1378*, p.R232*, p.E1309fs*4, p.R216*, p.Q1367*), CTNNB1 gene mutation (including, but is limited to, amino acid mutation p.S45del; p.T41A, p.S45F, p.S45P, p.S37F, p.S33C, p.S37C, p.D32Y, p.S45F, p.S33F, p.T41I), AXIN1 gene mutation (including, but is limited to, amino acid mutation p.L396M; p.R146*, p.G508fs*197, p.S611*, p.G265*, p.E406*, p.W85*, p.R533*, p.Q190*, p.R395C, p.E465*), AXIN2 gene mutation (including, but is limited to, amino acid mutation p.R538W; p.G665fs*24, p.N666fs*41, p.E19fs*19, p.E405fs*56, p.R659W, p.A603P, p.V40M, p.T107A, p.F805L, p.R671H), TCF4 gene mutation (including, but is limited to, p.P647fs*21, p.R174*, p.R174Q, p.D197N, p.P177S, p.R385Q, p.S464I).

Examples of the cancer type with the gene mutations include colorectal cancer, small intestine cancer, lung cancer, breast cancer, stomach cancer, soft tissue tumor, pancreatic cancer, liver cancer, endometrial cancer, ovarian cancer, adrenal cancer, urothelial cancer, bile duct cancer, renal cell cancer, brain tumor, and pituitary adenoma. The cancer type is not limited thereto as long as it has the above gene mutations.

As shown in the results of the following Test Example 10, it was demonstrated that the present compound showed a remarkably potent anti-tumor effect in the treatment of cancer with the above gene mutations.

## EXAMPLES

Hereinafter, the invention is illustrated in more detail with Reference Examples, Examples, and Test Examples, but the invention should not be limited thereto. The compound names as shown in the following Reference Examples and Examples do not necessarily follow the IUPAC nomenclature system.

The following abbreviations may be used herein.

THF: tetrahydrofuran
TFA: trifluoroacetic acid
TBSCl: tert-butyldimethylchlorosilane
DAST: N,N-diethylaminosulfur trifluoride
DMF: N,N-dimethylformamide
WSCI • HCl: 1-(3-dimethylaminopropyl)-3-ethylcarbodiimide hydrochloride
HOBt: 1-hydroxybenzotriazole
HATU: 0-(7-azabenzotriazol-1-yl)-N,N,N',N'-tetramethyluroni um hexafluorophosphate
Me: methyl
Et: ethyl
Ac: acetyl
TBS: tert-butyldimethylsilyl
Boc: tert-butoxycarbonyl
THP: tetrahydropyranyl
DMAP: N,N-dimethylaminopyridine

LC/MS analysis condition in the compound identification is as follows. The compounds of Reference Examples or Examples were analyzed under LC/MS analysis condition A, B, or C described below.

| Analysis condition A: LC/MS | |
|---|---|
| MS | detector Perkin-Elmer Sciex API 150EX Mass spectrometer (40eV) |
| HPLC | Shimadzu LC 8A |
| Column | Shiseido CAPCELL PAK C18 Type-MG (5μm, 4.6 mm X 50 mm), Cat.No.-90105 or Shiseido CAPCELL PAK C18 Type-ACR (5μm, 4.6 mm X 50 mm), Cat.No.-91105 |

(continued)

| Analysis condition A: LC/MS | |
|---|---|
| Detector | UV: 220 nm |
| Solvent | A: 0.035% TFA/$CH_3CN$, B: 0.05% TFA/$H_2O$ |
| Flow rate | 3.5 mL/min |
| Gradient condition | 0.0-0.5 min A 10%, 0.5-4.2 min Linear gradient from A 10% to 99%, 4.2-6.3 min A 99% |

| Analysis condition B: LC/MS | |
|---|---|
| Detection device | ACQUITY® SQ deteceter (Waters) |
| HPLC | ACQUITY UPLC® system |
| Column | Waters ACQUITY UPLC® BEH C18 (1.7 μm, 2.1 mm X 30 mm) |
| Solvent | 0.06% formic acid/$H_2O$, B solution: 0.06% formic acid/$CH_3CN$ |
| Gradient condition | 0.0-1.3 min Linear gradient from B 2% to 96% |
| Flow rate | 0.8 mL/min |
| Detector | UV: 220 nm and 254 nm |

| Analysis condition C: LC/MS | |
|---|---|
| MS | detector Perkin-Elmer Sciex API 150EX Mass spectrometer (40eV) |
| HPLC | Agilent 1100 Series |
| Column | YMC CombiScreen ODS-A (S-5 μm, 12 nm) 50 X 4.6 mm |
| Detector | UV: 220 nm |
| Solvent | A: 0.035% TFA/$CH_3CN$, B: 0.05% TFA/$H_2O$, |
| Flow rate | 3.5 mL/min |
| Gradient condition | 0.0-1min A 10%, 1-4.7min Linear gradient from A 10% to 99%, 4.7-4.9min A 99% |

Reference Example 1

1-(3-(Trifluoromethyl)benzyl)-1H-imidazole-4-amine hydrochloride

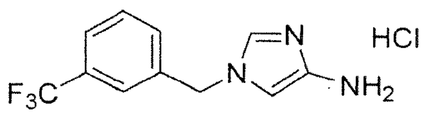

To a solution of 4-nitroimidazole (20 g) in acetonitrile (150 mL) were added potassium carbonate (26.9 g) and potassium iodide (0.074 g), and then a solution of 3-trifluoromethylbenzyl bromide (42.3 g) in acetonitrile (50 mL) was added dropwise thereto at room temperature. The mixture was stirred at 80°C for 4 hours and cooled to room temperature, and water was added thereto, and then the mixture was extracted with ethyl acetate. The organic layer was dried over anhydrous sodium sulfate, filtrated, and then concentrated *in vacuo*. To a solution of the resulting crude product (46.1 g) in ethyl acetate (500 mL) was added rhodium-carbon (23.1 g), and the mixture was stirred at room temperature under hydrogen atmosphere. After 20 hours, the reaction mixture was filtrated through Celite®. To the resulting filtrate was added 4 mol/L hydrochloric acid-dioxane (55.3 mL), and the mixture was stirred at room temperature. The resulting precipitate was collected on a filter and washed with ethyl acetate to give the title compound (22.8 g).

LC-MS, condition B ([M+H]$^+$/Rt (min)): 242.1/0.529

Reference Examples 2-6

The compounds of Reference Examples 2-6 were prepared from each corresponding starting compound according to a similar process to that of Reference Example 1.

| Reference Example | Chemical Structural Formula | LC-MS, condition B: [M+H]$^+$/Rt (min) |
|---|---|---|
| 2 | 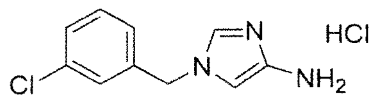 | 208.1/0.461 |
| 3 | 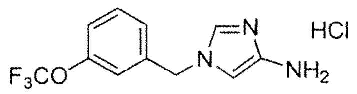 | 258.1/0.564 |
| 4 | 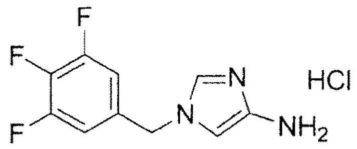 | 228.1/0.473 |
| 5 | 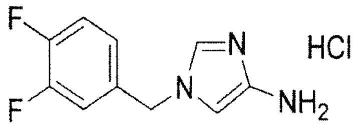 | 210.1/0.424 |
| 6 | 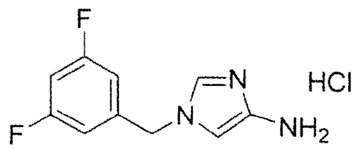 | 210.1/0.422 |

Reference Example 7-1 2-(2-(5-Bromo-2-methoxyphenoxy)ethoxy)tetrahydro-2*H*-pyrane

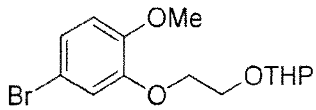

To a solution of 5-bromo-2-methoxyphenol (10.0 g) in DMF (50 mL) were added 2-(2-bromoethoxy)tetrahydro-2H-pyrane (10.8g) and potassium carbonate (8.86 g), and the mixture was stirred at 80°C for 2.5 hours. To the reaction mixture was added water, and the mixture was extracted with ethyl acetate. The organic layer was washed with brine, dried over magnesium sulfate, filtrated, and then concentrated *in vacuo* to give the title compound (15.1 g).
$^1$H-NMR (400 MHz, $CDCl_3$) $\delta$ 7.12 (1H, d, *J* = 2.0 Hz), 7.05 (1H, dd, *J* = 8.4, 2.0 Hz), 6.75 (1H, d, *J* = 8.4 Hz), 4.72 (1H, t, *J* = 3.6 Hz), 4.26-4.19 (2H, m), 4.14-4.03 (1H, m), 3.92-3.82 (2H, m), 3.85 (3H, s), 3.57-3.50 (1H, m), 1.90-1.78 (1H, m), 1.78-1.70 (1H, m), 1.68-1.53 (4H, m).

Reference Example 7-2

Ethyl (*E*)-3-(4-methoxy-3-(2-((tetrahydro-2H-pyran-2-yl)oxy)ethoxy)phenyl)acrylate

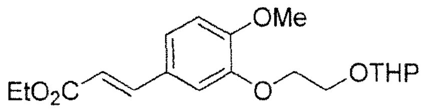

To a solution of the compound of Reference Example 7-1 (14.0 g) in propionitrile (120 mL) were added ethyl acrylate (6.9 mL), N,N-diisopropylethylamine (14.7 mL), palladium acetate (0.48 g), and tris(o-tolyl)phosphine (1.29 g), and the mixture was stirred at 100°C for 13 hours. To the reaction mixture was added water, and the mixture was extracted with ethyl acetate. The organic layer was washed with brine, dried over magnesium sulfate, filtrated, and then concentrated *in vacuo.* The residue was purified by silica gel column chromatography (hexane/ethyl acetate) to give the title compound (8.0 g).
$^1$H-NMR (400 MHz, $CDCl_3$) δ 7.62 (1H, d, *J* = 16.0 Hz), 7.19 (1H, d, *J* = 2.0 Hz), 7.12 (1H, dd, *J* = 8.4, 2.0 Hz), 6.87 (1H, d, *J* = 8.4 Hz), 6.31 (1H, d, *J* = 16.0 Hz), 4.73 (1H, t, *J* = 3.6 Hz), 4.31-4.22 (4H, m), 4.15-4.08 (1H, m), 3.93-3.86 (5H, m), 3.57-3.51 (1H, m), 1.88-1.81 (1H, m), 1.78-1.71 (1H, m), 1.68-1.53 (4H, m), 1.35 (3H, t, *J* = 6.8 Hz).

Reference Example 7-3

(E)-3-(4-Methoxy-3-(2-((tetrahydro-2H-pyran-2-yl)oxy)ethoxy)phenyl)acrylic acid

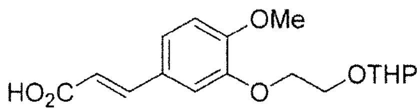

To a solution of the compound of Reference Example 7-2 (3.6 g) in THF/methanol (20 mL/20 mL) was added 2 mol/L aqueous sodium hydroxide solution (15 mL), and the mixture was stirred at 60°C for 7 hours. To the reaction mixture was added aqueous hydrochloric acid solution to adjust pH to 5.0, and the mixture was extracted with ethyl acetate. The organic layer was washed with brine, dried over magnesium sulfate, filtrated, and then concentrated in *vacuo* to give the title compound (3.1 g).
$^1$H-NMR (400 MHz, $CDCl_3$) δ 7.72 (1H, d, *J* = 15.6 Hz), 7.23 (1H, d, *J* = 1.6 Hz), 7.15 (1H, dd, *J* = 8.4, 2.0 Hz), 6.89 (1H, d, *J* = 8.4 Hz), 6.33 (1H, d, *J* = 15.6 Hz), 4.74 (1H, t, *J* = 3.6 Hz), 4.33-4.25 (2H, m), 4.15-4.07 (1H, m), 3.95-3.86 (5H, m), 3.58-3.53 (1H, m), 1.88-1.81 (1H, m), 1.79-1.71 (1H, m), 1.69-1.51 (4H, m).

Reference Example 8

The compound of Reference Example 8 was prepared from the corresponding starting compound according to a similar process to that of Reference Example 7.

| Reference Example | Chemical Structural Formula | $^1$H-NMR (400 MHz, $CDCl_3$) |
|---|---|---|
| 8 | 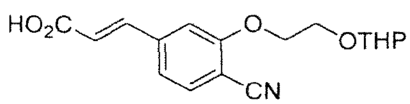 | δ7.74 (1H, d, *J* = 15.6 Hz), 7.61 (1H, d, *J* = 8.0 Hz) , 7.24-7.19 (2H, m), 6.53 (1H, d, *J* = 15.6 Hz), 4.78-4.76 (1H, m), 4.40-4.33 (2H, m), 4.18-4.13 (1H, m), 3.97-3.89 (2H, m), 3.60-3.56 (1H, m), 1.87-1.73 (2H, m), 1.67-1.51 (4H, m). |

Reference Example 9

(*E*)-*N*-(1-(3-Chlorobenzyl)-1H-imidazol-4-yl)-3-(4-methoxy-3-(2-((tetrahydro-2H-pyran-2-yl)oxy)ethoxy)phenyl)acrylamide

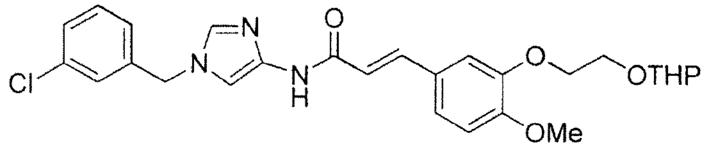

To a solution of a compound of Reference Example 2 (1.20 g) in DMF (30 mL) were added a compound of Reference Example 7-3 (1.90 g), WSCI·HCl (1.13 g), HOBt (0.80 g), and triethylamine (2.2 mL) and the mixture was stirred at room temperature for 2 hours. To the reaction mixture was added water and the mixture was extracted with chloroform. The organic layer was washed with brine, dried over magnesium sulfate, filtrated, and then concentrated *in vacuo.* The residue was purified by silica gel column chromatography (chloroform/methanol) to give the title compound (1.25 g).
$^1$H-NMR (400 MHz, $CDCl_3$) δ 8.84 (1H, s), 7.64 (1H, d, J = 16.0 Hz), 7.48-7.43 (2H, m), 7.35-7.29 (2H, m), 7.23-7.17 (2H, m), 7.14-7.10 (2H, m), 6.87 (1H, d, *J* = 8.0 Hz), 6.43 (1H, d, *J* = 16.0 Hz), 5.10 (2H, s), 4.72-4.69 (1H, m), 4.29-4.24 (2H, m), 4.16-4.07 (1H, m), 3.94-3.85 (5H, m), 3.56-3.51 (1H, m), 1.88-1.80 (1H, m), 1.78-1.71 (1H, m), 1.64-1.52 (4H, m).

Reference Examples 10-12

The compounds of Reference Examples 10-12 were prepared from each corresponding starting compound according to a similar process to that of Reference Example 9.

| Reference Example | Chemical Structural Formula | LC-MS, condition B: $[M+H]^+$/Rt (min) |
|---|---|---|
| 10 | 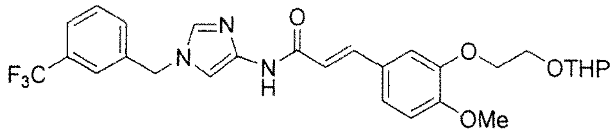 | 546.8/0.968 |
| 11 | 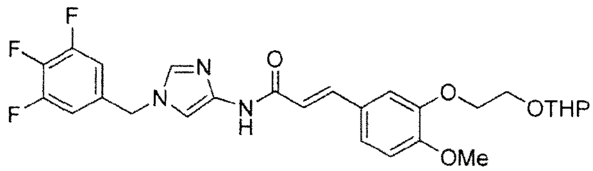 | 532.3/0.964 |
| 12 | 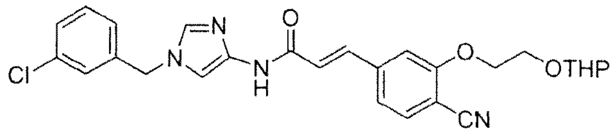 | 507.2/1.010 |

Reference Example 13

Methyl 6-({1-[3-(trifluoromethyl)benzyl]-1H-imidazol-4-yl}carbamoyl) nicotinate

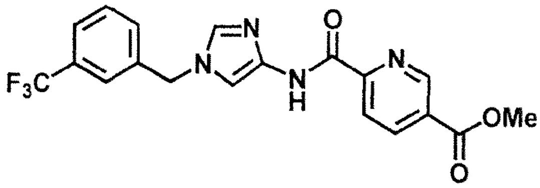

The title compound was prepared from the compound of Reference Example 1 and the corresponding starting compound according to a similar process of that of Reference Example 9.

LC-MS, condition B ([M+H]$^+$/Rt (min)): 404.9/0.901

Reference Example 14-1

Methyl 1-(3,4,5-trifluorobenzyl-1H-imidazole-4-carboxylate

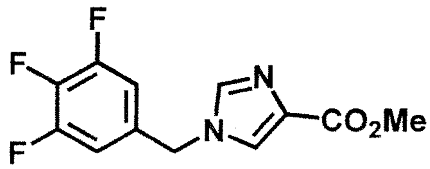

To a solution of methyl 1H-imidazole-4-carboxylate (14.0 g) in acetonitrile (200 mL) were added potassium carbonate (19.9 g) and potassium iodide (0.092 g), 3,4,5-trifluorobenzyl bromide (14.6 mL) was added dropwise thereto at room temperature, and then the mixture was stirred at 70°C for 6 hours. The mixture was cooled to room temperature, and to the reaction mixture was added water, and then the mixture was extracted with ethyl acetate. The organic layer was dried over anhydrous magnesium sulfate, filtrated, and then concentrated *in vacuo*. The resulting crude product was washed with hexane/ethyl acetate (1/2, 60 mL) to give the title compound (14.0 g).
LC-MS, condition B ([M+H]$^+$/Rt (min)): 271.4/0.725

Reference Example 14-2

1-(3,4,5-Trifluorobenzyl)-1H-imidazole-4-carboxylic acid

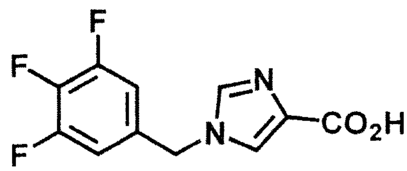

To a solution of the compound of Reference Example 14-1 (4.75 g) in methanol/THF (50 mL/50 mL) was added 2 mol/L aqueous sodium hydroxide solution (13.2 mL), and the mixture was stirred at 50°C for 5 hours. The reaction mixture was concentrated *in vacuo,* and the residue was dissolved in water, and then aqueous hydrochloric acid solution was added thereto to adjust pH to 5. The resulting precipitate was collected on a filter, washed with water and hexane, and then dried *in vacuo* at 50°C to give the title compound (4.52 g).
LC-MS, condition B ([M+H]$^+$/Rt (min)): 257.1/0.513

Reference Example 15

6-({[*tert*-Butyl(dimethyl)silyl]oxy}methyl)pyridine-3-amine

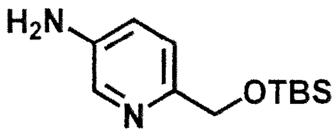

To a solution of (5-aminopyridin-2-yl)methanol (135 mg) in THF (15 mL) were added triethylamine (0.30 mL) and TBSCl (328 mg), and the mixture was stirred at room temperature for 6 hours. The solvent was removed *in vacuo,* and the resulting residue was purified by silica gel column chromatography (chloroform/methanol) to give the title compound (99 mg).
LC-MS, condition B ([M+H]$^+$/Rt (min)): 239.2/0.726

Reference Example 16

2-({[tert-Butyl(dimethyl)silyl]oxy}methyl)quinoline-6-amine

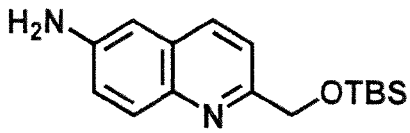

The title compound was prepared from (6-aminoquinolin-2-yl)methanol according to the process of Reference Example 15.
LC-MS, condition B ([M+H]$^+$/Rt (min)): 289.9/0.836

Reference Example 17

According to the process of Reference Example 9, the compound of Reference Example 17 was prepared from the compound of Reference Example 14-2 and the corresponding starting compound.

| Reference Example | Chemical Structural Formula | LC-MS, condition B: [M+H]$^+$/Rt (min) |
|---|---|---|
| 17 | 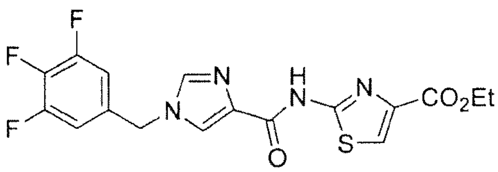 | 411.2/0.915 |

Reference Example 18-1

Methyl 5-(2-*tert*-butoxy-2-oxoethoxy)-picolinate

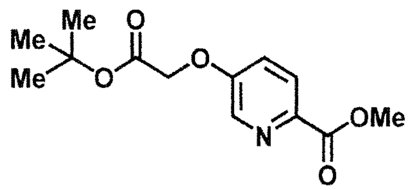

To a solution of methyl 5-hydroxy-picolinate (200 mg) in DMF (5 mL) were added potassium carbonate (361 mg) and tert-butyl bromoacetate, and the mixture was 70°C for 20 minutes. The mixture was cooled to room temperature, to the reaction mixture was added water, and then the mixture was extracted with ethyl acetate. The organic layer was washed with brine twice, dried over anhydrous magnesium sulfate, filtrated, and then concentrated *in vacuo* to give the title compound (320 mg).
LC-MS, condition B ([M+H]$^+$/Rt (min)): 268.2/0.777

Reference Example 18-2

{[6-(Methoxycarbonyl)pyridin-3-yl]oxy}acetic acid

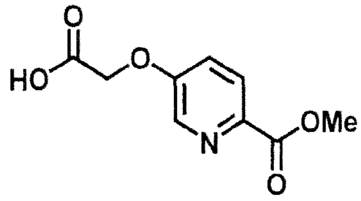

To a solution of a compound of Reference Example 18-1 (320 mg) in dichloromethane (4 mL) was added TFA (2 mL) and the mixture was stirred at room temperature. The solvent was removed to give the title compound (253 mg).
LC-MS, condition B ([M+H]$^+$/Rt (min)): 212.1/0.394

Reference Example 18-3

Methyl 5-(2-oxo-2-{[1-(3,4,5-trifluorobenzyl)-1H-imidazol-4-yl]amino}ethoxy)-picolinate

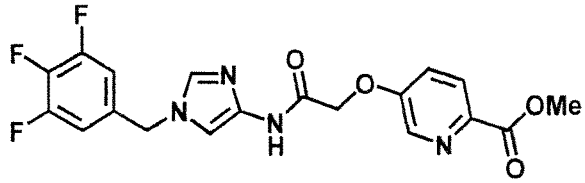

The title compound was prepared from the compounds of Reference Examples 4 and 18-2 according to the process of Reference Example 9.
LC-MS, condition B ([M+H]$^+$/Rt (min)): 421.2/0.731

Reference Example 19-1

Methyl 6-chloro-5-(dibromomethyl)-nicotinate

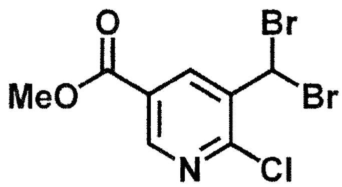

To a suspension of methyl 6-chloro-5-methyl-nicotinate (467 mg) in carbon tetrachloride (25 mL) were added N-bromosuccinimide (1.34 g) and benzoyl peroxide (218 mg), and the mixture was stirred at 100°C for 7.5 hours. The mixture was cooled to room temperature, and to the reaction mixture were added saturated aqueous sodium thiosulfate solution and water, and the reaction mixture was extracted with chloroform. The organic layer was washed with brine, dried over anhydrous sodium sulfate, filtrated, and then concentrated *in vacuo.* The residue was purified by silica gel column chromatography (hexane/ethyl acetate) to give the title compound (833 mg).
LC-MS, condition B ([M+H]$^+$/Rt (min)): 341.9/1.011

Reference Example 19-2

Methyl 6-chloro-5-formyl-nicotinate

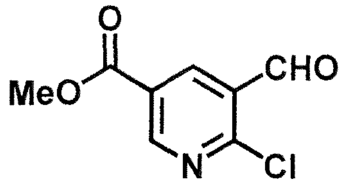

To a solution of the compound of Reference Example 19-1 (2.71 g) in acetonitrile (40 mL)/water (20 mL) was added silver nitrate (6.70 g), and the mixture was stirred at 100°C for 3 hours. The insoluble product was removed by filtration, and the solvent was removed. To the residue was added saturated aqueous sodium hydrogen carbonate solution to adjust pH to 8, and the mixture was extracted with ethyl acetate. The organic layer was washed with brine, dried over anhydrous sodium sulfate, filtrated, and then concentrated *in vacuo* to give the title compound (0.84 g).
LC-MS, condition B ($[M+H]^+$/Rt (min)): 200.0/0.671

Reference Example 19-3

Methyl 6-chloro-5-(difluoromethyl)-nicotinate

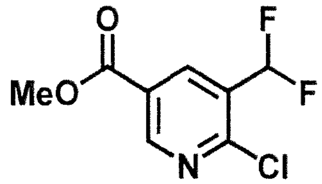

To a solution of the compound of Reference Example 19-2 (0.84 g) in dichloromethane (20 mL) was added DAST (1.11 mL) with ice-cooling, and the mixture was stirred with ice-cooling for 30 minutes. To the reaction mixture was added saturated aqueous sodium hydrogen carbonate solution to adjust pH to 8, and the mixture was extracted with chloroform. The organic layer was washed with brine, dried over anhydrous sodium sulfate, filtrated, and then concentrated *in vacuo*. The residue was purified by silica gel column chromatography (hexane/ethyl acetate) to give the title compound (0.45 g).
LC-MS, condition B ($[M+H]^+$/Rt (min)): 222.0/0.828

Reference Example 19-4

Methyl 5-(difluoromethyl)-6-(ethenyl)-nicotinate

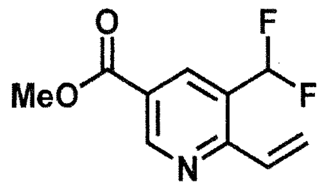

To a solution of the compound of Reference Example 19-3 (450mg) in a mixture of 1,2-dimethoxyethane (15 mL)/water (1.5 mL) were added vinylboronic acid pinacol ester (0.521 mL), tetrakis(triphenylphosphine)palladium (235 mg), and potassium carbonate (702 mg), and the mixture was stirred at 100°C for 3.5 hours. The reaction mixture was cooled to room temperature, water was added thereto, and the reaction mixture was extracted with chloroform. The organic layer was washed with brine, dried over sodium sulfate, filtrated, and then concentrated *in vacuo*. The residue was purified by silica gel column chromatography (hexane/ethyl acetate) to give the title compound (240 mg).
LC-MS, condition B ($[M+H]^+$/Rt (min)): 214.1/0.842

Reference Example 19-5

Methyl 5-(difluoromethyl)-6-(formyl)-nicotinate

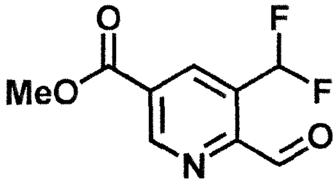

To a solution of the compound of Reference Example 19-4 (243 mg) in a mixture of acetone (5 mL)/water (2.5 mL) were added sodium periodate (488 mg) and osmium tetroxide (2.5 wt% in tert-butanol, 0.716 mL), and the mixture was stirred at room temperature for 8 hours. To the reaction mixture was added water, and the reaction mixture was extracted with ethyl acetate. The organic layer was washed with brine, dried over sodium sulfate, filtrated, and then concentrated *in vacuo.* The residue was purified by silica gel column chromatography (hexane/ethyl acetate) to give the title compound (120 mg).
LC-MS, condition B ($[M+H]^+$/Rt (min)): 216.1/0.736

Reference Example 19-6

Methyl 5-(difluoromethyl)-6-(hydroxymethyl)-nicotinate

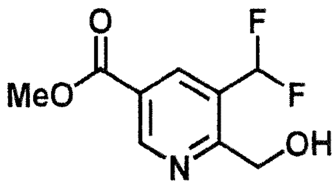

To a solution of the compound of Reference Example 19-5 (120 mg) in methanol (3 mL) was added sodium borohydride (21 mg), and the mixture was stirred at room temperature for 1 hour. To the reaction mixture were added saturated aqueous ammonium chloride solution and water, and the mixture was extracted with ethyl acetate. The organic layer was washed with brine, dried over sodium sulfate, filtrated, and then concentrated *in vacuo* to give the title compound (116 mg).
LC-MS, condition B ($[M+H]^+$/Rt (min)): 218.1/0.564

Reference Example 20

Methyl 6-(hydroxymethyl)-5-(trifluoromethyl)-nicotinate

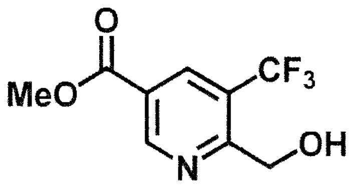

The title compound was prepared from methyl 6-chloro-5-(trifluoromethyl)-nicotinate according to the processes of Reference Examples 19-4, 19-5, and 19-6.
LC-MS, condition B ($[M+H]^+$/Rt (min)): 236.1/0.649

Reference Example 21

Methyl 5-(hydroxymethyl)pyrazine-2-carboxylate

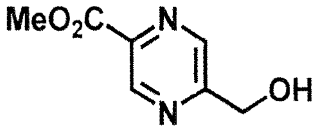

The title compound was prepared from methyl 5-chloropyrazine-2-carboxylate according to the processes of Reference Examples 19-4, 19-5, and Reference Example 19-6.
LC-MS, condition B ($[M+H]^+$/Rt (min)): 169.0/0.334

Reference Example 22

1-(3,4-Difluorobenzyl)-1H-imidazole-4-carboxylic acid

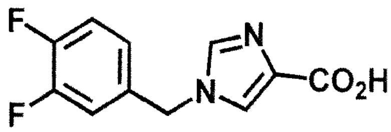

The title compound was prepared from 3,4-difluorobenzyl bromide according to the processes of Reference Examples 14-1 and 14-2.
LC-MS, condition B ($[M+H]^+$/Rt (min)): 239.1/0.460

Reference Example 23

*tert*-Butyl 6-{[(trifluoromethyl)sulfonyl]oxy}-3,4-dihydro-2,7-naphthyridine-2(1H)-carboxylate

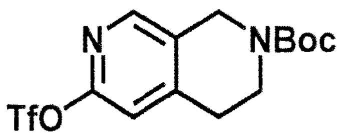

To a solution of *tert*-butyl 6-hydroxy-1,2,3,4-tetrahydro-2,7-naphthyridine-2-carboxylate (1.73 g) in pyridine (20 mL) was added trifluoromethanesulfonic anhydride (1.28 mL) with ice-cooling, and the mixture was stirred at room temperature for 2 hours. The reaction mixture was concentrated *in vacuo,* and the residue was purified by silica gel column chromatography (hexane/ethyl acetate) to give the title compound (1.72 g).
LC-MS, condition B ($[M+H]^+$/Rt (min)): 383.2/1.112

Reference Example 24

tert-Butyl 6-bromo-5-fluoro-3,4-dihydroisoquinoline-2(1H)-carboxylate

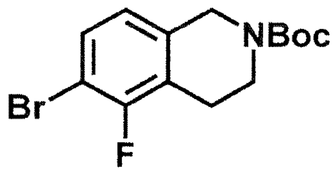

To acetic acid (15 mL) was added sodium borohydride (340 mg) at room temperature. To the reaction solution was added 6-bromo-5-fluoroisoquinoline (1.0 g), and the mixture was stirred at room temperature for 15 hours. To the reaction solution was added sodium borohydride (345 mg), and the mixture was stirred at room temperature for 1 hour. The reaction mixture was concentrated *in vacuo,* and the residue was dissolved in THF (20 mL). Di-*tert*-butyl dicarbonate (2.04g) and triethylamine (3.1 mL) were added thereto, and the mixture was stirred at room temperature for 2 hours. To the reaction mixture was added water, and the mixture was extracted with ethyl acetate. The organic layer was washed with brine, dried over sodium sulfate, filtrated, and then concentrated *in vacuo.* The residue was purified by silica gel column chromatography (hexane/ethyl acetate) to give the title compound (1.17 g).
LC-MS, condition B ($[M+H]^+$/Rt (min)): 330.2/1.213

Reference Examples 25-26

According to the process of Reference Example 24, the compounds of Reference Examples 25 and 26 were prepared from each corresponding starting compound.

| Reference Example | Chemical Structural Formula | LC-MS, condition B: $[M+H]^+$/Rt (min) |
|---|---|---|
| 25 | 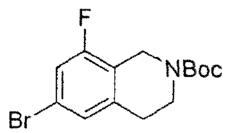 | 330.1/1.244 |
| 26 | 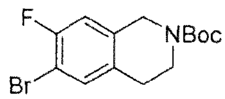 | 330.4/1.217 |

Reference Example 27-1

tert-Butyl 6-cyano-8-fluoro-3,4-dihydroisoquinoline-2(1H)-carboxylate

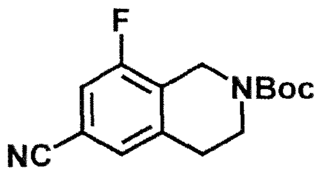

To a solution of the compound of Reference Example 25 (124 mg) in DMF (1 mL) was added tetrakis(triphenylphosphine)palladium (45 mg) and zinc cyanide (57 mg), and the mixture was stirred at 120°C for 8 hours. The reaction mixture was concentrated *in vacuo,* and the residue was purified by silica gel column chromatography (hexane/ethyl acetate) to give the title compound (48 mg).
LC-MS, condition B ($[M+H]^+$/Rt (min)): 277.2/1.048

Reference Example 27-2

2-(*tert*-Butoxycarbonyl)-8-fluoro-1,2,3,4-tetrahydroquinoline-6-carboxylic acid

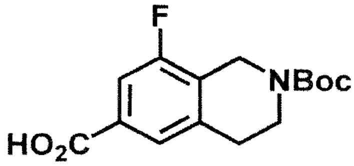

To a solution of the compound of Reference Example 27-1 (2.13 g) in 2-propanol (40 mL) were added water (10 mL) and sodium hydroxide (5 g), and the mixture was stirred at 110°C for 11 hours. The reaction mixture was concentrated *in vacuo,* and the residue was extracted with saturated aqueous sodium hydrogen carbonate solution. The aqueous layer was adjusted to acidity with sodium hydrogen sulfate and extracted with chloroform. The resulting organic layer was dried over sodium sulfate and concentrated *in vacuo* to give the title compound (2.54 g).
LC-MS, condition B ($[M+H]^+$/Rt (min)): 296.2/0.907

Reference Example 28

Methyl 6-(hydroxymethyl)-5-methyl-nicotinate

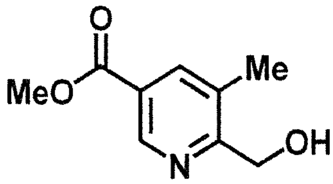

The title compound was prepared from methyl 6-chloro-5-methyl-nicotinate according to the processes of Reference Examples 19-4, 19-5, and 19-6.
LC-MS, condition B ($[M+H]^+$/Rt (min)): 182.0/0.354

Reference Example 29-1

Methyl 5-[(tert-butoxycarbonyl)amino]-6-ethenyl-nicotinate

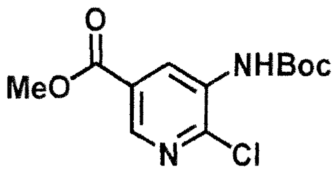

To a solution of methyl 5-amino-6-chloro-nicotinate (325 mg) in THF (10 mL) were added di-tert-butyl dicarbonate (760 mg) and DMAP (11 mg), and the mixture was stirred at room temperature for 15.5 hours. Additional di-*tert*-butyl dicarbonate (38 mg) was added thereto, and the mixture was stirred at 60°C for 45 minutes. The mixture was cooled to room temperature, and then the solvent was removed. To the residue were added methanol (5 mL) and potassium carbonate (481 mg), and the mixture was stirred at room temperature for 2.5 hours. Saturated aqueous ammonium chloride solution was added thereto, and the mixture was extracted with ethyl acetate. The organic layer was washed with brine, dried over anhydrous sodium sulfate, filtrated, and then concentrated *in vacuo.* The residue was purified by silica gel column chromatography (hexane/ethyl acetate) to give the title compound (321 mg).
LC-MS, condition B ($[M+H]^+$/Rt (min)): 287.1/0.985

Reference Example 29-2

Methyl 5-[(tert-butoxycarbonyl)amino]-6-(hydroxymethyl)-nicotinate

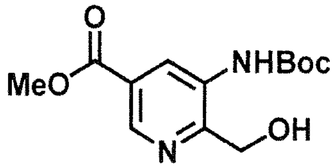

The title compound was prepared from the compound of Reference Example 29-1 according to the processes of Reference Examples 19-4, 19-5, and 19-6.
LC-MS, condition B ([M+H]+/Rt (min)): 282.8/0.761

Reference Example 29-3

2-Oxo-1,4-dihydro-2H-pyrido[3,2-d][1,3]oxazine-7-carboxylic acid

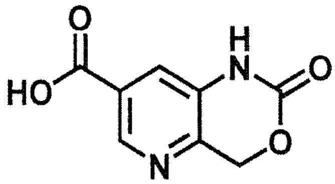

To a solution of the compound of Reference Example 29-2 (111 mg) in THF (2 mL)/methanol (4 mL) was added 2 mol/L aqueous sodium hydroxide solution (0.39 mL), and the mixture was stirred at room temperature for 16 hours. To the reaction solution was added 2 mol/L hydrochloric acid (0.25 mL) to adjust pH to 7. The reaction mixture was concentrated *in vacuo* to give the title compound (76 mg).
LC-MS, condition B ([M+H]+/Rt (min)): 195.1/0.325

Reference Examples 30-32

According to the processes of Reference Examples 27-1 and 27-2, the compounds of Reference Examples 30-32 were prepared from the compounds of Reference Examples 23, 24, and 26.

| Reference Example | Chemical Structural Formula | LC-MS: [M+H]+/Rt (min) |
|---|---|---|
| 30 | 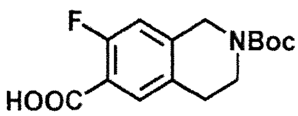 | 296.2/0.867 |
| 31 | 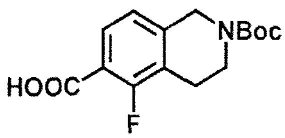 | 296.1/0.864 |
| 32 | 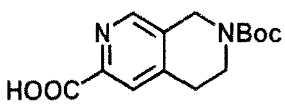 | 279.0/0.537 |

Example 1-1

(2*E*)-3-[4-(Acetylamino)phenyl]-*N*-(1-[3-(trifluoromethyl)benzyl]-1*H*-imidazol-4-yl)prop-2-enamide

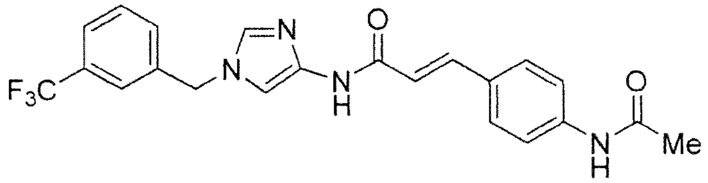

To a solution of the compound of Reference Example 1 (2.0 g) in dimethylformamide (20 mL) were added (*E*)-3-(4-acetylaminophenyl)acrylic acid (1.41 g), HATU (2.88 g), and diisopropylethylamine (2.97 mL), and the mixture was stirred at room temperature overnight. To the reaction mixture were added saturated aqueous sodium hydrogen carbonate solution and water, and then the resulting precipitate was collected on a filter and washed with water and acetonitrile. The resulting solid was purified by silica gel column chromatography (chloroform/methanol) to give the title compound (0.706 g).
$^1$H-NMR (400 MHz, DMSO-$d_6$) δ 10.51 (1H, s), 10.09 (1H, s), 7.71-7.66 (3H, m), 7.63-7.59 (4H, m), 7.47 (2H, d, *J* = 8.5 Hz), 7.40 (1H, d, *J* = 15.9 Hz), 7.36 (1H, d, *J* = 1.8 Hz), 6.74 (1H, d, *J* = 15.9 Hz), 5.28 (2H, s), 2.05 (3H, s). LC-MS, conditions ([M+H]$^+$/Rt (min)): 429.5/0.88

Example 1-2

(2*E*)-3-[4-(Acetylamino)phenyl]-*N*-{1-[3-(trifluoromethyl)benzyl]-1*H*-imidazol-4-yl)prop-2-enamide hydrochloride

To a suspension of the compound of Example 1-1 (500 mg) in ethanol was added 4 mol/L hydrochloric acid-ethyl acetate (350 pL) at 60°C, and the mixture was stirred at this temperature for 5 minutes. An oil bath was removed, a seed crystal was added thereto, and the mixture was stirred at room temperature for 40 minutes and then for 35 minutes with ice-cooling. The resulting precipitate was collected on a filter, washed with iced ethanol, and then dried *in vacuo* to give the title compound (474 mg).
$^1$H-NMR (400 MHz, DMSO-$d_6$) 5 11.13 (1H, brs), 10.20 (1H, s), 8.62 (1H, brs), 7.85 (1H, s), 7.74-7.62 (5H, m), 7.57-7.49 (4H, m), 6.74 (1H, d, *J* = 15.8 Hz), 5.42 (2H, s), 2.05 (3H, s) .

Examples 2-4

The compounds of Examples 2-4 were prepared from each corresponding starting compound according to a similar process to that of Example 1-1.

| Example | Chemical Structural Formula | $^1$H-NMR (400 MHz, DMSO-$d_6$) | LC-MS, condition B: [M+H]$^+$/Rt (min) |
|---|---|---|---|
| 2 | 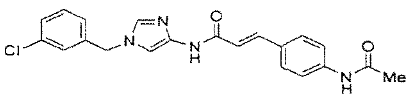 | δ10.51 (1H, s), 10.10 (1H, s), 7.64 (1H, d, *J* = 1.2 Hz), 7.62 (2H, d, *J* = 8.5 Hz), 7.47 (2H, d, *J* = 8.5 Hz), 7.42-7.36 (4H, m), 7.33 (1H, d, *J* = 1.2 Hz), 7.27-7.24 (1H, m), 6.74 (1H, d, *J* = 15.8 Hz), 5.18 (2H, s), 2.05 (3H, s). | 395.2/0.80 |
| 3 | 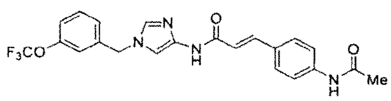 | δ10.50 (1H, s), 10.09 (1H, s), 7.64 (1H, brs), 7.61 (2H, d, *J* = 8.5 Hz), 7.53-7.50 (1H, m), 7.47 (2H, d, *J* = 8.5 Hz), 7.40 (1H, d, *J* = 15.9 Hz), 7.35-7.29 (4H, m), 6.74 (1H, d, *J* = 15.9 Hz), 5.23 (2H, s), 2.05 (3H, s). | 445.2/0.89 |

(continued)

| Example | Chemical Structural Formula | $^1$H-NMR (400 MHz, DMSO-$d_6$) | LC-MS, condition B: $[M+H]^+$/Rt (min) |
|---|---|---|---|
| 4 | 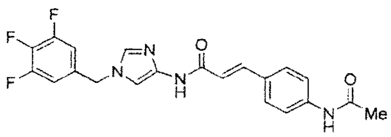 | δ10.51 (1H, s), 10.10 (1H, s), 7.64-7.61 (3H, m), 7.47 (2H, d, *J* = 8.5 Hz), 7.41 (1H, d, *J* = 15.9 Hz), 7.37 (1H, d, *J* = 1.2 Hz), 7.34-7.29 (2H, m), 6.74 (1H, d, *J* = 15.9 Hz), 5.15 (2H, s), 2.05 (3H, s). | 415.3/0.78 |

Example 5

(*E*)-3-(3-(2-Hydroxyethoxy)-4-methoxyphenyl)-*N*-(1-(3,4,5-trifluorobenzyl)-1*H*-imidazol-4-yl)acrylamide

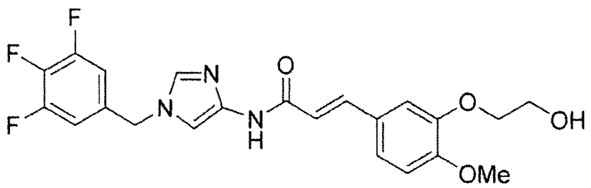

To a solution of the compound of Reference Example 11 (125 mg) in methanol (10 mL) was added 4 mol/L hydrochloric acid-dioxane (88 μL), and the mixture was stirred at 80°C for 40 minutes. The reaction mixture was concentrated in *vacuo,* and 2 mol/L aqueous sodium hydroxide solution was added thereto, and then the mixture was extracted with chloroform. The organic layer was washed with brine, dried over magnesium sulfate, filtrated, and then concentrated *in vacuo.* The residue was purified by silica gel column chromatography (chloroform/methanol) to give the title compound (72 mg).
$^1$H-NMR (400 MHz, DMSO-$d_6$) δ 10.39 (1H, s), 7.63 (1H, d, *J* = 1.2 Hz), 7.42-7.30 (4H, m), 7.15-7.11 (2H, m), 6.99 (1H, d, *J* = 8.8 Hz), 6.74 (1H, d, *J* = 16.0 Hz), 5.15 (2H, s), 4.85 (1H, t, *J* = 5.6 Hz), 4.02-3.98 (2H, m), 3.78 (3H, s), 3.74-3.70 (2H, m).
LC-MS, condition B ($[M+H]^+$/Rt (min)): 448.3/0.758

Example 6

(2*E*)-N-[1-(3-Chlorobenzyl)-1H-imidazol-4-yl]-3-[3-(2-hydroxyethoxy)-4-methoxyphenyl]prop-2-enamide

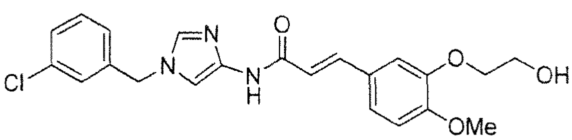

To a solution of the compound of Reference Example 9 (1.25 g) in methanol (10 mL) was added tosic acid monohydrate (0.46 g), and the mixture was stirred at 40°C for 2.5 hours. To the reaction mixture were added saturated aqueous sodium hydrogen carbonate solution and chloroform, and the resulting solid was washed with water and dried. The filtrate was extracted with chloroform, washed with brine, and then dried over magnesium sulfate. The reaction mixture was filtrated and concentrated *in vacuo,* and then the resulting solid was washed with methanol and ethyl acetate and combined with the above solid to give the title compound (0.84 g).
$^1$H-NMR (400 MHz, DMSO-$d_6$) δ 10.40 (1H, s), 7.64 (1H, d, *J* = 1.2 Hz), 7.43-7.36 (4H, m), 7.33 (1H, d, *J* = 1.2 Hz), 7.27-7.24 (1H, m), 7.16-7.10 (2H, m), 7.00 (1H, d, *J* = 8.8 Hz), 6.75 (1H, d, *J* = 16.0 Hz), 5.18 (2H, s), 4.87 (1H, t, *J* = 5.6 Hz), 4.02-3.98 (2H, m), 3.78 (3H, s), 3.75-3.70 (2H, m). LC-MS, condition B ($[M+H]^+$/Rt (min)): 428.2/0.772

Examples 7-8

The compounds of Examples 7 and 8 were prepared from the compounds of Reference Examples 10 and 12 according to a similar process to that of Example 6.

| Example | Chemical Structural Formula | LC-MS, condition B: $[M+H]^+$/Rt (min) |
|---|---|---|
| 7 | 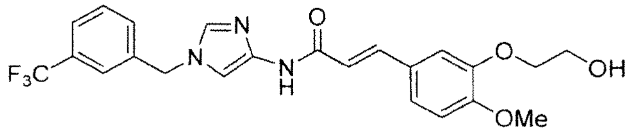 | 462.2/0.86 |
| 8 | 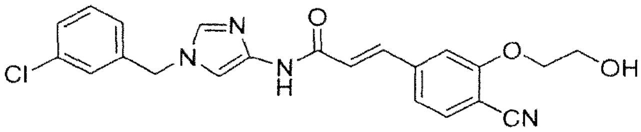 | 423.2/0.815 |

Example 9-1

(2*E*)-*N*-(1-(3-Chlorobenzyl)-1*H*-imidazol-4-yl)-3-(pyridin-3-yl)prop-2-enamide

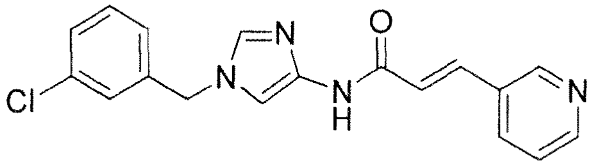

The title compound was prepared from the compound of Reference Example 2 and the corresponding starting compound according to a similar process of that of Reference Example 9.
$^1$H-NMR (400 MHz, DMSO-$d_6$)δ 10.7 (1H, s), 8.75 (1H, s), 8.56-8.55 (1H, m), 7.95 (1H, d, *J* = 9.0 Hz), 7.67 (1H, s), 7.55-7.38 (6H, m), 7.27-7.25 (1H, m), 6.96 (1H, d, *J* = 15.0 Hz), 5.19 (2H, s).

Example 9-2

(2E)-N-(1-(3-Chlorobenzyl)-1H-imidazol-4-yl)-3-(pyridin-3-yl)prop-2-enamide dihydrochloride

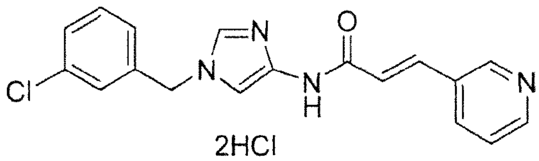

2HCl

To a solution of the compound of Example 9-1 (2.00 g) in 1,4-dioxane (30 mL) was added 4 mol/L hydrochloric acid-dioxane (2.27 mL), and the mixture was stirred at room temperature for 2 hours. The reaction mixture was concentrated *in vacuo,* and the resulting solid was washed with ethyl acetate to give the title compound (1.70 g) (yield: 100%).
$^1$H-NMR (300 MHz, DMSO-$d_6$) δ 11.6 (s, 1H), 9.02 (s, 1H), 8.78 (d, 1H, *J* = 3.0 Hz), 8.65 (s, 1H), 8.46 (d, 1H, *J* =6.0 Hz), 7.88-7.83 (m, 1H), 7.71 (d, 1H, J = 15.0 Hz), 7.58 (d, 1H, *J* = 21.0 Hz), 7.44-7.38 (m, 3H), 7.16 (d, 1H, *J* = 15.0 Hz), 5.34 (s, 2H).

Example 10-1

N-[1-(3-chlorobenzyl)-1H-imidazol-4-yl]-3,4-dimethoxybenzamide

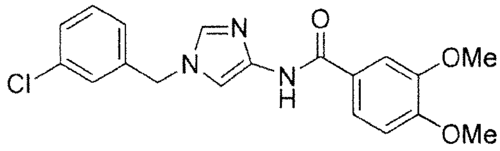

To a solution of the compound of Reference Example 2 (11.0 g) in methylene chloride (240 mL) were added triethylamine (15.8 mL) and 3,4-dimethoxybenzoyl chloride (9.04 g), and the mixture was stirred at room temperature overnight. The reaction solution was concentrated *in vacuo,* and then the resulting solid was washed with ethyl acetate and collected on a filter to give the title compound (9.7 g).
LC-MS, condition C ($[M+H]^-$/Rt (min)): 372.0/2.69
$^1$H-NMR (400 MHz, DMSO-$d_6$) δ 10.64 (1H, s), 7.63 (1H, d, *J* = 1.2 Hz), 7.60-7.56 (2H, m), 7.39-7.31 (4H, m), 7.25-7.21 (1H, m), 6.97 (1H, d, *J* = 8.4 Hz), 5.16 (2H, s), 3.78 (3H, s), 3.76 (3H, s).

Example 10-2

N-[1-(3-Chlorobenzyl)-1H-imidazol-4-yl]-3,4-dimethoxybenzamide hydrochloride

To a solution of the compound of Example 10-1 (70.0 g) in 1,4-dioxane (1.5 L) was added 4 mol/L hydrochloric acid-dioxane (94 mL) and a seed crystal, and the mixture was placed in an ultrasound bath. The solvent was removed, and to the residue was added ethanol (500 mL), the mixture was again placed in the ultrasound bath. The resulting precipitate was collected on a filter and dried *in vacuo* to give the title compound (72.4 g).
$^1$H-NMR (400 MHz, DMSO-$d_6$) δ 11.53 (1H, s), 8.87 (1H, s), 7.68-7.64 (3H, m), 7.58 (1H, s), 7.46-7.40 (3H, m), 7.09 (1H, d, *J* = 8.8 Hz), 5.40 (2H, s), 3.83 (3H, s), 3.82 (3H, s).

Examples 11-12

The compounds of Examples 11 and 12 were prepared from each corresponding starting compound according to a similar process to that of Example 10-1.

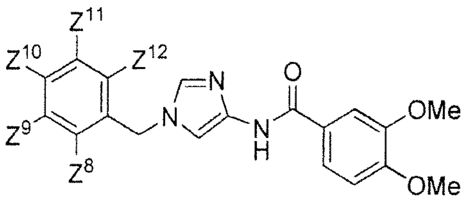

| Example | $Z^8$ | $Z^9$ | $Z^{10}$ | $Z^{11}$ | $Z^{12}$ | LC-MS, condition A: $[M+H]^+$/Rt (min) |
|---|---|---|---|---|---|---|
| 11 | H | F | F | H | H | 374.5/3.47 |
| 12 | H | F | H | F | H | 374.5/3.49 |

Examples 13-14

The compounds of Examples 13 and 14 were prepared from each corresponding starting compound according to a similar process to that of Reference Example 9.

| Example | Chemical Structural Formula | $^1H$-NMR (400MHz, DMSO-$d_6$ ) | LC-MS, condition B: $[M+H]^+$/Rt (min) |
|---|---|---|---|
| 13 | 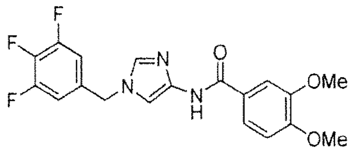 | δ 10.63 (1H, s), 7.64-7.57 (3H, m), 7.40-7.39 (1H, m), 7.32-6.97 (2H, m), 8.8 Hz), 5.13 (2H, s), 3.78 (3H, s), 3.76 (3H, s). | 392.3/0.794 |
| 14 | 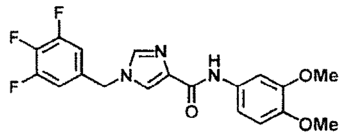 | δ 9.63 (1H, s), 7.94-7.91 (1H, m), 7.89-7.87 (1H, m), 7.48-7.46 (1H, m), 7.42-7.32 (3H, m), 6.87-6.82 (1H, m), 5.23 (2H, s), 3.69 (6H, s). | 392.2/0.85 |

Examples 11-2 to 13-2

The compounds of Examples 11-2 to 13-2 were prepared from each corresponding starting compound according to a similar process to that of Example 10-2.

| Example | Chemical Structural Formula | $^1H$-NMR (400 MHz, DMSO-$d_6$) |
|---|---|---|
| 11-2 | 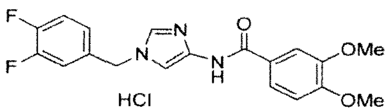 | δ 11.3 (s, 1H), 8.68 (s, 1H), 7.62-7.52 (m, 4H), 7.49-7.41 (m, 1H), 7.28 (s, 1H), 7.05 (1H, d, *J* = 8.4 Hz), 5.31 (s, 2H), 3.79 (s, 3H), 3.78 (s, 3H). |
| 12-2 | 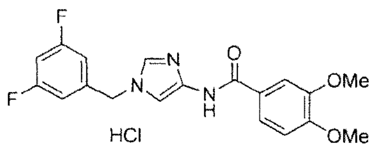 | δ 11.1 (s, 1H), 8.53 (s, 1H), 7.62-7.52 (m, 3H), 7.25-7.13 (m, 3H), 7.04 (1H, d, *J* = 8.4 Hz), 5.32 (s, 2H), 3.79 (s, 6H). |
| 13-2 | 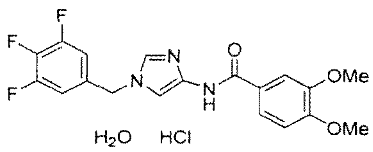 | δ 11.2 (s, 1H), 8.50 (s, 1H), 7.62-7.55 (m, 3H), 7.48-7.39 (m, 2H), 7.04 (d, 1H, J = 8.4 Hz), 5.28 (s, 2H), 3.79 (s, 3H), 3.78 (s, 3H). |

Example 15

5-(Hydroxymethyl)-N-{1-[3-(trifluoromethyl)benzyl]-1H-imidazol-4-yl}picolinamide

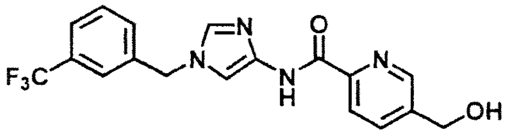

To a solution of the compound of Reference Example 13 (100 mg) in THF (2 mL)/methanol (1 mL) was added lithium borohydride (3 mol/L in THF, 0.08 mL), and the mixture was stirred at room temperature for 3 hours. To the reaction mixture were added saturated aqueous ammonium chloride solution and water, and the mixture was extracted with ethyl acetate. The organic layer was washed with brine, dried over anhydrous sodium sulfate, filtrated, and then concentrated *in vacuo.* To the resulting solid were added ethyl acetate (2 mL) and hexane (2 mL), and the mixture was placed in an ultrasound bath. The resulting solid was collected on a filter, washed wish hexane/ethyl acetate (1/1, 1 mL x 2), and then dried *in vacuo* at 40°C to give the title compound (70 mg).

LC-MS, condition B ([M+H]+/Rt (min)): 377.2/0.733

Examples 16-17

The compounds of Examples 16 and 17 were prepared from the compounds of Reference Examples 17 and 18-3 according to the process of Example 15.

| Example | Chemical Structural Formula | LC-MS, condition B: [M+H]+/Rt (min) |
|---|---|---|
| 16 | 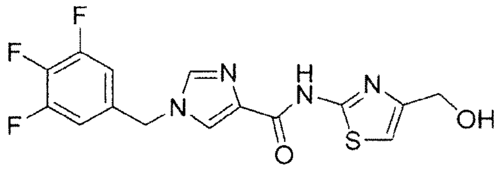 | 369.1/0.732 |
| 17 | 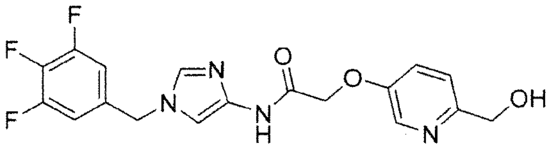 | 393.2/0.594 |

Examples 18-19

The compounds of Examples 18 and 19 were prepared from the compounds of Reference Examples 1 and 4 and each corresponding starting compound according to the process of Reference Example 9.

| Example | Chemical Structural Formula | LC-MS, condition B: [M+H]+/Rt (min) |
|---|---|---|
| 18 | 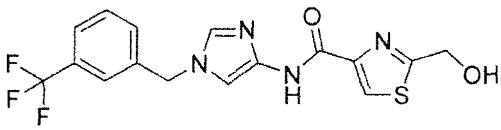 | 383.2/0.729 |
| 19 | 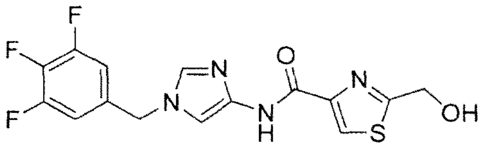 | 369.1/0.690 |

Example 20

6-(Hydroxymethyl)-N-[1-(3,4,5-trifluorobenzyl)-1H-imidazol-4-yl]nicotinamide

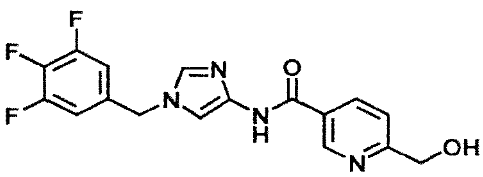

To a solution of methyl 6-(hydroxymethyl)-nicotinate (0.924 g) in THF (22 mL) was added 5 mol/L aqueous potassium hydroxide solution (2.2 ml). The mixture was stirred at room temperature overnight, concentrated *in vacuo* to remove the solvent, and then dried *in vacuo.* To a solution of the resulting solide in DMF (25 mL) were added the compound of Reference Example 4 (1.61 g), HATU (2.52 g), and diisopropylethylamine (2.38 mL), and the mixture was stirred at room temperature for 1 hour. To the reaction mixture were added saturated aqueous sodium hydrogen carbonate

solution and water, and the resulting precipitate was collected on a filter. The resulting solid was washed with water and acetonitrile, ethyl acetate, and then dried *in vacuo* to give the title compound (1.375 g).
$^1$H-NMR (400 MHz, DMSO-$d_6$) δ 11.00 (1H, s), 9.01 (1H, d, *J* = 1.8 Hz), 8.30 (1H, dd, *J* = 7.9, 1.8 Hz), 7.69 (1H, d, *J* = 1.2 Hz), 7.54 (1H, d, *J* = 7.9 Hz), 7.48 (1H, d, *J* = 1.2 Hz), 7.38-7.30 (2H, m), 5.52 (1H, t, *J* = 6.1 Hz), 5.18 (2H, s), 4.60 (2H, d, *J* = 6.1 Hz).
LC-MS, condition B ([M+H]$^+$/Rt (min)): 363.1/0.66

Examples 21-27

The compounds of Examples 21-27 were prepared from the compounds of each Reference Example according to the process of Example 20.

| Example | Chemical Structural Formula | Instrumental Analysis Data |
|---|---|---|
| 21 | 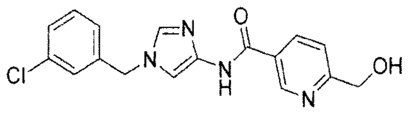 | LC-MS, condition B: [M+H]$^+$/Rt (min) 343.2/0.611 |
| 22 | 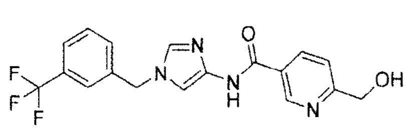 | $^1$H-NMR, (400 MHz, DMSO-$d_6$) δ 10.99 (1H, s), 9.01 (1H, d, *J* = 1.8 Hz), 8.30 (1H, dd, *J* = 8.5, 1.8 Hz), 7.73-7.72 (2H, m), 7.70-7.68 (1H, m), 7.64-7.59 (2H, m), 7.54 (1H, d, *J* = 8.5 Hz), 7.47 (1H, d, *J* = 1.2 Hz), 5.51 (1H, t, *J* = 6.1 Hz), 5.31 (2H, s), 4.60 (2H, d, *J* = 6.1 Hz).<br>LC-MS, condition B: [M+H] $^+$/Rt (min) 377.3/0.672 |
| 23 | 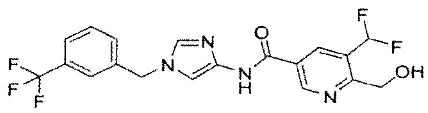 | $^1$H-NMR (400 MHz, DMSO-$d_6$) δ 11.27 (1H, s), 9.17 (1H, d, *J* = 1.8 Hz), 8.54 (1H, d, *J* = 1.8 Hz), 7.75-7.73 (2H, m), 7.70-7.68 (1H, m), 7.64-7.61 (2H, m), 7.49 (1H, d, *J* = 1.2 Hz), 7.42 (1H, t, *J* = 54.3 Hz), 5.61 (1H, t, *J* = 5.8 Hz), 5.31 (2H, s), 4.75 (2H, d, *J* = 5.8 Hz).<br>LC-MS, condition B: [M+H] $^+$/Rt (min) 427.2/0.787 |
| 24 | 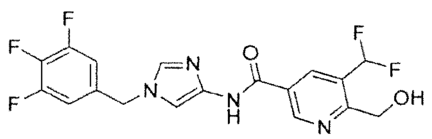 | $^1$H-NMR (400 MHz, DMSO-$d_6$) δ 11.27 (1H, s), 9.17 (1H, s), 8.54 (1H, s), 7.71 (1H, s), 7.51 (1H, s), 7.42 (1H, t, *J* = 54.8 Hz), 7.37-7.33 (2H, m), 5. 61 (1H, t, *J* = 5.8 Hz), 5.19 (2H, s), 4.75 (2H, d, *J* = 5.8 Hz).<br>LC-MS, condition B: [M+H]$^+$/Rt (min) 413.2/0.751 |
| 25 | 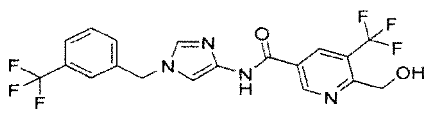 | LC-MS, condition B: [M+H]$^+$/Rt (min) 445.2/0.852 |
| 26 | 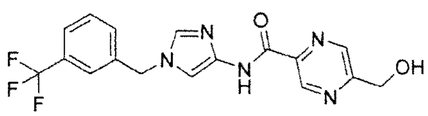 | LC-MS, condition B: [M+H]$^+$/Rt (min) 378.2/0.713 |
| 27 | 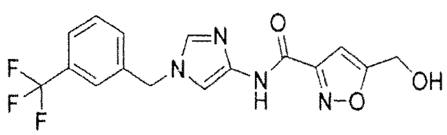 | $^1$H-NMR (400 MHz, DMSO-$d_6$) δ 10.97 (1H, s), 7.73-7.68 (3H, m), 7.64-7.57 (2H, m) , 7.45 (1H, s), 6.81 (1H, s), 5.73 (1H, t, *J* = 5.8 Hz), 5.30 (2H, s), 4.60 (2H, d, *J* = 5.8 Hz).<br>LC-MS, condition B: [M+H]$^+$/Rt (min) 367.2/0.735 |

Example 28

N-[6-(Hydroxymethyl)pyridin-3-yl]-1-(3,4,5-trifluorobenzyl)-1H-imidazole-4-carboxamide

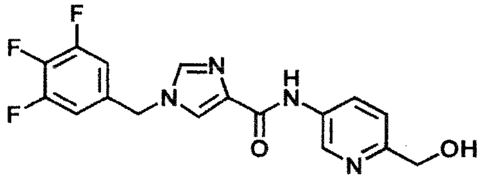

To a solution of the compound of Reference Example 14-2 (138 mg) and the compounds of Reference Example 15 (141 mg) in DMF (15 mL) were added WSCI·HCl (124 mg), HOBt (87 mg), and N,N-diisopropylethylamine (0.188 mL), and the mixture was stirred at 80°C for 6 hours. To the reaction mixture was added water and aqueous sodium hydroxide solution, and the mixture was extracted with chloroform. The organic layer was washed with brine, dried over magnesium sulfate, filtrated, and then concentrated *in vacuo.* The resulting residue was dissolved in methanol (5 mL), 2 mol/L hydrochloric acid-methanol (0.81 mL) was added thereto, and the mixture was stirred at 40°C for 5 hours. To the reaction mixture were added water and then aqueous sodium hydroxide solution, and the mixture was extracted with chloroform. The organic layer was washed with brine, dried over magnesium sulfate, filtrated, and then concentrated *in vacuo.* The residue was purified by silica gel column chromatography (chloroform/methanol) to give the title compound (86.4 mg). LC-MS, condition B ($[M+H]^+$/Rt (min)) 363.2/0.640

$^1$H-NMR (400 MHz, DMSO-$d_6$) δ 10.06 (1H, s), 8.84 (1H, s), 8.19-8.14 (1H, m), 7.97-7.95 (2H, m), 7.42-7.34 (3H, m), 5.31-5.26 (1H, m), 5.24 (2H, s), 4.48 (2H, d, *J* = 4.8 Hz).

Example 29

The compound of Example 29 was prepared from the compound of corresponding Reference Example according to the process of Example 28.

| Example | Chemical Structural Formula | Instrumental Analysis Data |
|---|---|---|
| 29 | 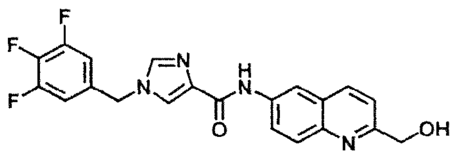 | $^1$H-NMR (400 MHz, DMSO-$d_6$) δ 10.13 (1H, s), 8.49 (1H, d, J = 2.0 Hz), 8.24 (1H, d, *J* = 9.2 Hz), 8.04 (1H, dd, *J* = 9.2, 2.0 Hz), 8.00 (1H, s), 7.98 (1H, s), 7.85 (1H, d, *J* = 8.8 Hz), 7.57 (1H, d, *J* = 8.8 Hz), 7.44-7.38 (2H, m), 5.50-5.46 (1H, m), 5.25 (2H, s), 4.60 (2H, d, *J* = 5.6 Hz) . LC-MS, condition B: $[M+H]^+$/Rt (min) 413.3/0.673 |

Example 30

N-(7-Fluoro-1,2,3,4-tetrahydroquinolin-6-yl)-1-(3,4,5-trifluorobenzyl)-1H-imidazole-4-carboxamide

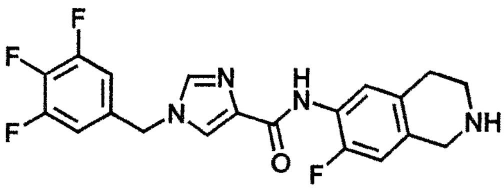

According to the process of Reference Example 9, tert-butyl 7-fluoro-6-({[1-(3,4,5-trifluorobenzyl)-1H-imidazol-4-yl]carbonyl}amino)-3,4-dihydroisoquinoline-2(1H)-carboxylate was prepared from the compound of Reference Example 14-2 and tert-butyl 6-amino-7-fluoro-3,4-dihydroisoquinoline-2(1H)-carboxylate. To a solution of said compound in methanol was added 4 mol/L hydrochloric acid-dioxane, and the mixture was stirred at room temperature overnight. The reaction mixture was concentrated *in vacuo,* and then water and 2 mol/L aqueous sodium hydroxide solution were added thereto. The resulting precipitate was collected on a filter, washed with water and hexane/ethyl acetate (2/1), and dried *in vacuo* to give the title compound.

LC-MS, condition B ($[M+H]^+$/Rt (min)): 405.2/0.665

$^1$H-NMR (400 MHz, DMSO-$d_6$) δ 9.27 (1H, s), 7.96-7.93 (2H, m), 7.69 (1H, d, *J* = 8.0 Hz), 7.43-7.34 (2H, m), 6.91 (1H,

d, *J* = 11.6 Hz), 5.23 (2H, s), 3.75 (2H, s), 2.90-2.86 (2H, m), 2.62-2.57 (2H, m).

Examples 31-32

The compounds of Examples 31 and 32 were prepared from the compounds of Reference Examples 14-2 and 22 and each corresponding compound according to the process of Example 30.

| Example | Chemical Structure Formula | Instrumental Analysis Data |
|---|---|---|
| 31 | 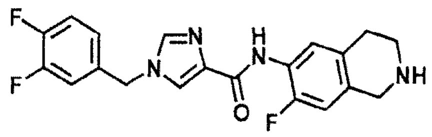 | $^1$H-NMR (400 MHz, EMSO-$d_6$) δ 9.28 (1H, s), 7.96-7.93 (2H, m), 7.71 (1H, d, *J* = 7.6 Hz), 7.53-7.42 (2H, m), 7.22-7.18 (1H, m), 6.93 (1H, d, *J* = 10.8 Hz), 5.24 (2H, s), 3.78 (2H, s), 2.93-2.89 (2H, m), 2.65-2.59 (2H, m) . LC-MS, condition B: $[M+H]^+$/Rt (min) 387.0/0.660 |
| 32 | 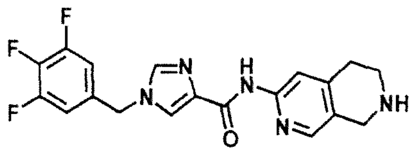 | $^1$H-NMR (400 MHz, DMSO-$d_6$) 5 9.30 (1H, s), 8.02 (1H, s), 7.97 (1H, s), 7.96 (1H, s), 7.88 (1H, s), 7.43-7.37 (2H, m), 5.23 (2H, s), 3.78 (2H, s), 2.92-2.88 (2H, m), 2.71-2.66 (2H, m) . LC-MS, condition B: $[M+H]^+$/Rt (min) 388.2/0.601 |

Example 33

N-(1,2,3,4-Tetrahydroquinolin-6-yl)-1-(3,4,5-trifluorobenzyl)-1H-imidazole-4-carboxamide dihydrochloride

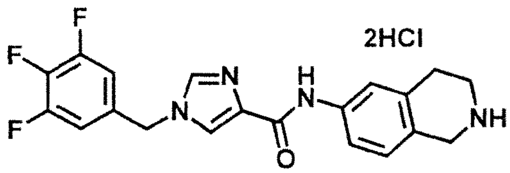

2HCl

According to the process of Reference Example 9, tert-butyl 6-({[1-(3,4,5-trifluorobenzyl)-1H-imidazol-4-yl]carbonyl}amino)-3,4-dihydroisoquinoline-2 (1H)-carboxylate was prepared from the compound of Reference Example 14-2 and *tert*-butyl 6-amino-3,4-dihydroisoquinoline-2(1H)-carboxylate. To a solution of said compound in methanol was added 4 mol/L hydrochloric acid-dioxane, and the mixture was stirred at 80°C. The resulting precipitate was collected on a filter, washed with diisopropyl ether, and then dried *in vacuo* to give the title compound.
LC-MS, condition B ($[M+H]^+$/Rt (min)): 387.2/0.615

Examples 34-49

The compounds of Examples 34-49 were prepared from the compounds of each Reference Example and each corresponding starting compound according to the process of Example 33.

| Example | Chemical Structural Formula | Instrumental Analysis Data |
|---|---|---|
| 34 | 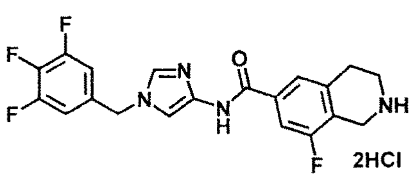 2HCl | $^1$H-NMR (400 MHz, $CD_3OD$) δ 8.94 (1H, d, *J* = 1.6 Hz), 7.78 (1H, s), 7.65 (1H, d, *J* = 9.2 Hz), 7.58 (1H, d, *J* = 2.0 Hz), 7.33-7.30 (2H, m), 5.44 (2H, s), 4.46 (2H, s), 3.56 (2H, t, J = 6.4 Hz), 3.24 (2H, t, *J* = 6.0 Hz). LC-MS, condition B : $[M+H]^+$/Rt (min) 405.2/0.645 |

(continued)

| Example | Chemical Structural Formula | Instrumental Analysis Data |
|---|---|---|
| 35 | 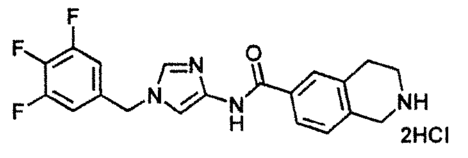 | $^1$H-NMR (400 MHz, $CD_3OD$) δ 8.89 (1H, d, *J* = 2.0 Hz), 7.87 (2H, m), 7.55 (1H, d, *J* = 2.0 Hz), 7.42 (1H, d, *J* = 8.4 Hz), 7.33-7.27 (2H, m), 5.43 (2H, s), 4.46 (2H, s), 3.56 (2H, t, *J* = 6.4 Hz), 3.22 (2H, t, *J* = 6.4 Hz) . LC-MS, condition B: $[M+H]^+$/Rt (min) 387.2/0.635 |
| 36 | 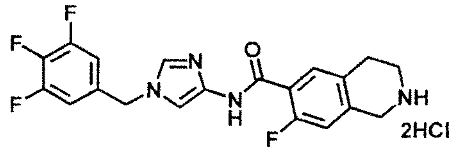 | $^1$H-NMR (400 MHz, $CD_3OD$) δ 7.77 (1H, d, *J* = 6.8 Hz), 7.58 (1H, s), 7.27-7.22 (4H, m), 5.37 (2H, s), 4.44 (2H, s), 3.54 (2H, t, *J* = 6.4 Hz), 3.16 (2H, t, *J* = 6.4 Hz) . LC-MS, condition B: $[M+2H]^{2+}$/Rt (min) 203.1/0.620 |
| 37 | 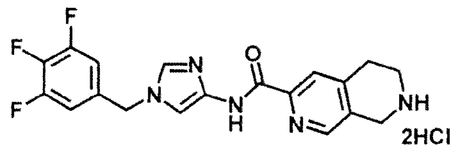 | $^1$H-NMR (400 MHz, $CD_3OD$) δ 8.90 (1H, d, *J* = 1.6 Hz), 8.61 (1H, s), 8.17 (1H, s), 7.65 (1H, d, *J* = 1.6 Hz), 7.35-7.28 (2H, m), 5.43 (2H, s), 4.54 (2H, s), 3.58 (2H, t, *J* = 6.4 Hz), 3.26 (2H, t, *J* = 6.4 Hz). LC-MS, condition B: $[M+H]^+$/Rt (min) 388.2/0.554 |
| 38 | 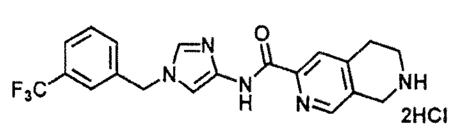 | $^1$H-NMR (400 MHz, $CD_3OD$) δ 8.94 (1H, s), 8.60 (1H, s), 8.12 (1H, s), 7.82 (1H, s), 7.76-7.65 (4H, m), 5.55 (2H, s), 4.53 (2H, s), 3.58 (2H, t, *J* = 5.2 Hz), 3.26 (2H, t, *J* = 6.4 Hz) LC-MS, condition B: $[M+2H]^{2+}$/Rt (min) 201. 7/0.659 |
| 39 | 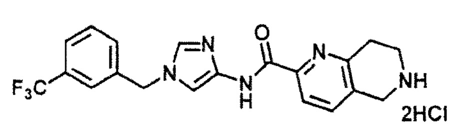 | $^1$H-NMR (400 MHz, $CD_3OD$) δ 8.84 (1H, s), 8.12 (1H, d, *J* = 8.4 Hz), 7.91 (1H, d, *J* = 8.4 Hz) , 7.81 (1H, s), 7.75-7.64 (4H, m), 5.54 (2H, s), 4.53 (2H, s), 3.69 (2H, t, *J* = 6.4 Hz), 3.36 (2H, t, *J* = 6.4 Hz). LC-MS, condition B : $[M+2H]^{2+}$/Rt (min) 201. 7/0.620 |
| 40 | 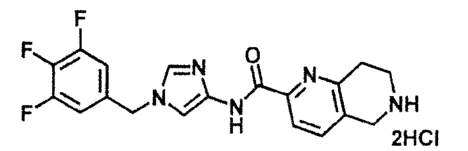 | $^1$H-NMR (400 MHz, $CD_3OD$) δ 8.80 (1H, d, *J* = 1.6 Hz), 8.13 (1H, d, *J* = 8.0 Hz), 7.92 (1H, d, J = 8.4 Hz), 7.67 (1H, d, *J* = 2.0 Hz), 7.33-7.26 (2H, m), 5.42 (2H, s), 4.54 (2H, s), 3.70 (2H, t, *J* = 6.4 Hz), 3.37 (2H, t, *J* = 6.4 H). LC-MS, condition B: $[M+2H]^{2+}$/Rt (min) 194.7/0.636 |
| 41 | 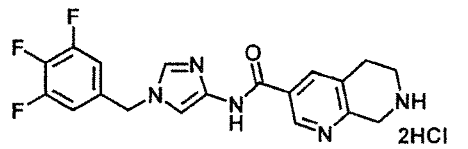 | $^1$H-NMR (400 MHz, $CD_3OD$) δ 9.03 (1H, s), 9.00 (1H, s), 8.31 (1H, s), 7.61 (1H, s), 7.32 (2H, t, *J* = 7.2 Hz), 5.44 (2H, s), 4.50 (2H, s), 3.61 (2H, t, *J* = 6.4 Hz), 3.26 (2H, t, *J* = 6.4 Hz). LC-MS, condition B : $[M+2H]^{2+}$/Rt (min) 194.7/0.624 |
| 42 | 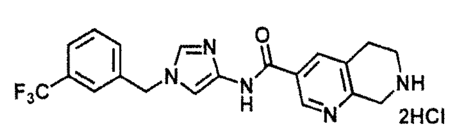 | $^1$H-NMR (400 MHz, $CD_3OD$) δ 9.00 (1H, d, J = 2.4 Hz), 8.76 (1H, s), 8.26 (1H, d, *J* = 1.6 Hz), 7.78-7.57 (4H, m), 7.56 (1H, d, *J* = 0.8 Hz), 5.51 (2H, s), 4.48 (2H, s), 3.60 (2H, t, *J* = 6.4 Hz), 3.24 (2H, t, *J* = 6.4 Hz). LC-MS, condition B: $[M+H]^+$/Rt (min) 402.3/0.590 |
| 43 | 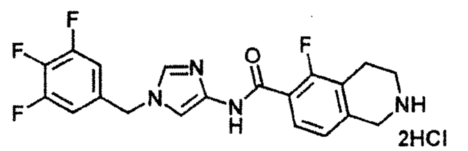 | $^1$H-NMR (400 MHz, $CD_3OD$) δ 8.72 (1H, s), 7.77 (1H, t, *J* = 7.6 Hz), 7.59 (1H, d, *J* = 1.2 Hz), 7.29-7.24 (3H, m), 5.39 (2H, s), 4.47 (2H, s), 3.58 (2H, t, *J* = 6.4 Hz), 3.15 (2H, t, J = 6.4 Hz) . LC-MS, condition B: $[M+2H]^{2+}$/Rt (min) 203.1/0.650 |

(continued)

| Example | Chemical Structural Formula | Instrumental Analysis Data |
|---|---|---|
| 44 | 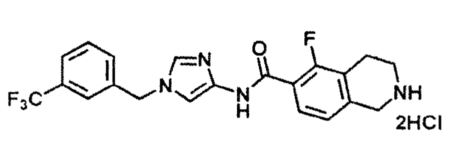 | $^1$H-NMR (400 MHz, $CD_3OD$) δ 8.83 (1H, s), 7.80-7.61 (5H, m), 7.59 (1H, d, J = 1.2 Hz), 7.24 (1H, d, *J* = 7.6 hz), 5.52 (2H, s), 4.47 (2H, s), 3.58 (2H, t, *J* = 6.4 Hz), 3.15 (2H, t, *J* = 6.4 Hz).<br>LC-MS, condition B: $[M+2H]^2$/Rt (min) 210.1/0.708 |
| 45 | 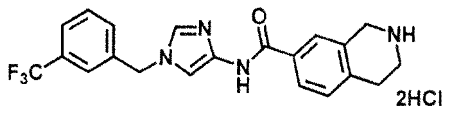 | LC-MS, condition B:$[M+H]^+$/Rt (min) 401.3/0.588 |
| 46 | 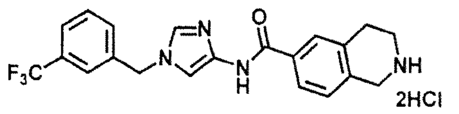 | LC-MS, condition B: $[M+2H]^{2+}$/Rt (min) 201.2/0.663 |
| 47 | 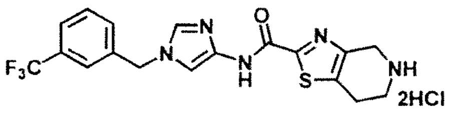 | LC-MS, condition B:$[M+H]^+$/Rt (min) 408.2/0.603 |
| 48 | 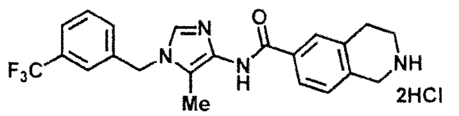 | $^1$H-NMR (400 MHz, $CD_3OD$) δ 8.93 (1H, s), 7.91-7.88 (2H, m), 7.78-7.60 (4H, m), 7.42 (1H, d, J = 8.0 Hz), 5.57 (2H, s), 4.47 (2H, s), 3.56 (2H, t, J = 6.4Hz), 3.22 (2H, t, J = 6.4 Hz), 2.21 (3H, d, J = 1.2 Hz). LC-MS, condition B: $[M+2H]^{2+}$/Rt (min) 208.2/0.668 |
| 49 | 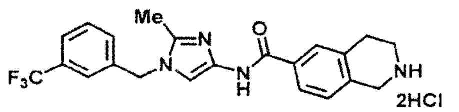 | $^1$H-NMR (400 MHz, $CD_3OD$) : δ 7.88-7.85 (2H, m), 7.75-7.73 (2H, m), 7.68-7.60 (2H, m), 7.42-7.38 (2H, m), 5.49 (2H, s), 4.46 (2H, s), 3.55 (2H, t, J = 6.4 Hz), 3.21 (2H, t, J = 6.0 Hz), 2.68 (3H, d, J = 2.4 Hz).<br>LC-MS, condition B : $[M+2H]^{2+}$/Rt (min) 208.2/0.583 |

Example 50

N-[1-(3,4,5-Trifluorobenzyl)-1H-imidazol-4-yl]-(1,2,3,4-tetrahydroquinoline-6-carboxamide ditrifluoroacetate

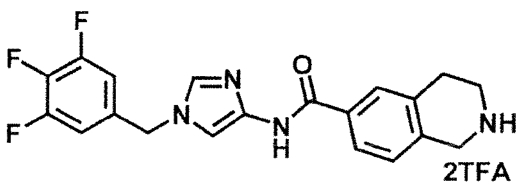

According to the process of Reference Example 9, N-(1-(3,4,5-trifluorobenzyl)-1H-imidazol-4-yl)-1,2,3,4-tetrahydroquinoline-6-carboxamide was prepared from the compound of Reference Example 4 and 2-(tert-butoxycarbonyl)-1,2,3,4-tetrahydroquinoline-6-carboxylate. To a solution of said compound in chloroform was added trifluoroacetic acid, the mixture was stirred at room temperature, and then the reaction mixture was concentrated *in vacuo.* To the residue was added a mixture of hexane and ethyl acetate, and the resulting precipitate was collected on a filter and dried *in vacuo* to give the title compound.
LC-MS, condition B ($[M+2H]^{2+}$/Rt (min)): 194.1/0.580
$^1$H-NMR (400 MHz, $CDCl_3$)δ 7.94 (1H, d, *J* = 1.6 Hz), 7.85-7.83 (2H, m), 7.47 (1H, d, *J* = 2.0 Hz), 7.38 (1H, d, *J* = 8.4 Hz), 7.14 (2H, dd, *J* = 8.4, 6.8 Hz), 5.26 (2H, s), 4.44 (2H, s), 3.55 (2H, t, *J* = 6.4 Hz), 3.20 (2H, t, *J* = 6.4 Hz).

Examples 51-54

The compounds of Examples 51-54 were prepared from the compounds of corresponding Reference Example and each corresponding starting compound according to the process of Example 50.

| Example | Chemical Structure Formula | LC-MS, condition B: $[M+H]^+$/Rt (min) |
|---|---|---|
| 51 | 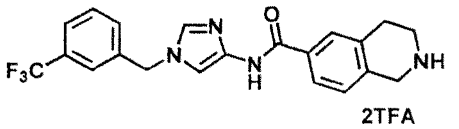 | 401.3/0.588 |
| 52 | 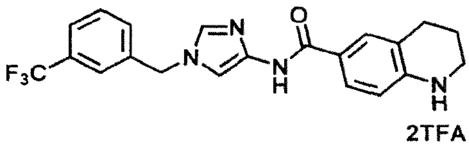 | 401.3/0.899 |
| 53 | 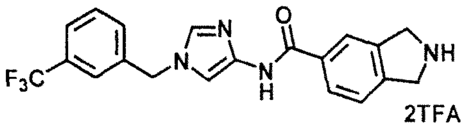 | 194.58 ($[M+2H]^{2+}$) /0.601 |
| 54 | 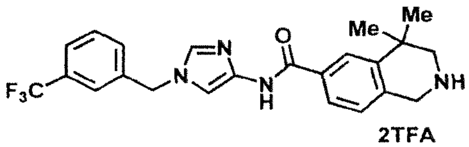 | 429.3/0.706 |

Example 55

6-(Hydroxymethyl)-5-methyl-N-[1-(3,4,5-trifluorobenzyl)-1H-imidazol-4-yl]nicotinamide

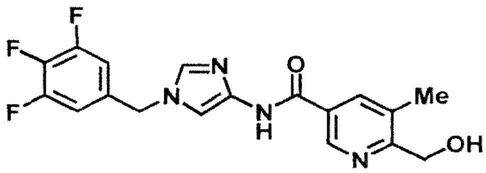

The title compound was prepared from the compound of Reference Example 28 according to the process of Example 20.
LC-MS, condition B ($[M+H]^+$/Rt (min)): 377.2/0.631
$^1$H-NMR (400 MHz, DMSO-$d_6$)$\delta$ 10.97 (1H, s), 8.87 (1H, d, $J$ = 1.8 Hz), 8.10 (1H, d, $J$ = 1.8 Hz), 7.69 (1H, d, $J$ = 1.2 Hz), 7.48 (1H, d, $J$ = 1.2 Hz), 7.38-7.31 (2H, m), 5.18 (2H, s), 5.11 (1H, t, $J$ = 5.5 Hz), 4.60 (2H, d, $J$ = 5.5 Hz), 2.35 (3H, s).

Examples 56-57

The compounds of Examples 56 and 57 were prepared from the compounds of Reference Examples 1, 4, and 29-3 according to the process of Reference Example 9.

| Example | Chemical Structure Formula | LC-MS, condition B: $[M+H]^+$/Rt (min) |
|---|---|---|
| 56 | 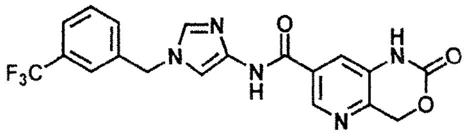 | 418.2/0.711 |
| 57 | 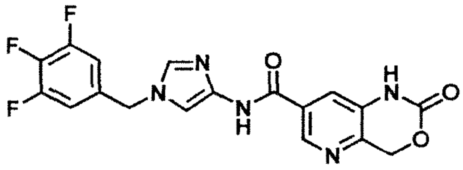 | 404.2/0.670 |

Example 58

5-Amino-6-(hydroxymethyl)-N-{1-[3-(trifluoromethyl)benzyl]-1H-imidazol-4-yl}nicotinamide

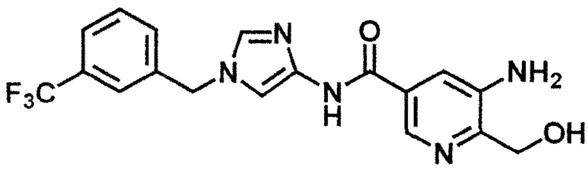

To a suspention of the compound of Example 56 (13 mg) in THF (0.5 mL)/methanol (0.5 mL) was added 2 mol/L aqueous sodium hydroxide solution (0.031 mL), and the mixture was stirred at 60°C for 3 hours and then at 90°C for 6.5 hours. The reaction mixture was cooled to room temperature, water was added thereto, and then the mixture was stirred at room temperature for 5 minutes. The resulting precipitate was collected on a filter, washed with water, and then dried *in vacuo* at 50°C to give the title compound (7 mg).
LC-MS, condition B ($[M+H]^+$/Rt (min)): 392.2/0.647
$^1H$-NMR (400 MHz, DMSO-$d_6$)$\delta$ 10.79 (1H, s), 8.29 (1H, d, *J* = 1.8 Hz), 7.72-7.68 (3H, m), 7.64-7.581 (2H, m), 7.43 (1H, d, *J* = 1.2 Hz), 7.42 (1H, d, *J* = 1.8 Hz), 5.36 (2H, s), 5.30 (2H, s), 5.18 (1H, t, *J* = 5.5 Hz), 4.52 (2H, d, *J* = 5.5 Hz)

Example 59

The compound of Example 59 was prepared from the compound of Example 57 according to the process of Example 58.

| Example | Chemical Structure Formula | LC-MS, condition B: $[M+H]^+$/Rt (min) |
|---|---|---|
| 59 | 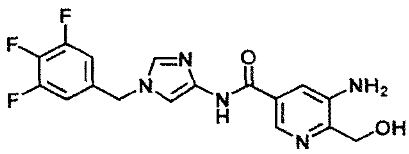 | 378.2/0.603 |

Example 60

(E)-2-Methoxy-5-(3-oxo-3-((1-(3-(trifluoromethyl)benzyl)-1H-imidazol-4-yl)amino)prop-1-en-1-yl)phenolacetate

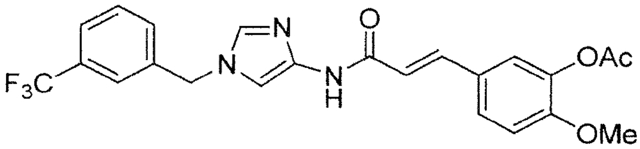

To a solution of (E)-3-(3-acetoxy-4-methoxyphenyl)acrylic acid (71.0 mg) in dichloroethane (2 mL) were added oxalyl chloride (39 μL) and DMF (2 μL), and the mixture was stirred at room temperature for 1 hour. The reaction mixture was concentrated *in vacuo* and dried to give acid chloride. To a solution of the compound of Reference Example 1 (70.0 mg) in dichloromethane (5 mL) triethylamine (105uL), and the acid chloride was added dropwise thereto. The mixture was stirred overnight, water was added thereto, and the mixture was extracted with chloroform. The organic layer was washed with brine, dried over magnesium sulfate, filtrated, and then concentrated *in vacuo.* The residue was purified by silica gel column chromatography (chloroform/methanol) to give the title compound (30 mg). LC-MS, condition B ($[M+H]^+$/Rt (min)): 460.2/1.01

Test Example 1: Test for inhibiting sphere-forming ability of cancer cells

The reliable methods established for measuring the self-renewal ability of cells which is one of the CSC's properties include a method for measuring the sphere-forming ability of cancer cells in non-adherent condition in the absence of serum (Cancer Res 65, 5506-5511 (2005)). HCT-116 cells were available from the American Type Culture Collection (ATCC). HCT-116 cells were cultured at 37°C and 5% $CO_2$ using the McCoy's 5a medium containing 10% fetal bovine serum (FBS), 100 U/ml penicillin, and 100 μg/ml streptomycin. HCT-116 cells were seeded in a 384 Well Black Clear Bottom Ultra-Low Attachment Microplate (Corning Cat. No.3827) in an amount of 350-800 cells/well using the DMEM/F12 medium containing 2% B27 supplement (GIBCO), 20 ng/mL epidermal growth factor (EGF) (peprotech), 10 ng/mL basic fibroblast growth factor (bFGF) (peprotech), 5 μg/mL insulin (Sigma), and 1% penicillin/streptomycin. The test compounds were added into each well to adjust the final concentration of DMSO to 0.1%, and the cells were cultured for 4 days. The number of viable cells in each well was then measured with CellTiter-Glo® Luminescent Cell Viability Assay (Promega) to calculate the concentration of each test compound for 50% inhibition of cell proliferation (Sphere $IC_{50}$ value; μmol/L).

The experiment of Test Example 1 was performed for the compounds of each Example. The concentrations of each test compound for 50% inhibition of cell proliferation (Sphere $IC_{50}$ value; μlmol/L) are shown in Table below. The value indicated by % shows (100% - cell proliferation inhibition rate) in 1 μmol/L.

| Example | $IC_{50}$ (μmol/L) | Example | $IC_{50}$ (μmol/L) | Example | $IC_{50}$ (μmol/L) |
|---|---|---|---|---|---|
| 1-2 | 0.31 | 20 | 0.007 | 39 | 0.04 |
| 2 | 0.22 | 21 | 0.056 | 40 | 0.05 |
| 3 | 0.63 | 22 | <0.01 | 41 | 0.07 |
| 4 | <0.01 | 23 | <0.01 | 42 | 0.03 |
| 5 | 0.06 | 24 | <0.01 | 43 | 0.43 |
| 6 | 0.06 | 25 | 0.070 | 44 | 0.36 |
| 7 | 0.09 | 26 | 0.053 | 45 | 0.67 |
| 8 | 0.08 | 27 | 0.653 | 47 | 65.15 |
| 9 | 0.89 | 28 | <0.01 | 48 | 6.20 |
| 10-2 | 0.63 | 29 | 0.079 | 49 | 0.20 |
| 11 | 0.35 | 30 | 0.08 | 50 | 0.06 |
| 12 | 0.09 | 31 | 0.66 | 51 | 0.08 |
| 13 | 0.06 | 32 | 0.06 | 52 | 0.69 |
| 14 | 0.21 | 33 | 0.66 | 55 | <0.01 |
| 15 | 0.030 | 34 | 0.07 | 56 | 0.52 |
| 16 | 0.629 | 35 | 0.06 | 58 | 0.029 |

(continued)

| Example | $IC_{50}$ (μmol/L) | Example | $IC_{50}$ (μmol/L) | Example | $IC_{50}$ (μmol/L) |
|---|---|---|---|---|---|
| 17 | <0.01 | 36 | 0.39 | 59 | 0.01 |
| 18 | 0.024 | 37 | 0.09 | | |
| 19 | 0.077 | 38 | 0.07 | | |

Test Example 2: Anti-tumor effect to HCT-116 tumor-bearing mouse

The present compound can be used to evaluate the anti-tumor effect thereof. A 4 to 7-week-old nude mouse (BALB/cAnNCrj-nu/nu, female, CHARLES RIVER LABORATORIES JAPAN, INC.) received intradermal transplantation of HCT-116 cells (ATCC) in an amount of 3 x $10^6$ cells/mouse around the ventral portion. The engraftment of HCT-116 cells was observed 5 to 14 days after the transplantation, and then each compound suspended in a solvent such as 0.5% methylcellulose solution was orally administrated to the mouse in a dose of 1 to 200 mg/kg one to twice daily. The tumor volume was measured over time after the administration to evaluate the effect for reducing the tumor volume by the administration of each compound. The tumor volume can be calculated from the minor axis and the major axis of the tumor measured with a digital caliper (Mitutoyo) according to the following formula:

$$\text{Tumor volume } [mm^3] = 0.5 \times \text{minor axis } [mm] \times (\text{major axis } [mm])^2$$

The tumor volume in control administration group treated with only a solvent such as 0.5% methylcellulose solution was compared with that of the present compound administration group, and T/C value was calculated according the following formula to evaluate the anti-tumor effect of the present compound.

T/C(%) = (the tumor volume at the end of administration in the present compound administration group - the tumor volume at the start of administration in the present compound administration group) / (the tumor volume at the end of administration in the control administration group - the tumor volume at the start of administration in the control administration group) x 100

The T/C values (%) of the present compound on each dosage and administration period in the HCT-116 tumor-bearing mouse are shown below.

| Example | dosage (mg/kg) | administration period (day) | T/C (%) |
|---|---|---|---|
| 1-2 | 200 | 15 | 51 |
| 9-2 | 150 | 16 | 65 |
| 10-2 | 150 | 19 | 53 |
| 11-2 | 150 | 19 | 52 |
| 12-2 | 150 | 19 | 84 |
| 13-2 | 15 | 22 | 72 |

(continued)

| Example | dosage (mg/kg) | administration period (day) | T/C (%) |
|---|---|---|---|
| 13-2 | 150 | 22 | 45 |
| 22 | 100 | 16 | 49 |
| 23 | 30 | 17 | 74 |
| 23 | 100 | 17 | 75 |
| 24 | 30 | 17 | 63 |
| 24 | 100 | 17 | 54 |
| 32 | 10 | 17 | 90 |
| 32 | 30 | 17 | 79 |
| 34 | 30 | 17 | 71 |
| 34 | 100 | 17 | 42 |
| 35 | 30 | 17 | 73 |
| 39 | 30 | 17 | 55 |
| 39 | 100 | 17 | 49 |
| 40 | 30 | 17 | 62 |
| 40 | 100 | 17 | 46 |
| 41 | 30 | 17 | 79 |
| 41 | 100 | 17 | 73 |
| 42 | 30 | 17 | 62 |
| 42 | 100 | 17 | 59 |
| 55 | 30 | 17 | 79 |
| 55 | 100 | 17 | 65 |
| 58 | 30 | 17 | 90 |
| 58 | 100 | 17 | 81 |
| 59 | 30 | 17 | 91 |
| 59 | 100 | 17 | 91 |

Test Example 3: Exploration of combination drug for enhacing inhibitory effect on the sphere-forming ability

Various types of cultured cancer cells (colon cancer-derived HCT116 cells, lung cancer-derived H460 cells) were treated with trypsin and collected. The collected cells were suspended in the sphere formation medium (DMEM/F12 medium: 2% B27® supplement, 20 ng/mL bFGF, 20 ng/mL EGF, 5 μg/mL human insulin, 100 U/ml penicillin, and 100 μg/ml streptomycin) and seeded in a 384-well plate with Ultra-Low Attachment surface (Corning) in an amount of $3x10^3$-$8x10^3$ cells/well. The compound of Example 24 was then added into each well to adjust the final concentration to 10-1000 nmol/L. A test compound was then added into each well in various concentrations, and the plate was cultured at 37°C and 5% carbon dioxide in an incubator for 4 days. After the culture, CellTiterGlo (Promega) was added into all of the wells, and the plate was allowed to stand at room temperature for 10 minutes to measure the intensity of luminescence of each well. The intensity of luminescence of each well with the compound of Example 24 (Lsample) was divided by that of the well containing only cells with no compound (Lcontrol) to calculate the sphere formation rate. The formula for calculating the rate is shown below.

$$\text{Sphere formation rate (\%)} = (\text{Lsample})/(\text{Lcontrol}) \times 100$$

The theoretical sphere formation rate of a combination of the compound of Example 24 and a test compound (Lc) was defined as the value calculated by multiplying the sphere formation rate of the compound of Example 24 alone (La) and the sphere formation rate of a test compound alone (Lb) together. Also, the measured sphere formation rate of the combination was defined as (Ld).

The (Ld)/(Lc) values of all combinations of the compound of Example 24 and each test compound in each concentration were calculated. The minimum value thereof (hereinafter also referred to as sCI, as necessary) was used as the criteria for screening. The minimum value of sCI of each test compound is shown in Table below.

| HCT116 | |
|---|---|
| **sCI** | **Test Compound** |
| <0.5 | Toremifene, Obatoclax, Nutlin-3, |
| 0.5-0.6 | Raloxifen, GSK690693, |
| 0.6-0.7 | Gemcitabine, Fluvastatin, XL147, Tretinoin, Ezetimibe, BKM120, SB 203580, GDC-0879, Iniparib, Paclitaxel, ENMD-2076, |
| 0.7-0.8 | Dasatinib, Lapatinib, Sunitinib, Cisplatin, Ftorafur, Docetaxel, Fingolimod, Triamcinolone Acetonide, Exemestane, LDE225, AT7519, KU-55933, PD153035, STF-62247,MK-1775, Vatalanib, Maraviroc, Brivanib, Amcasertib, SRT1720, Procarbazine, SB 216763, Mercaptopurine, Tie2 kinase inhibitor, Megestrol Acetate, PF-04217903, BIRB 796, Cyclophosphamide monohydrate, Cyt387, D-glutamine, GSK1904529A, GSK2126458, SB 431542, Ostarine, BI 2536, Decitabine, Flutamide, Quercetin, Pamidronate, Afatinib, Bosutinib, Linsitinib, JNJ-7706621, SGX-523, Disulfiram, DAPT, RG108, Hydroxyurea, AG14361, Floxuridine, Ifosfamide, AZD6244, SGI-1776, Busulfan, PHA-665752, EX 527, Dexamethasone acetate, JNJ 26854165, Thalidomide, Valproic acid sodium salt, Daunorubicin, BMS-599626, E7080, Sirtinol, TG101348, JNJ-26481585, Anagrelide, BMS 777607, Rucaparib, OSI-420, Teniposide, LY294002, Danusertib, Rigosertib, Tozasertib, BMS 794833, Vinpocetine, Silibinin, |
| 0.8-0.9 | Bicalutamide, Tamoxifen, Temozolomide, Capecitabine, Irinotecan, Oxaliplatin, PD173074, Estrone, Gossypol, Geldanamycin, Prednisone, CHIR-99021, PF-562271, Ruxolitinib, GSK461364, Formestane, WAY-362450, YM155, Etoposide, Linifanib, (-)-Epigallocatechin gallate, MK-2206, Aminoglutethimide, Simvastatin, BAY 11-7082, Bendamustine, S-Ruxolitinib, Carboplatin, Hydrocortisone, Ibrutinib, Streptozotocin, Cladribine,Dorzolamide, CP-466722, LY2157299, NVP-BSK805, Phloretin, JNJ-38877605, WP1130, CH5132799, PF 573228, Letrozole, Mesna, SNS-314, Sotrastaurin, Triptolide, Telatinib, 2-Methoxyestradiol, PD0325901, PF-03814735, TAK-733, Doxorubicin, LY2228820, LY2109761, CAL-101, Chrysophanic acid, Bleomycin sulfate, Motesanib, IC-87114, Enzastaurin, Saracatinib, Coenzyme Q10, Rebastinib, Ranolazine, XL765, Zibotentan, Cyclopamine, Fostamatinib, Mifepristone, Raltitrexed, Itraconazole, Silmitasertib, CUDC-101, Aprepitant, Pomalidomide, Febuxostat, ABT-888, Betapar, Tipifarnib, Neratinib, Lomustine, Mycophenolate, R406, AT9283, PHA-793887, Alisertib, Ganetespib, Roscovitine, XAV-939, Axitinib, SB 743921, Leucovorin, AT-406, AZD8055, Amuvatinib, Doxercalciferol, Cyclosporin A, Y-27632, Tivozanib, Pelitinib, Vemurafenib, Nocodazole, Vinblastine, Barasertib, Epothilone A, APO866, Estradiol, 17-DMAG, Volasertib, ZSTK474, Rosiglitazone, Dapagliflozin, Vismodegib, Enzalutamide, Napabucasin, SB590885, Elesclomol, PAC-1, Entinostat, Trametinib, Abiraterone, BEZ235, Quizartinin, Pioglitazone, Celecoxib, CEP33779,BIBR 1532, Epothilone B, Dexamethasone, Vargatef, Masitinib, Topotecan, Canagliflozin, GW4064, Fludarabine, Zileuton, Dimesna, Nelarabine, Vincristine, KU-60019, KX2-391, Fludarabine Phosphate, Dacomitinib, SU11274, DMXAA, Bexarotene, PI-103, Cytarabine, Carmofur, Sodium butyrate, PIK-75, PIK-90, AR-42, BX-795, PH-797804, Desmethyl Erlotinib, Palbociclib, Medroxyprogesterone acetate, Vorinostat, Pemetrexed, Tofacitinib, Altretamine, ABT-737, |
| 0.9-1 | Imatinib Mesylate, Sorafenib, Lapatinib Ditosylate, Imatinib, Pazopanib,Gemcitabine HCl, AZD7762, Nilotinib, TAME, NU7441, AMG 900, Anastrozole, WZ4002, Idarubicin, AEE788, Ubenimex, YM201636, ABT-751, Triciribine, BTZ043, Bortezomib, Isotretinoin, Vandetanib, GDC-0941, Ispinesib, Ku-0063794, Dalcetrapib, Navitoclax, AUY922, YO-01027, Adrucil, Regorafenib, Lonidamine, PF-3845, CYC116, TAE684, Everolimus, TW-37, Dacarbazine, Tosedostat, WYE-354, PIK-93, Imiquimod, Trichostatin A, MK-0752, Cyclophosphamide, MLN2238, Flavopiridol, Andarine, Azacitidine, MLN9708, Torin 1, 3-Methyladenine, LY2603618, Clofarabine, Crizotinib, Dovitinib, Gefitinib, TPCA-1, Olaparib, LDN193189, CI-1040, Ponatinib, Belinostat, Erlotinib, Mitoxantrone, AZ628, OSI-930, Torin 2, A-769662, AZ 3146, Rapamycin, Cediranib, Lenalidomide, Tanespimycin, Tandutinib, SNS-032, SB 525334, Fulvestrant, |

(continued)

| HCT116 | |
|---|---|
| sCI | Test Compound |
| 1< | INK 128, Palomid 529, BIIB021, Epirubicin, Temsirolimus, PCI-24781, Pracinostat, GW3965, Deforolimus, Azathioprine, Mocetinostat, Mycophenolate mofetil, Crenolanib, Abitrexate, Salinomycin, |
| H460 | |
| sCI | Test Compound |
| <0.5 | Toremifene, Tamoxifen, Raloxifen, Sorafenib, Lapatinib, Fluvastatin, Simvastatin, STF-62247, Fingolimod, BMS-599626, LY2228820, Tie2 kinase inhibitor, GSK1904529A, Obatoclax, Masitinib, Linifanib, Crenolanib, Sotrastaurin, |
| 0.5-0.6 | Dasatinib, Lapatinib Ditosylate, WP1130, Palomid 529, PHA-665752, SGI-1776, Fludarabine Phosphate, |
| 0.6-0.7 | Imatinib, Ezetimibe, NVP-BSK805, PIK-93, Tivozanib, Estradiol, |
| 0.7-0.8 | Temozolomide, Regorafenib, CHIR-99021, SB 203580, Ifosfamide, Raltitrexed, Fulvestrant, Dacomitinib, Nocodazole, Bexarotene, SNS-032, Procarbazine, Vatalanib, Rosiglitazone, BMS 794833, Irinotecan, Elesclomol, Vismodegib, Triciribine, Dovitinib, SB 431542, Gossypol, Ganetespib, R406, Streptozotocin, APO866, YM201636, Canagliflozin, Brivanib, Telatinib, E7080, Amuvatinib, GW4064, Clofarabine, Dapagliflozin, Thalidomide, GDC-0879, |
| 0.8-0.9 | SB 203580, Ifosfamide, Raltitrexed, Fulvestrant, Dacomitinib, Nocodazole, Bexarotene, SNS-032, Procarbazine, Vatalanib, Rosiglitazone, BMS 794833, Irinotecan, Elesclomol, Vismodegib, TEMOZOLOMIDE, Triciribine, Dovitinib, SB 431542, Regorafenib, Gossypol, Ganetespib, R406, Streptozotocin, APO866, YM201636, Canagliflozin, Brivanib, Telatinib, E7080, Amuvatinib, GW4064, Clofarabine, Dapagliflozin, Thalidomide, GDC-0879, |
| 0.9-1 | Oxaliplatin, Cisplatin, Carboplatin, Capecitabine, Estrone, Formestane, Letrozole, EX 527Tozasertib, Cediranib, Quercetin, AZ 3146, OSI-930, LDE225, (-)-Epigallocatechin gallate, Exemestane, Cyclosporin A, CP-466722, Enzastaurin, LY2157299, SGX-523, Tretinoin, PAC-1, Vorinostat, Quizartinin, Cyclophosphamide, PF-03814735, Saracatinib, SirtinolPhloretin, SB 216763, Anagrelide, Pioglitazone, Lonidamine, BMS 777607, Flutamide, Doxercalciferol, SB 525334, Amcasertib, Sodium butyrate, Abiraterone, WAY-362450, TAME, WZ4002, 3-Methyladenine, Mesna, CI-1040, Disulfiram, Medroxyprogesterone acetate, Dimesna, Trichostatin A, Rigosertib, Bendamustine, Vinpocetine, Megestrol Acetate, KX2-391, XAV-939, Azacitidine, AEE788, Nelarabine, WYE-354, Cyclophosphamide monohydrate, Anastrozole, Prednisone, Cyclopamine, Vandetanib, Salinomycin, Vemurafenib, MK-1775, Iniparib, Y-27632, MK-0752, Ubenimex, A-769662, Nilotinib, Chrysophanic acid, Leucovorin, LY2109761, Mifepristone, S-Ruxolitinib, Zibotentan, Valproic acid sodium salt, Andarine, Epirubicin, Pomalidomide, DMXAA, Rucaparib, Tofacitinib, PIK-75, Cladribine, GSK690693, JNJ-38877605, |
| 1< | GW3965, AZ628, PD173074, PH-797804, Erlotinib, PD153035, XL147, CH5132799, Axitinib, ABT-888, XL765, Ranolazine, Hydroxyurea, Isotretinoin, CAL-101, Fludarabine, BAY 11-7082, TW-37, Maraviroc, Lenalidomide, YM155, ENMD-2076, Flavopiridol, IC-87114, DAPT, AT-406, Zileuton, Lomustine, PIK-90, Imiquimod, PF-04217903, MLN9708, D-glutamine, TG101348, Rebastinib, RG108, YO-01027, Pamidronate, Alisertib, Pemetrexed, Itraconazole, PF-3845, Febuxostat, Aprepitant, Tosedostat, Tandutinib, LY294002, NU7441, Betapar, SU11274, ZSTK474, Triamcinolone Acetonide, TAE684, Celecoxib, Belinostat, PF 573228, JNJ-26481585, Silmitasertib, SB590885, Bleomycin sulfate, Carmofur, Bicalutamide, Gemcitabine HCl, BIRB 796, BTZ043, ABT-737, Dalcetrapib, CEP33779, Dacarbazine, Pazopanib, Enzalutamide, Gefitinib, BI 2536, Busulfan, Pracinostat, Desmethyl Erlotinib, Ostarine, LDN193189, Dexamethasone acetate, Fostamatinib, Docetaxel, AG14361, CUDC-101, Vargatef, Neratinib, GDC-0941, Aminoglutethimide, KU-55933, Gemcitabine, PD0325901, Afatinib, Roscovitine, |

Test Example 4: Evaluation of enhanced inhibitory effect on sphere-forming ability

According to the following procedures, the enhanced inhibitory effect of a sphere-forming ability inhibitor on sphere-forming ability in combination with a test compound can be evaluated.

Cultured cancer cells are suspended in the sphere formation medium (DMEM/F12 medium of Test Example 3

was used) and seeded in a 384-well plate with Ultra-Low Attachment surface (Corning) in an amount of $3x10^3$-$10x10^3$ cells/well. A sphere-forming ability inhibitor is then added into each well to adjust the final concentration to 2-1000 nmol/L. A test compound is added into each well in various concentrations, and the plate is cultured at 37°C and 5% carbon dioxide in an incubator for 4 days. After the culture, CellTiterGlo (Promega) is added into all of the wells, and the plate is allowed to stand at room temperature for 10 minutes to measure the intensity of luminescence of each well. The intensity of luminescence of each well with the inhibitor (Lsample) is divided by that of the well containing only cells with no compound (Lcontrol) to calculate the sphere formation rate. The formula for calculating the rate is shown below.

$$\text{Sphere formation rate (\%)} = (\text{Lsample})/(\text{Lcontrol}) \times 100$$

The value obtained by dividing the sphere formation rate by 100 and subtracting the calculated value from 1 is defined as a sphere formation inhibitory effect. The formula for calculating the rate is shown below.

$$\text{Sphere formation inhibitory effect} = 1 - (\text{Sphere formation rate}/100)$$

CombinationIndex (CI) is calculated from the culculated sphere formation inhibitory effect using Calcusyn software (Biosoft). When the sphere formation inhibitory effect of a test compound alone is less than 0.5 and CI is not calculated, the theoretical sphere formation rate of the combination of the test compound (Lc) is defined as the value calculated by multiplying the sphere formation rate of a sphere-forming ability inhibitor alone (La) and the sphere formation rate of the test compound alone (Lb) together. Also, the measured sphere formation rate of the combination is defined as (Ld).

The (Ld)/(Lc) values of all combinations of the sphere-forming ability inhibitor and each test compound in each concentration are calculated. The minimum value thereof is used as the criteria for evaluation.

Test Example 5: Exploration of combination drug for enhancing inhibitory effect on cell proliferation

Various types of cultured cancer cells (colon cancer-derived HCT116 cells, lung cancer-derived H460 cells) were suspended in the medium containing 10% FBS and seeded in a 384-well culture plate (Greiner) in an amount of $3X10^3$-$8x10^3$ cells/well. The compound of Example 24 was then added into each well to adjust the final concentration to 10-1000 nmol/L. Test Compounds for various concentrations were then added in each well, and the plate was cultured at 37°C and 5% carbon dioxide in an incubator for 4 days. After the culture, CellTiterGlo (Promega) was added into all of the wells, and the plate was allowed to stand at room temperature for 10 minutes to measure the intensity of luminescence of each well. The intensity of luminescence of each well with the compound of Example 24 (Lsample) was divided by that of the well containing only cells with no compound (Lcontrol) to calculate the cell survival rate. The formula for calculating the rate is shown below.

$$\text{Cell survival rate (\%)} = (\text{Lsample})/(\text{Lcontrol}) \times 100$$

The theoretical cell survival rate of a combination of the compound of Example 24 and a test compound (Lc) was defined as the value calculated by multiplying the cell survival rate of the compound of Example 24 alone (La) and the cell survival rate of a test compound alone (Lb) together. Also, the measured cell survival rate of the combination was defined as (Ld).

The (Ld)/(Lc) values of all combinations of the compound of Example 24 and each test compound in each concentration were calculated.

The minimum value of the values (sCI) was used as evaluation criteria for screening. The minimum sCI value for each test compound is shown in Table below.

| HCT116 | |
|---|---|
| **sCI** | **Test Compound** |
| 0.6-0.7 | Crenolanib, BKM120, ABT-751, |
| 0.7-0.8 | MLN9708, Etoposide, |

(continued)

| HCT116 | |
|---|---|
| **sCI** | **Test Compound** |
| 0.8-0.9 | Simvastatin, BIIB021, SGI-1776, Napabucasin, Thalidomide, Valproic acid sodium salt, Alisertib, Ganetespib, Torin 2, Rosiglitazone, |
| 0.9-1 | Tamoxifen, Fluvastatin, Irinotecan HCl Trihydrate, Ftorafur, Temozolomide, Cisplatin, Carboplatin, Gemcitabine, Pazopanib, Sunitinib, Fingolimod, Pamidronate, PAC-1, CYC116, Belinostat, Andarine, JNJ 26854165, Triptolide, XAV-939, Bosutinib, Floxuridine, Capecitabine, Tofacitinib, ENMD-2076, PHA-665752, Imiquimod, BAY 11-7082, Regorafenib, AT9283, Abiraterone, SB590885, GW4064, YM155, Mycophenolate, Exemestane, RG108, Daunorubicin, Letrozole, Roscovitine, Chrysophanic acid, Crizotinib, Amuvatinib, E7080, Idarubicin, 2-Methoxyestradiol, KX2-391, Vemurafenib, Vorinostat, BMS-599626, APO866, SB 525334, Dacomitinib, Maraviroc, Bleomycin sulfate, Fulvestrant, Imatinib, Docetaxel, Brivanib, STF-62247, AR-42, Tretinoin, Raltitrexed, Sodium butyrate, CEP33779, Vandetanib, AMG 900, Ostarine, BTZ043, LY2109761, Salinomycin, GSK2126458,Hydrocortisone, Teniposide, NU7441, (-)-Epigallocatechin gallate, Disulfiram, Megestrol Acetate, Nelarabine, Nilotinib, Bexarotene, Ifosfamide, Epothilone A, Dexamethasone acetate, Vinpocetine, Leucovorin, LDE225, CAL-101, Mesna, 17-DMAG, Cediranib, Palbociclib, JNJ-26481585, Cyclophosphamide monohydrate, Tanespimycin, Gefitinib, PD173074, MK-0752, Rucaparib, Geldanamycin, Raloxifen, Busulfan, Formestane, Vismodegib, Prednisone, SNS-032, AT-406, SB 431542, SB 216763, Pomalidomide, Estradiol, Fludarabine Phosphate, WZ4002, Vincristine, KU-60019, AEE788, Ponatinib, PF-03814735, Celecoxib, Dexamethasone, CUDC-101, Sirtinol, Neratinib, Ibrutinib, Coenzyme Q10, GSK1904529A, Gossypol, Bendamustine, Tozasertib, Telatinib, Mocetinostat, Amcasertib,Tie2 kinase inhibitor, OSI-930, BMS 794833, 3-Methyladenine, Dimesna, S-Ruxolitinib, Topotecan, Enzalutamide, Cyclopamine, Tivozanib, JNJ-7706621, OSI-420, GDC-0879, Medroxyprogesterone acetate, AZ628, Navitoclax, Aprepitant, Cyt387, CI-1040, DMXAA, Itraconazole, D-glutamine, Flutamide, Imatinib Mesylate, TG101348, TAME, Quercetin, |
| 1< | AZ 3146, Streptozotocin, BX-795, Nocodazole, Desmethyl Erlotinib, Volasertib, CP-466722, Doxorubicin, XL147, Barasertib, Ku-0063794, Danusertib, AUY922, PF-562271, YO-01027, IC-87114, TAE684, SB 743921, Enzastaurin, AT7519, Anastrozole, NVP-BSK805, Paclitaxel, CH5132799, Mifepristone, Elesclomol, Flavopiridol, WP1130, Altretamine, GSK461364, Ranolazine, AZD8055, JNJ-38877605, LY2603618, Deforolimus, XL765, R406, Dalcetrapib, Hydroxyurea, Lenalidomide, Olaparib, Triamcinolone Acetonide, Quizartinin, Anagrelide, SRT1720, Oxaliplatin, A-769662, Sorafenib, Dapagliflozin, PD0325901, Bicalutamide, SGX-523, Ruxolitinib, Tandutinib, Fludarabine, Trametinib, Iniparib, LY294002, AZD7762, Lapatinib, BIBR 1532, LY2157299, CHIR-99021, Febuxostat, KU-55933, LDN193189, Dacarbazine, Dovitinib, PIK-90, BI 2536, PH-797804, WYE-354, PF 573228, Aminoglutethimide, Procarbazine, Lonidamine, Erlotinib, LY2228820, Cyclosporin A, PF-04217903, EX 527, Phloretin, Motesanib, Linsitinib, Betapar, Cyclophosphamide, Toremifene, PCI-24781, Abitrexate, Pioglitazone, Obatoclax, AG14361, Decitabine, MLN2238, Fostamatinib, Cladribine, PF-3845, Ubenimex, Canagliflozin, BIRB 796, TW-37, TPCA-1, Cytarabine, Lapatinib Ditosylate, Ezetimibe, TAK-733, Vatalanib, ZSTK474, WAY-362450, ABT-888, Sotrastaurin, Palomid 529, Mercaptopurine, Dasatinib, INK 128, Pracinostat, BMS 777607, Tosedostat, Torin 1, PIK-75, Y-27632, Gemcitabine HCl, YM201636, Carmofur, MK-1775, Lomustine, Entinostat, GW3965, Pemetrexed, Vinblastine, Rapamycin, Axitinib, Everolimus, Pelitinib, Trichostatin A, Clofarabine, Azathioprine, Nutlin-3, Dorzolamide, BEZ235, Temsirolimus, DAPT, SNS-314, Rebastinib, Epirubicin, Adrucil, Bortezomib, Mycophenolate mofetil, Zileuton, PD153035, Isotretinoin, GDC-0941, Epothilone B, ABT-737, GSK690693, Saracatinib, Silibinin, Masitinib, Triciribine, Linifanib, PIK-93, Rigosertib, SB 203580, Silmitasertib, Doxercalciferol, PI-103, Mitoxantrone, SU11274, MK-2206, PHA-793887, Vargatef, AZD6244, Estrone, Tipifarnib, Zibotentan, Afatinib, Azacitidine, Ispinesib, |
| **H460** | |
| **sCI** | **Test Compound** |
| <0.5 | Pazopanib, Tipifarnib, Dexamethasone acetate, CH5132799, PIK-75, Dexamethasone, TG101348, CHIR-99021, Pracinostat, CUDC-101, Vorinostat, SB590885, PCI-24781, |
| 0.5-0.6 | BIIB021, Trichostatin A, AUY922, Rigosertib, MLN9708, |

(continued)

| H460 | |
|---|---|
| sCI | Test Compound |
| 0.6-0.7 | Docetaxel, Temozolomide, ENMD-2076, Etoposide, Abitrexate, LY2157299, Mercaptopurine, YO-01027, Mycophenolate mofetil, Fludarabine, SB 431542, LY2109761, Napabucasin, Fludarabine Phosphate, Estradiol, Quercetin, Deforolimus, Betapar, SGX-523, Tanespimycin, Doxercalciferol, Doxorubicin, Epirubicin, Daunorubicin, Sotrastaurin, |
| 0.7-0.8 | Ftorafur, Irinotecan, Tie2 kinase inhibitor, Raltitrexed, Mifepristone, JNJ-38877605, Rosiglitazone, 17-DMAG, AZD8055, Tozasertib, PHA-665752, ABT-888, Idarubicin, Cyclopamine, Mycophenolate, Nelarabine, Palomid 529, Sodium butyrate, Valproic acid sodium salt, Barasertib, Bexarotene, Maraviroc, Megestrol Acetate, Anagrelide, YM201636, SRT1720MLN2238, WZ4002, Afatinib, Coenzyme Q10, Pioglitazone, Rebastinib, Pemetrexed, MK-1775, Torin 1, Vinpocetine, Silmitasertib, Enzastaurin, Isotretinoin, Axitinib, Imatinib, Chrysophanic acid, Cyclophosphamide monohydrate, Rapamycin, D-glutamine, NVP-BSK805, Palbociclib, Mitoxantrone, APO866, Ifosfamide, Roscovitine, Formestane, Thalidomide, Bortezomib, Cyclosporin A, Ranolazine, Dimesna, GDC-0879, WAY-362450, GSK2126458, MK-0752, AZ 3146, XL147, Ezetimibe, (-)-Epigallocatechin gallate, Estrone, Epothilone A, Vismodegib, Pamidronate, Danusertib, Lonidamine, Tivozanib, GSK1904529A, Vatalanib, Anastrozole, PF-04217903, Phloretin, Prednisone, PF-03814735, Cyclophosphamide, XL765, A-769662, PD173074, Fulvestrant, DAPT, Disulfiram, |
| 0.8-0.9 | Raloxifen, Toremifene, Tamoxifen, Cisplatin, Lapatinib, Sunitinib, Sorafenib, Simvastatin, Bicalutamide, Fingolimod, TAME, TW-37, SNS-314, Dalcetrapib, Capecitabine, AMG 900, Exemestane, Itraconazole, CP-466722, Dorzolamide, Aprepitant, BMS 777607, Imiquimod, Letrozole, Leucovorin, Nilotinib, Hydroxyurea, STF-62247, TAE684, Celecoxib, Streptozotocin, Ostarine, Febuxostat, BIBR 1532, Abiraterone, Ubenimex, PD153035, BX-795, SNS-032, PF 573228, Pomalidomide, Telatinib, Temsirolimus, Bleomycin sulfate, Tofacitinib, LY294002, XAV-939, SB 216763, Quizartinin, Andarine, 3-Methyladenine, Procarbazine, Neratinib, NU7441, Medroxyprogesterone acetate, Everolimus, BMS-599626, Mesna, Iniparib, PAC-1, Epothilone B, CAL-101, Dapagliflozin, KU-55933, PIK-90, E7080, R406, Aminoglutethimide, IC-87114, Lomustine, Dacomitinib, BAY 11-7082, Regorafenib, GW4064, DMXAA, SB 743921, SGI-1776, Tandutinib, EX 527, Bendamustine, Nocodazole, AT7519, Ibrutinib, Flavopiridol, BMS 794833, Sirtinol, Canagliflozin, Gefitinib, Y-27632, Vemurafenib, Busulfan, GW3965, Enzalutamide, Dacarbazine, Floxuridine, Amuvatinib, Linifanib, JNJ 26854165, ABT-751, Clofarabine, Crenolanib, Altretamine, Linsitinib, Ispinesib, |
| 0.9-1 | Oxaliplatin, Lapatinib Ditosylate, RG108, Pelitinib, Amcasertib, Cediranib, Ruxolitinib, S-Ruxolitinib, LDE225, AT-406, KU-60019, SU11274, WYE-354, Lenalidomide, BEZ235, OSI-930, Vargatef, BIRB 796, Motesanib, KX2-391, SB 203580, PF-3845, Navitoclax, Fostamatinib, CEP33779, Adrucil, Hydrocortisone, Flutamide, Mocetinostat, PI-103, Carmofur, Vandetanib, SB 525334, Triamcinolone Acetonide, Tretinoin, Belinostat, Saracatinib, AG14361, Obatoclax, Zibotentan, Bosutinib, Geldanamycin, Gossypol, |
| 1< | Topotecan, Tosedostat, 2-Methoxyestradiol, ABT-737, PH-797804, AZ628BTZ043, GSK461364, WP1130, Gemcitabine, LDN193189, Volasertib, Ganetespib, ZSTK474, LY2228820, AEE788, Zileuton, AR-42, Cyt387, Carboplatin, Entinostat, Triptolide, Masitinib, Desmethyl Erlotinib, Dasatinib, INK 128, Azathioprine, Triciribine, OSI-420, Erlotinib, Vinblastine, Cladribine, Dovitinib, YM155, CI-1040, AZD6244, BKM120, Gemcitabine HCl, PIK-93, Alisertib, GDC-0941, CYC116, TAK-733, Fluvastatin, Rucaparib, Crizotinib, Ponatinib, Decitabine, PD0325901, GSK690693, AT9283Azacitidine, Paclitaxel, Olaparib, Vincristine, Elesclomol, BI 2536, Trametinib, Teniposide, PHA-793887, Ku-0063794, Salinomycin, Cytarabine, MK-2206, PF-562271, Torin 2, Brivanib, Nutlin-3, LY2603618, AZD7762, TPCA-1, JNJ-7706621, JNJ-26481585, |

Test Example 6: Exploration of combination durg for enhancing inhibitory effect on the sphere-forming ability

HCT116 cells, H460 cells, or LNCap cells were suspended in the sphere formation medium (DMEM/F12 medium of Test Example 3 was used) and seeded in a 384-well plate with Ultra-Low Attachment surface (Corning) in an amount of 5x$10^3$-10x$10^3$ cells/well. The compound of Example 24 was then added into each well to adjust the final concentration to 2-1000 nmol/L. A test compound is added into each well in various concentrations, and the plate was cultured at 37°C and 5% carbon dioxide in an incubator for 4 days. After the culture, CellTiterGlo (Promega) was added into all of the

wells, and the plate is allowed to stand at room temperature for 10 minutes to measure the intensity of luminescence of each well. The intensity of luminescence of each well with the compound of Example 24 (Lsample) was divided by that of the well containing only cells with no compound (Lcontrol) to calculate the sphere formation rate. The formula for calculating the rate is shown below.

$$\text{Sphere formation rate (\%)} = (\text{Lsample})/(\text{Lcontrol}) \times 100$$

The value obtained by dividing the sphere formation rate by 100 and subtracting the calculated value from 1 is defined as a sphere formation inhibitory effect. The formula for calculating the effect is shown below.

$$\text{Sphere formation inhibitory effect} = 1 - (\text{Sphere formation rate}/100)$$

The theoretical cell survival rate of a combination of the compound of Example 24 and a test compound (Lc) was defined as the value calculated by multiplying the cell survival rate of the compound of Example 24 alone (La) and the cell survival rate of a test compound alone (Lb) together. Also, the measured cell survival rate of the combination was defined as (Ld).

The (Ld)/(Lc) values of all combinations of the compound of Example 24 and each test compound in each concentration were calculated. The minimum value thereof (sCI) was used as the criteria for screening. The minimum value of sCI of each test compound is shown in Table below.

| HCT116 | |
|---|---|
| Test Compound | sCI |
| Toremifen | 0.020 |
| Raloxifen | 0.010 |
| Tamoxifen | 0.046 |
| BBI-503 | 0.017 |
| Sunitinib | 0.062 |
| BBI-608 | 0.159 |
| Fingolimod | 0.013 |
| Fluvastatin | 0.163 |
| SRT1720 | 0.007 |
| SGI-1776 | 0.026 |
| BI-2536 | 0.623 |
| Obatoclax | 0.103 |
| Dasatinib | 0.171 |
| Lapatinib | 0.024 |
| Simvastatin | 0.257 |
| Lovastatin | 0.150 |
| Pravastatin | 0.810 |
| Atorvastatin | 0.3 |

| H460 | |
|---|---|
| Test Compound | sCI |
| Pazopanib | 0.507 |
| Palomid 529 | 0.428 |
| Doxercalciferol | 0.304 |
| CHIR-99021 | 0.378 |
| JNJ-38877605 | 0.711 |
| Fingolimod | 0.007 |
| Dasatinib | 0.514 |
| BMS-599626 | 0.081 |
| LY2228820 | 0.020 |
| Masitinib | 0.010 |
| Lapatinib | 0.076 |
| Vorinostat | 0.573 |
| Tipifarnib | 0.564 |
| 17-AAG | 0.386 |
| AZD8055 | 0.651 |
| Obatoclax | 0.061 |
| Gossypal | 0.026 |
| STF62247 | 0.221 |
| Linfanib | 0.142 |
| GSK-1904529A | 0.166 |
| Sotrastaurin | 0.030 |

| LNCap | |
|---|---|
| Test Compound | sCI |
| Bicalutamide | 0.421 |
| Enzalutamide | 0.826 |

| | |
|---|---|
| Tie 2 kinase inhibitor | 0.123 |

Test Example 7: Exploration of combination durg for enhancing inhibitory effect on tumor growth in tumor-bearing mouse model

Various cultured cancer cells (human colon cancer-derived HCT116 cells, human colon cancer-derived Colo205 cells, human lung cancer-derived H460 cells, mouse colon cancer-derived CT26 cells) were treated with trypsin and collected, the collected cells were suspended in PBS, HBSS, PBS containing 50% Matrigel (Corning), or HBSS containing 50% Matrigel. BALB/c-nu/nu mice or BALB/c mice were used, and the cells were subcutaneously transplanted on the ventral side of the mice in an amount of $2\times10^5$-$5\times10^6$ cells per mouse, and expanded until the tumor was palpable. The major axis and the minor axis of the tumor were then measured with a caliper every 3-4 days, and the measured values were applied to the formula: (minor axis)$^2$ x (major axis)/2 to calculate the tumor volume. When the tumor volume reached about 80-230 mm$^3$, the mice were classified into the following 4 groups and received drug administration. The tumor volume was measured every 3-4 days during the administration period.

(1) Untreated group
(2) Sphere-forming ability inhibitor single administration group
(3) Test compound single administration group
(4) Sphere-forming ability inhibitor-test compound combination administration group

The major axis and the minor axis of the tumor were measured with a caliper every 3-4 days during the administration period, and the measured values were applied to the formula: (minor axis)$^2$ x (major axis)/2 to calculate the tumor volume. The results are shown in Fig. 1.

Test Example 8-1: Evaluation of enhanced inhibitory effect on tumor growth in tumor-bearing mouse model

According to the following procedures, the enhanced inhibitory effect of a sphere-forming inhibitor on tumor growth in combination with a test compound can be evaluated in a tumor-bearing mouse model.

Various cultured cancer cell lines are treated with trypsin and collected, the collected cells are suspended in PBS, HBSS, PBS containing 50% Matrigel (Corning), or HBSS containing 50% Matrigel. Female or male immunodeficient mice or wild-type mice are used, and the cells are subcutaneously transplanted on the ventral side of the mice in an amount of $0.2$-$10\times10^6$ cells per mouse, and expanded until the tumor is palpable. The major axis and the minor axis of the tumor are then measured with a caliper every 3-4 days, and the measured values are applied to the formula: (minor axis)$^2$ x (major axis) /2 to calculate the tumor volume. When the tumor volume reaches about 80-230 mm$^3$, the mice are classified into the following 4 groups and receive drug administration.

(1) Untreated group
(2) Sphere-forming ability inhibitor single administration group
(3) Test compound single administration group
(4) Sphere-forming ability inhibitor-test compound combination administration group

The major axis and the minor axis of the tumor are measured with a caliper every 3-4 days during the administration period, and the measured values are applied to the formula: (minor axis)$^2$ x major axis [mm]/2 to calculate the tumor volume. The tumor volume is measured over time from the start of administration to evaluate the effect for reducing the tumor volume of the compound administration groups.

Test Example 8-2: Evaluation of enhanced inhibitory effect on tumor growth in tumor-bearing mouse model

According to the following procedures, the enhanced inhibitory effect of a sphere-forming ability inhibitor on tumor growth in combination with the test compound which is tamoxifen, raloxifene, toremifene, fulvestrant, chlormadinone, bicalutamide, enzalutamide, goserelin, buserelin, leuprorelin, degarelix, anastrozole, letrozole, exemestane, abiraterone, ramucirumab, and aflibercept can be evaluated in a tumor-bearing mouse model.

Various cultured cancer cell lines are treated with trypsin and collected, the collected cell lines are suspended in PBS, HBSS, PBS containing 50% Matrigel (Corning), or HBSS containing 50% Matrigel. Female or male immunodeficient mice (pretransplanted with estrogen-containing pellet or with no pre-treatment) or wild-type mice are used, and

the cells are subcutaneously transplanted on the ventral side of the mice in an amount of 0.2-10$\times$$10^6$ cells per mouse, and expanded until the tumor is palpable. (The estrogen-containing pellet may be pre-placed subcutaneously for the mice for transplantation.) The major axis and the minor axis of the tumor are then measured with a caliper every 3-4 days, and the measured values are applied to the formula: (minor axis)$^2$ x (major axis)/2 to calculate the tumor volume. When the tumor volume reaches about 80-230 $mm^3$, the mice are classified into the following 4 groups and receive drug administration.

(1) Untreated group
(2) Sphere-forming ability inhibitor single administration group
(3) Test compound single administration group
(4) Sphere-forming ability inhibitor-test compound combination administration group

The major axis and the minor axis of the tumor are measured with a caliper every 3-4 days during the administration period, and the measured values are applied to the formula: (minor axis)$^2$ x major axis [mm]/2 to calculate the tumor volume. The tumor volume is measured over time from the start of administration to evaluate the effect for reducing the tumor volume of the compound administration groups.

Test Example 9: Evaluation of anti-tumor effect in glioma cell line-orthotopically transplanted mouse model

According to the following procedures, the enhanced anti-tumor effect of a sphere-forming ability inhibitor in combination with test compounds can be evaluated in a glioma cell line-orthotopically transplanted mouse model.
A plasmid encoding firefly luciferase gene are introduced into the human glioma cell line U87-MG to establish luciferase expression strains (hereinafter, firefly luciferase expression U87-MG cell line is also referred to as U87-MG-Luc). BALB/c-nu/nu mice are used, the U87-MG-Luc cells in an amount of 1$\times$$10^6$-5$\times$$10^6$ cells per mouse are suspended in HBSS, and the suspended cells are transplanted into the brain of the mice. VivoGlo Luciferin (Promega) is administered via tail vein of the mice every 1 week after the transplantation, and the intensity of luminescence for the head of the mice is measured with IVIS Lumina (PerkinElmer). The mice are classified into the following 4 groups 1-3 weeks after the transplantation, and receive drug administration.

(1) Untreated group
(2) Sphere-forming ability inhibitor single administration group
(3) Test compound single administration group
(4) Sphere-forming ability inhibitor-test compound combination administration group

The intensity of luminescence for the head is measured every other week during the administration period. The intensity of luminescence for the head is measured over time to evaluate the anti-tumor effect of the compound administration groups.

Test Example 10: Test for inhibiting sphere-forming ability of cultured cancer cell lines

The colon cancer cell lines SW480, DLD1, HCT15, HT29, SW948, LS174T, LS411N, SW620, LoVo, HCT116, Colo205, and Hs698T, the pancreatic cancer cell lines MiaPaca2, HPAFII, Capan2, and Panc1, the head and neck cancer cell line FaDu, the breast cancer cell lines HCC1954 and T47D, the bladder cancer cel line SW780, the liver cancer cell line HepG2, the neuroblastoma cell lines U87MG and U251, the lung cancer cell lines H460, H1437, and A549, the prostate cancer cell lines DU145 and PC3, the soft tissue tumor cell line HT1080, the testicular cancer cell line NTERA2, and the ovarian cancer cell line SKOV3 was suspended in the sphere formation medium (DMEM-F12 medium of Test Example 3 was used), and seeded in a 384-well plate with Ultra-Low Attachment surface (Corning) in an amount of Sx$10^3$-10x$10^3$ cells/well. The compound of Example 24 was then added into each well to adjust the final concentration to 10-10000 nmol/L. The plate was cultured at 37°C and 5% carbon dioxide in an incubator for 4 days. After the culture, CellTiterGlo (Promega) was added into all of the wells, and the plate was allowed to stand at room temperature for 10 minutes to measure the intensity of luminescence of each well. The concentrations of each test compound for 50% inhibition of cell proliferation to the cell lines (Sphere $IC_{50}$ value; $\mu$mol/L) was calculated from the measured intensity of luminescence. The Sphere $IC_{50}$ values and the gene mutations in Wnt/$\beta$-catenin pathway for each cell line are shown in Table below. When each gene mutation in Table is reported, the mutation is represented by "+" (gene mutation information is cited from the ATCC website (https://www.atcc.org/en/Products/Cells_and_Microorganisms/ Cell_Lines/Cell_lines_by_genetic_mutation.aspx), Cancer Cell Line Encyclopedia (https://portals.broadinstitute.org/ccle_legacy/home), Proc. Natl. Acad. Sci. USA 94 (1997) 10330-10334).
It was demonstrated from the test results that the present compound had remarkably potent inhibitory effect

on sphere-forming ability of cancer cells in cancer cells with gene mutations in the Wnt/β-catenin pathway such as APC gene mutation, CTNNB1 gene mutation, AXIN1 gene mutation, and Axin2 gene mutation. Thus, it was demonstrated that the present compound had remarkably potent anti-tumor effect in the treatment of cancers with the above gene mutations.

| Cell line name | APC mutation | CTNNB1 mutation | AXIN1 mutation | AXIN2 mutation | sphere $IC_{50}$ (μmol/L) |
|---|---|---|---|---|---|
| SW480 | + | | | | 0.004 |
| DLD-1 | + | | | | 0.007 |
| HCT-15 | + | | + | | 0.008 |
| MIA PaCa-2 | | | | | 0.008 |
| HT-29 | + | | | | 0.009 |
| SW948 | + | | | | 0.009 |
| FaDu | | | | | 0.009 |
| LS 174T | | + | | | 0.015 |
| LS411N | + | | | | 0.019 |
| HCC1954 | | | | | 0.023 |
| SW620 | + | | | | 0.027 |
| LoVo | + | | | + | 0.028 |
| T-47D | | | | | 0.046 |
| SW 780 | | | | | 0.056 |
| Hep G2 | | | | | 0.072 |
| U-87 MG | | | | | 0.089 |
| HCT 116 | | + | | | 0.091 |
| COLO 205 | + | | | | 0.142 |
| HPAF-II | | | | | 0.764 |
| NCI-H460 | | | | | 0.883 |
| NCI-H1437 | | | | | 3,875 |
| A549 | | | | | >10 |
| Capan-2 | | | | | >10 |
| DU 145 | | + | | | >10 |
| Hs 698.T | | | | | >10 |
| HT-1080 | | | | | >10 |
| NTERA-2 | | | | | >10 |
| Panc-1 | | | | | >10 |
| PC-3 | | | | | >10 |
| SK-OV-3 | | | | | >10 |
| U-251 | | | | | >10 |

Also, it can be demonstrated according to the following clinical test that the anti-tumor agent of the present invention is useful in the treatment of cancer.

The subjects are patients diagnosed with cacer. The subjects are randomly classified into placebo administration group and test compound administration group, and receive continuous administration of the drug. The effectiveness for various items such as tumor regression effect, tumor progression-free survival, and overall survival is evaluated.

When the test compound administration group achieves a statistically significant improvement of the effectiveness as compared to the placebo administration group, it is determined that the test compound is effective against cancer. The response Evaluation Criteria in Solid Tumors (RECIST) is used as the criteria for tumor regression effect, and one of Complete Response (CR), Partial Response (PR), Stable Disease (SD), and Progressive Disease (PD) is determined according to the criteria.

INDUSTRIAL APPLICABILITY

The pharmaceutical composition of the present invention has an inhibitory effect on self-renewal ability of cancer stem cells, and is useful as an anti-tumor agent.

## Claims

1. An anti-tumor agent comprising a compound of formula (1) :

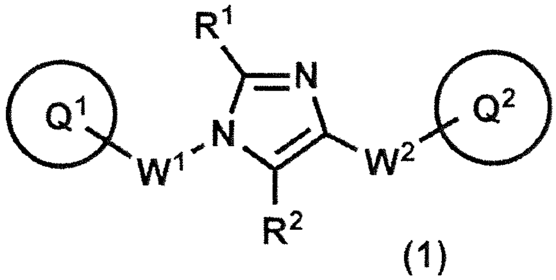

(1)

or a pharmaceutically acceptable salt thereof, wherein $Q^1$ is optionally-substituted $C_{6-10}$ aryl, optionally-substituted $C_{6-10}$ aryloxy, optionally-substituted $C_{6-10}$ arylthio, optionally-substituted $C_{3-10}$ cycloalkyl, or optionally-substituted 5- to 10-membered heteroaryl;
$R^1$ and $R^2$ are independently hydrogen atom, halogen atom, or $C_{1-6}$ alkyl which may be optionally substituted with the same or different 1 to 3 halogen atoms;
$W^1$ is $C_{1-4}$ alkylene which may be optionally substituted with 1 to 3 fluorine atoms or $C_{3-7}$ cycloalkyl;
$W^2$-$Q^2$ is $-NR^{3a}C(O)-Q^2$, $-NR^{3a}C(O)O-Q^2$, $-NR^{3a}C(O)OCH_2-Q^2$, - $NR^{3a}C(O)NR^{3b}-Q^2$, $-NR^{3a}C(O)NR^{3b}CH_2-Q^2$, $-NR^{3a}C(O)CH_2O-Q^2$, - $NR^{3a}C(O)CH_2-Q^2$, $-NR^{3a}C(O)CH_2CH_2-Q^2$, $-C(O)N^{3a}-Q^2$, $-C(O)NR^{3a}CH_2-Q^2$, $-C(O)NR^{3a}CH_2CH_2-Q^2$, or $-NR^{3a}C(O)-CR^{4c}=CR^{4d}-Q^2$ wherein $R^{3a}$ and $R^{3b}$ are independently hydrogen atom or $C_{1-6}$ alkyl; $R^{3c}$ and $R^{3d}$ are independently hydrogen atom, fluorine atom, or $C_{1-6}$ alkyl;
ring $Q^2$ is optionally-substituted $C_{6-10}$ aryl, or optionally-substituted 5- to 10-membered heteroaryl, in combination with at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof.

2. The anti-tumor agent according to claim 1, wherein $Q^1$ is phenyl which may be optionally substituted with the same or different 1 to 4 groups selected from the group consisting of halogen atom, and $C_{1-6}$ alkyl which may be optionally substituted with the same or different 1 to 3 halogen atoms;
$W^1$ is methylene;
$W^2$-$Q^2$ is $-NHC(O)-Q^2$, $-NHC(O)-CH=CH-Q^2$, $-C(O)NH-Q^2$, or - $NHC(O)CH_2O-Q^2$ ;
$R^1$ and $R^2$ are hydrogen atom;
ring $Q^2$ is

(1) phenyl which may be optionally substituted with the same or different 1 to 4 groups selected from the group consisting of

(a) halogen atom,
(b) $C_{1-6}$ alkyl which may be optionally substituted with the same or different 1 to 3 groups selected from the group consisting of halogen atom, hydroxy, and $C_{1-6}$ alkoxy,
(c) $C_{1-6}$ alkoxy which may be optionally substituted with the same or different 1 to 3 groups selected from the group consisting of halogen atom, hydroxy, and $C_{1-6}$ alkoxy,
(d) $C_{3-7}$ cycloalkyl,

(e) $C_{2-6}$ alkenyl,
(f) cyano,
(g) amino which may be optionally substituted with the same or different 1 to 2 $C_{1-6}$ alkyl groups, and
(h) $C_{1-6}$ alkyl-carbonylamino,

(2) 5- or 6-membered heteroaryl which may be optionally substituted with the same or different 1 to 4 groups selected from the group consisting of (a)-(h) defined in the above (1), or
(3) a group of the following formula (11), (12), (13), (14), (15), or (16):

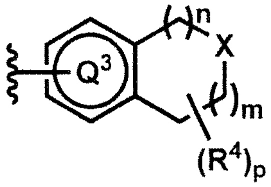
(11)

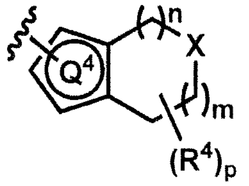
(12)

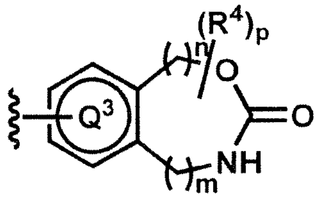
(13)

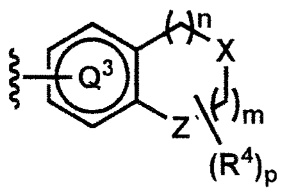
(14)

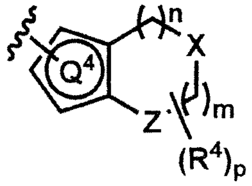
(15)

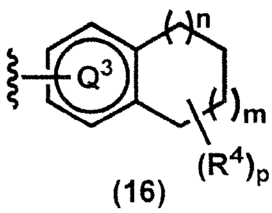
(16)

wherein ring $Q^3$ is optionally-substituted benzene ring, optionally-substituted pyridine ring, optionally-substituted pyrimidine ring, optionally-substituted pyridazine ring, or optionally-substituted pyrazine ring;
ring $Q^4$ is optionally-substituted 5-membered heteroaryl ring;
n and m are independently 0, 1, or 2, provided that n and m are not simultaneously 0;
X and Z are independently $NR^5$, $-NR^{3e}C(O)-$, $-C(O)NR^{3e}-$, or O wherein $R^5$ is hydrogen atom, $C_{1-6}$ alkyl which may be optionally substituted with the same or different 1 to 3 halogen atoms, or $C_{1-6}$ alkylcarbonyl; $R^{3e}$ is hydrogen atom or $C_{1-6}$ alkyl;
p is 1, 2, 3, 4, or 5;
$R^4$ is, independently when two or more exist, hydrogen atom, halogen atom, hydroxy, oxo, $C_{1-6}$ alkyl which may be optionally substituted with the same or different 1 to 3 halogen atoms, or $C_{1-6}$ alkoxy which may be optionally substituted with the same or different 1 to 3 halogen atoms.

**3.** The anti-tumor agent according to claim 1 or 2, wherein ring $Q^2$ is

(1) phenyl which may be optionally substituted with the same or different 1 to 2 groups selected from the group consisting of $C_{1-6}$ alkoxy which may be optionally substituted with hydroxy, and $C_{1-6}$ alkyl-carbonylamino,
(2) a group of fromula (2):

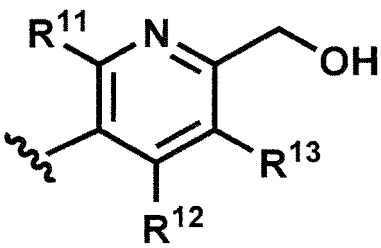
(2)

wherein $R^{11}$, $R^{12}$, and $R^{13}$ are independently

(a) hydrogen atom,
(b) halogen atom,
(c) $C_{1-6}$ alkyl which may be optionally substituted with 1 to 3 fluorine atoms, or
(d) amino which may be optionally substituted with the same or different 1 to 2 $C_{1-6}$ alkyl groups, or

(3) a group of formula (21):

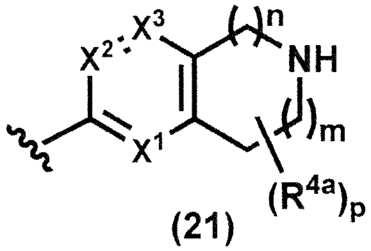

(21)

wherein $X^1$ is N or $CR^{14}$;
$X^2$ is N or $CR^{15}$;
$X^3$ is N or $CR^{16}$;
provided that $X^1$, $X^2$ and $X^3$ are not simultaneously N;
$R^{14}$, $R^{15}$, and $R^{16}$ are independently

(a) hydrogen atom,
(b) halogen atom,
(c) $C_{1-6}$ alkyl which may be optionally substituted with the same or different 1 to 3 halogen atoms, or
(d) $C_{1-6}$ alkoxy which may be optionally substituted with the same or different 1 to 3 halogen atoms;

n and m are independently 0, 1, or 2, provided that n and m are not simultaneously 0;
p is 1, 2, 3, 4, or 5;
$R^{4a}$ is, independently when two or more exist, hydrogen atom, halogen atom, or $C_{1-6}$ alkyl which may be optionally substituted with the same or different 1 to 3 halogen atoms.

**4.** The anti-tumor agent according to claim 3, wherein $R^{11}$ and $R^{12}$ are hydrogen atom;
$R^{13}$ is hydrogen atom, $C_{1-4}$ alkyl which may be optionally substituted with 1 to 3 fluorine atoms, or amino;
$R^{14}$, $R^{15}$, and $R^{16}$ are independently hydrogen atom or fluorine atom;
n is 1;
m is 0 or 1;
p is 1 or 2;
$R^{4a}$ is, independently when two or more exist, hydrogen atom or methyl.

**5.** The anti-tumor agent according to claim 3 or 4, wherein $W^2$-$Q^2$ is -NHC(O)-$Q^2$, or -C(O)NH-$Q^2$; and

**6.** The anti-tumor agent according to any one of claims 3-5, wherein $W^2$-$Q^2$ is -NHC(O)-$Q^2$; and
ring $Q^2$ is a group of formula (2).

**7.** The anti-tumor agent according to claim 1, wherein the compound of formula (1) or a pharmaceutically acceptable salt is selected from the following compounds:

(2E)-3-[4-(acetylamino)phenyl]-N-{1-[3-(trifluoromethyl)benzyl]-1H-imidazol-4-yl}prop-2-enamide,
(2E)-N-(1-(3-chlorobenzyl)-1H-imidazol-4-yl)-3-(pyridin-3-yl)prop-2-enamide,
N-[1-(3-chlorobenzyl)-1H-imidazol-4-yl]-3,4-dimethoxybenzamide,
N-[1-(3,4-difluorobenzyl)-1H-imidazol-4-yl]-3,4-dimethoxybenzamide,
N-[1-(2,4-difluorobenzyl)-1H-imidazol-4-yl]-3,4-dimethoxybenzamide,
3,4-dimethoxy-N-[1-(3,4,5-trifluorobenzyl)-1H-imidazol-4-yl]benzamide,
6-(hydroxymethyl)-N-{1-[3-(trifluoromethyl)benzyl]-1H-imidazol-4-yl}nicotinamide,

5-(difluoromethyl)-6-(hydroxymethyl)-N-{1-[3-(trifluoromethyl)benzyl]-1H-imidazol-4-yl}nicotinamide,
5-(difluoromethyl)-6-(hydroxymethyl)-N-[1-(3,4,5-trifluorobenzyl)-1H-imidazol-4-yl]nicotinamide,
N-(5,6,7,8-tetrahydro-2,7-naphthyridin-3-yl)-1-(3,4,5-trifluorobenzyl)-1H-imidazole-4-carboxamide,
8-fluoro-N-[1-(3,4,5-trifluorobenzyl)-1H-imidazol-4-yl]-1,2,3,4-tetrahydroquinoline-6-carboxamide,
N-[1-(3,4,5-trifluorobenzyl)-1H-imidazol-4-yl]-1,2,3,4-tetrahydroquinoline-6-carboxamide,
N-{1-[3-(trifluoromethyl)benzyl]-1H-imidazol-4-yl}-5,6,7,8-tetrahydro-1,6-naphthyridine-2-carboxamide,
N-[1-(3,4,5-trifluorobenzyl)-1H-imidazol-4-yl]-5,6,7,8-tetrahydro-1,6-naphthyridine-2-carboxamide,
N-[1-(3,4,5-trifluorobenzyl)-1H-imidazol-4-yL]-5,6,7,8-tetrahydro-1,7-naphthyridine-3-carboxamide,
N-{1-[3-(trifluoromethyl)benzyl]-1H-imidazol-4-yl}-5,6,7,8-tetrahydro-1,7-naphthyridine-3-carboxamide,
6-(hydroxymethyl)-5-methyl-N-[1-(3,4,5-trifluorobenzyl)-1H-imidazol-4-yl]nicotinamide,
5-amino-6-(hydroxymethyl)-N-{1-[3-(trifluoromethyl)benzyl]-1H-imidazol-4-yl}nicotinamide, and
5-amino-6-(hydroxymethyl)-N-[1-(3,4,5-trifluorobenzyl)-1H-imidazol-4-yl]nicotinamide.

**8.** The anti-tumor agent according to claim 1, wherein the compound of formula (1) or a pharmaceutically acceptable salt is selected from the following compounds:

(2E)-3-[4-(acetylamino)phenyl]-N-(1-[3-(trifluoromethyl)benzyl]-1H-imidazol-4-yl)prop-2-enamide,
N-[1-(3-chlorobenzyl)-1H-imidazol-4-yl]-3,4-dimethoxybenzamide,
3,4-dimethoxy-N-[1-(3,4,5-trifluorobenzyl)-1H-imidazol-4-yl]benzamide,
5-(difluoromethyl)-6-(hydroxymethyl)-N-[1-(3,4,5-trifluorobenzyl)-1H-imidazol-4-yl]pyridine-3-carboxamide,
and
N-{1-[3-(trifluoromethyl)benzyl]-1H-imidazol-4-yl}-5,6,7,8-tetrahydro-1,7-naphthyridine-3-carboxamide.

**9.** The anti-tumor agent according to any one of claims 1-8, wherein the anti-cancer agent is at least one selected from the group consisting of a chemotherapeutic agent, a hormonal therapeutic agent, an angiogenesis inhibitor, an immunotherapeutic agent, a kinase inhibitor, an antibody medicine, a proteasome inhibitor, a HDAC inhibitor, a PARP inhibitor, a thalidomide analog, a retinoic acid analog, and a pharmaceutically acceptable salt thereof.

**10.** The anti-tumor agent according to claim 9, wherein the chemotherapeutic agent is at least one selected from the group consisting of an alkylating agent, an anti-metabolite, an anticancer antibiotic, a microtubule inhibitor, a topoisomerase inhibitor, a platinum drug, and a pharmaceutically acceptable salt thereof.

**11.** The anti-tumor agent according to claim 9 or 10, wherein the chemotherapeutic agent is at least one selected from the group consisting of an alkylating agent and a pharmaceutically acceptable salt thereof.

**12.** The anti-tumor agent according to claim 9 or 10, wherein the chemotherapeutic agent is at least one selected from the group consisting of an anti-metabolite and a pharmaceutically acceptable salt thereof.

**13.** The anti-tumor agent according to claim 9 or 10, wherein the chemotherapeutic agent is at least one selected from the group consisting of an anti-cancer antibiotic and a pharmaceutically acceptable salt thereof.

**14.** The anti-tumor agent according to claim 9 or 10, wherein the chemotherapeutic agent is at least one selected from the group consisting of a microtubule inhibitor and a pharmaceutically acceptable salt thereof.

**15.** The anti-tumor agent according to claim 9 or 10, wherein the chemotherapeutic agent is at least one selected from the group consisting of a topoisomerase inhibitor and a pharmaceutically acceptable salt thereof.

**16.** The anti-tumor agent according to claim 9 or 10, wherein the chemotherapeutic agent is at least one selected from the group consisting of a platinum drug and a pharmaceutically acceptable salt thereof.

**17.** The anti-tumor agent according to any one of claims 9-16, wherein the hormonal therapeutic agent is at least one selected from the group consisting of an estrogen receptor modulator, an androgen receptor modulator, an LH-RH agonist, an LH-RH antagonist, an aromatase inhibitor, an androgen synthesis inhibitor, and a pharmaceutically acceptable salt thereof.

**18.** The anti-tumor agent according to any one of claims 9-17, wherein the hormonal therapeutic agent is at least one selected from the group consisting of an estrogen receptor modulator, an androgen receptor modulator, and a pharmaceutically acceptable salt thereof.

19. The anti-tumor agent according to any one of claims 9-16, wherein the anti-cancer agent is at least one selected from the group consisting of a chemotherapeutic agent and a pharmaceutically acceptable salt thereof.

20. The anti-tumor agent according to any one of claims 9 and 17-18, wherein the anti-cancer agent is at least one selected from the group consisting of a hormonal therapeutic agent and a pharmaceutically acceptable salt thereof.

21. The anti-tumor agent according to claim 9, wherein the anti-cancer agent is at least one selected from the group consisting of an angiogenesis inhibitor and a pharmaceutically acceptable salt thereof.

22. The anti-tumor agent according to claim 9, wherein the anti-cancer agent is at least one selected from the group consisting of an immunotherapeutic agent and a pharmaceutically acceptable salt thereof.

23. The anti-tumor agent according to claim 9, wherein the anti-cancer agent is at least one selected from the group consisting of a kinase inhibitor and a pharmaceutically acceptable salt thereof.

24. The anti-tumor agent according to claim 9, wherein the anti-cancer agent is at least one selected from the group consisting of an antibody medicine.

25. The anti-tumor agent according to any one of claims 1-24, wherein the anti-diabetic agent is at least one selected from the group consisting of a biguanide drug, a thiazolidine derivative, and a pharmaceutically acceptable salt thereof.

26. The anti-tumor agent according to any one of claims 1-25, wherein the agent for treating dyslipidemia is at least one selected from the group consisting of a HMG-CoA reductase inhibitor, a cholesterol absorption inhibitor, and a pharmaceutically acceptable salt thereof.

27. The anti-tumor agent according to any one of claim 1-24, wherein the agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof is an anti-cancer agent.

28. The anti-tumor agent according to any one of claims 1-8 and 25, wherein the agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof is an anti-diabetic agent.

29. The anti-tumor agent according to any one of claims 1-8 and 26, wherein the agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof is an agent for treating dyslipidemia.

30. An anti-tumor agent comprising the compound of formula (1) or a pharmaceutically acceptable salt thereof according to any one of claims 1-8 as an active ingredient, wherein the anti-tumor agent is administered in combination with at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof.

31. A preparation comprising the compound of formula (1) according to any one of claims 1-8 or a pharmaceutically acceptable salt thereof, and at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof, as a combination preparation for administering the compound and the agent simultaneously, separately, or with time-interval in a cancer therapy.

32. Use of the compound of formula (1) or a pharmaceutically acceptable salt thereof according to any one of claims 1-8 for the manufacture of a medicament for treating cancer in combination with at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof.

**33.** A method for treating cancer which comprises administering a therapeutically effective amount of a combination of the compound of formula (1) according to any one of claims 1-8 or a pharmaceutically acceptable salt thereof and at least one agent selected from the group consisting of an anti-cancer agent, an anti-diabetic agent, an agent for treating dyslipidemia, an agent for treating multiple sclerosis, a steroidal anti-inflammatory agent, a non-steroidal anti-inflammatory agent, an anti-fungal agent, and a pharmaceutically acceptable salt thereof to a patient in need thereof.

**34.** A medicament for treating a tumor with gene mutation in Wnt/β-catenin pathway, comprising the compound of formula (1) according to any one of claims 1-8 or a pharmaceutically acceptable salt thereof.

[Fig. 1-1]

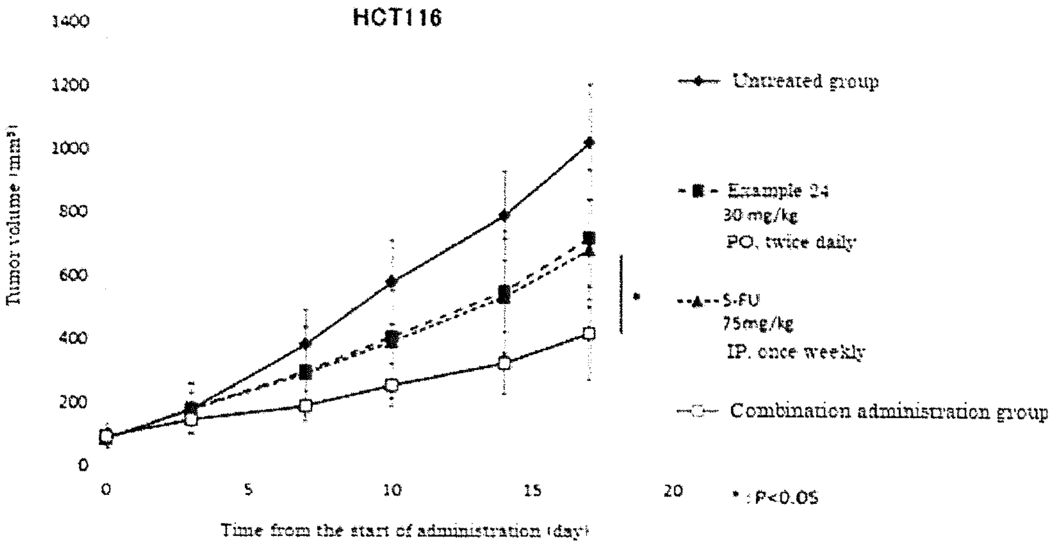
HCT116
Tumor volume (mm³)
1400
1200
1000
800
600
400
200
0
0
5
10
15
20
Time from the start of administration (day)
Untreated group
Example 24
30 mg/kg
PO, twice daily
5-FU
75mg/kg
IP, once weekly
Combination administration group
*
*: P<0.05

[Fig. 1-2]

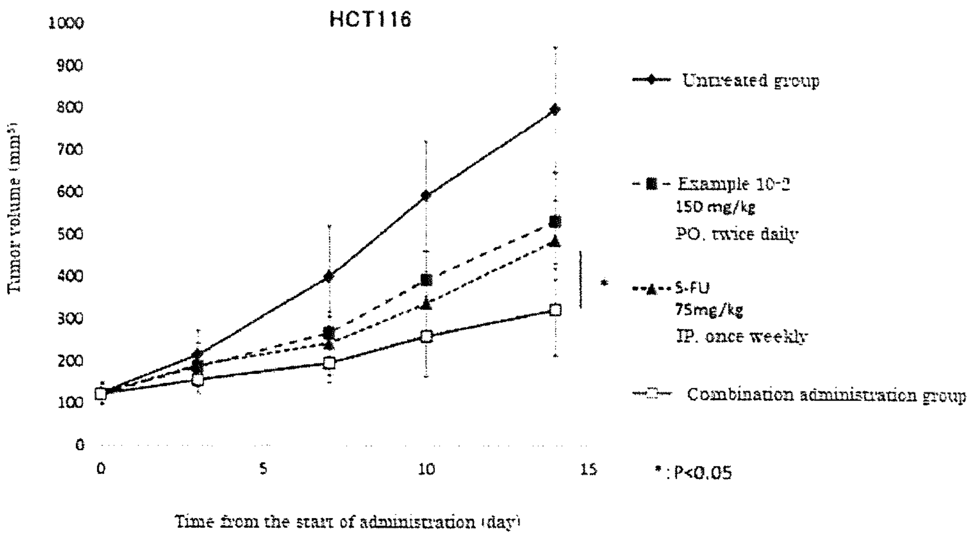
HCT116
Tumor volume (mm³)
1000
900
800
700
600
500
400
300
200
100
0
0
5
10
15
Time from the start of administration (day)
Untreated group
Example 10-2
150 mg/kg
PO, twice daily
5-FU
75mg/kg
IP, once weekly
Combination administration group
*
*: P<0.05

[Fig. 1-3]

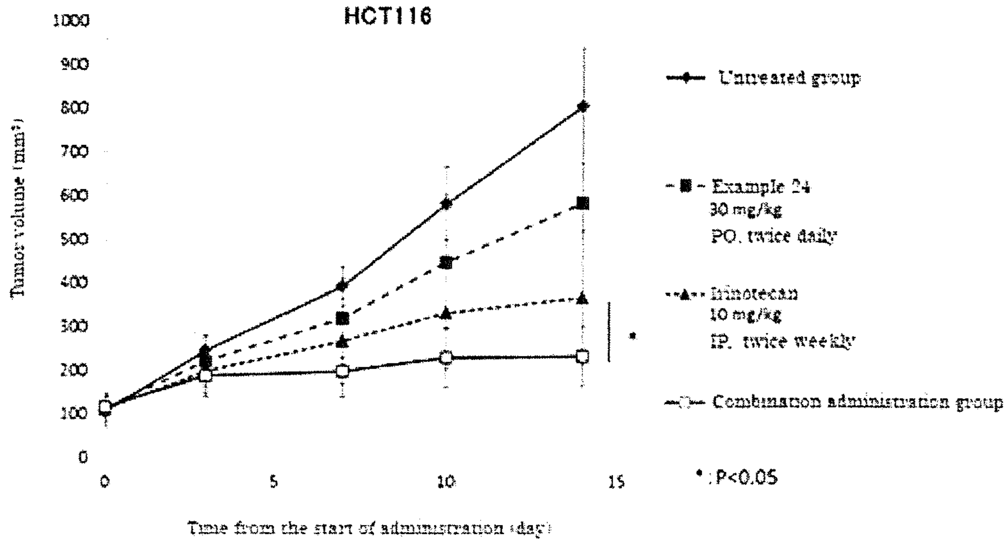
HCT116
Tumor volume (mm³)
1000
900
800
700
600
500
400
300
200
100
0
0
5
10
15
Time from the start of administration (day)
Untreated group
Example 24
30 mg/kg
PO. twice daily
Irinotecan
10 mg/kg
IP. twice weekly
Combination administration group
*
*: P<0.05

[Fig. 1-4]

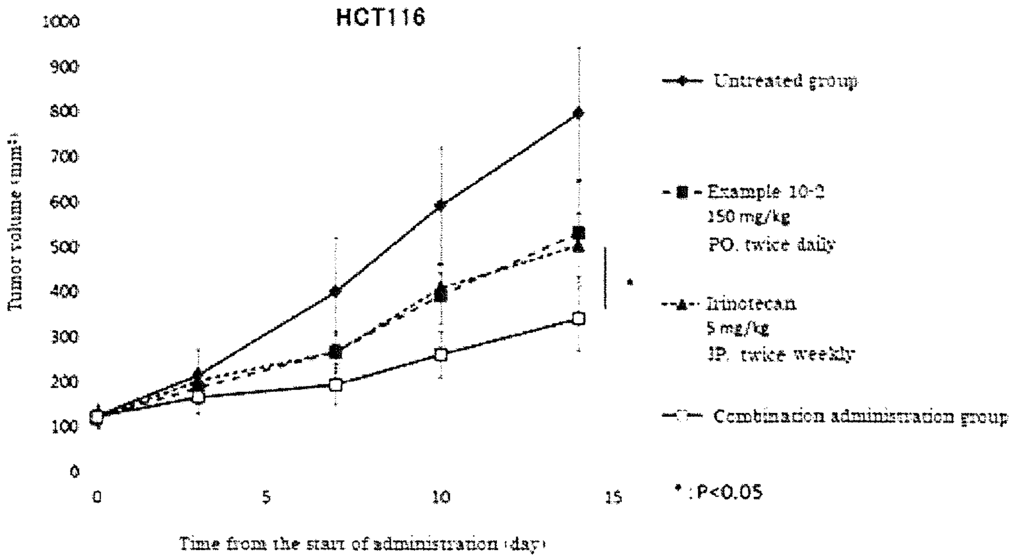
HCT116
Tumor volume (mm³)
1000
900
800
700
600
500
400
300
200
100
0
0
5
10
15
Time from the start of administration (day)
Untreated group
Example 10-2
150 mg/kg
PO. twice daily
Irinotecan
5 mg/kg
IP. twice weekly
Combination administration group
*
*: P<0.05

[Fig. 1-5]

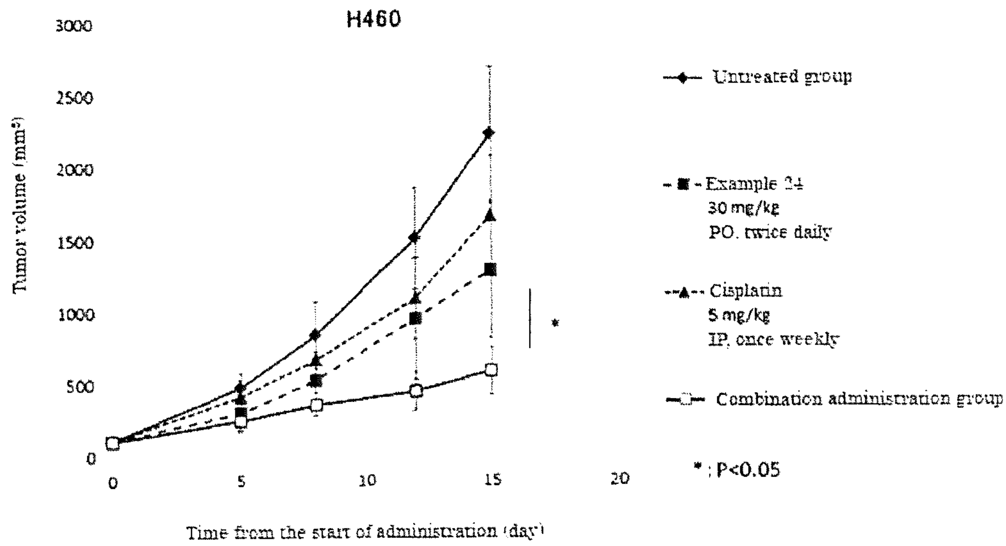
H460
3000
2500
2000
1500
1000
500
0
Tumor volume (mm³)
0
5
10
15
20
Time from the start of administration (day)
Untreated group
Example 24
30 mg/kg
PO, twice daily
Cisplatin
5 mg/kg
IP, once weekly
Combination administration group
*
*: P<0.05

[Fig. 1-6]

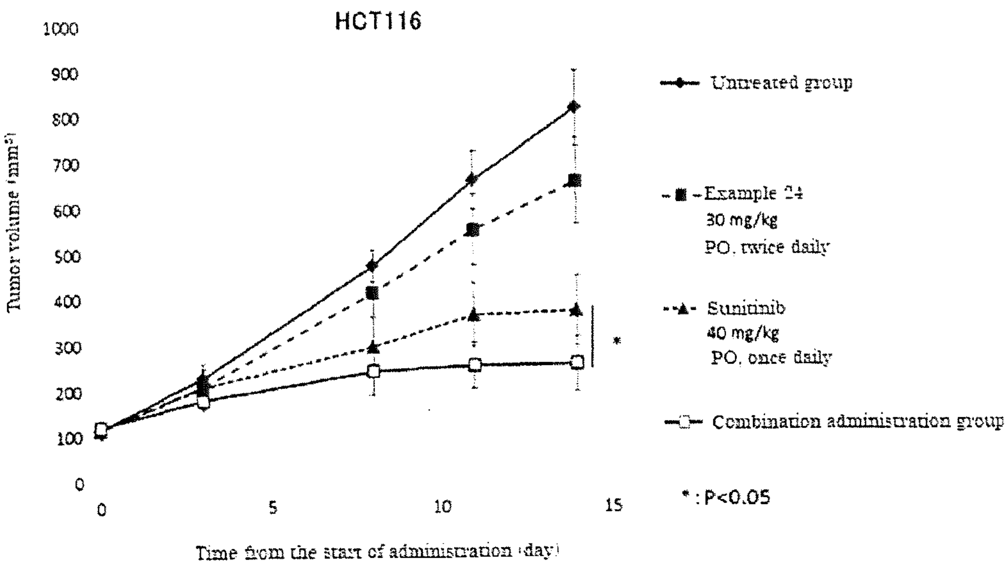
HCT116
1000
900
800
700
600
500
400
300
200
100
0
Tumor volume (mm³)
0
5
10
15
Time from the start of administration (day)
Untreated group
Example 24
30 mg/kg
PO, twice daily
Sunitinib
40 mg/kg
PO, once daily
Combination administration group
*
*: P<0.05

[Fig. 1-7]

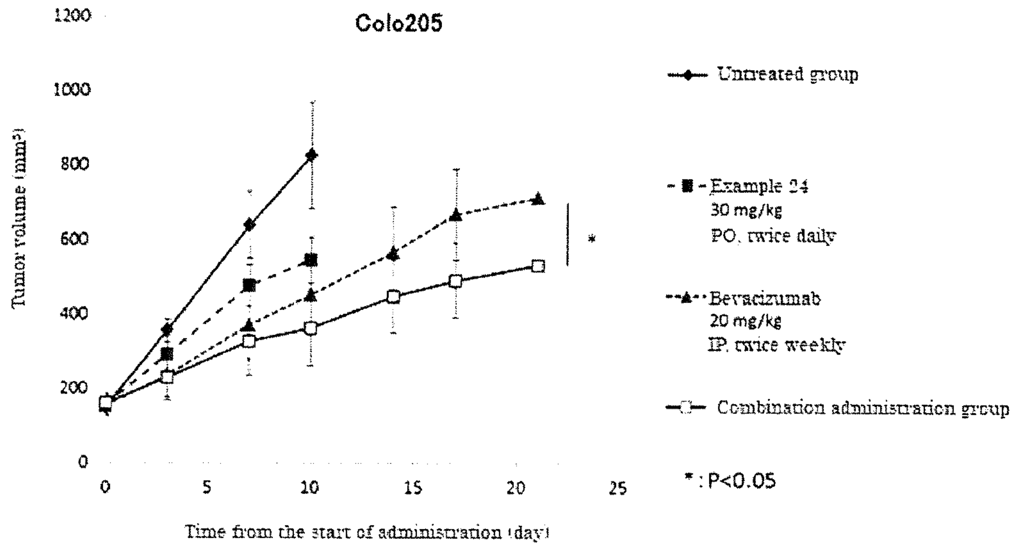
Colo205
1200
1000
800
600
400
200
0
Tumor volume (mm³)
0
5
10
15
20
25
Time from the start of administration (day)
Untreated group
Example 24
30 mg/kg
PO. twice daily
Bevacizumab
20 mg/kg
IP. twice weekly
Combination administration group
*
*: P<0.05

[Fig. 1-8]

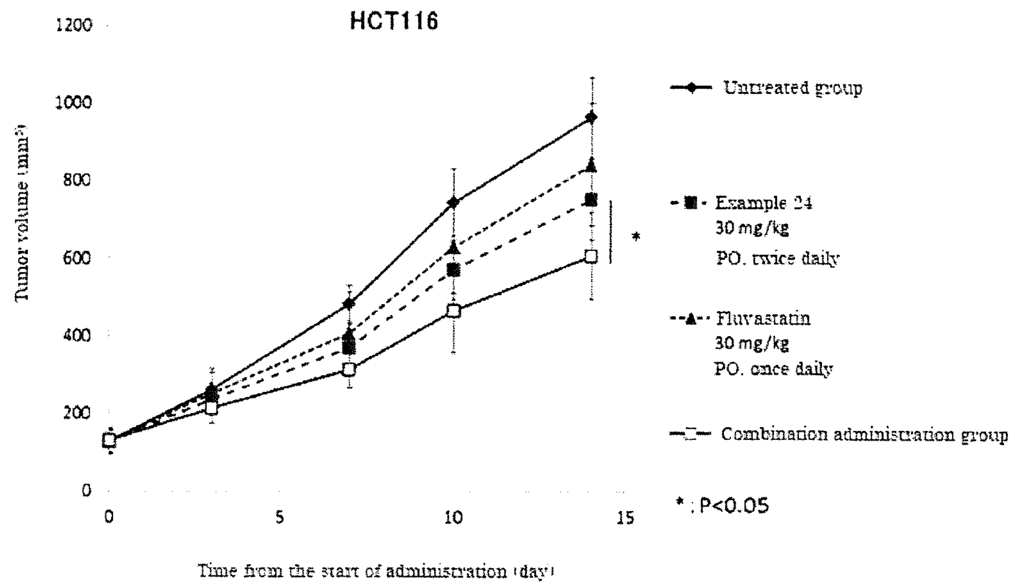
HCT116
1200
1000
800
600
400
200
0
Tumor volume (mm³)
0
5
10
15
Time from the start of administration (day)
Untreated group
Example 24
30 mg/kg
PO. twice daily
Fluvastatin
30 mg/kg
PO. once daily
Combination administration group
*
*: P<0.05

[Fig. 1-9]

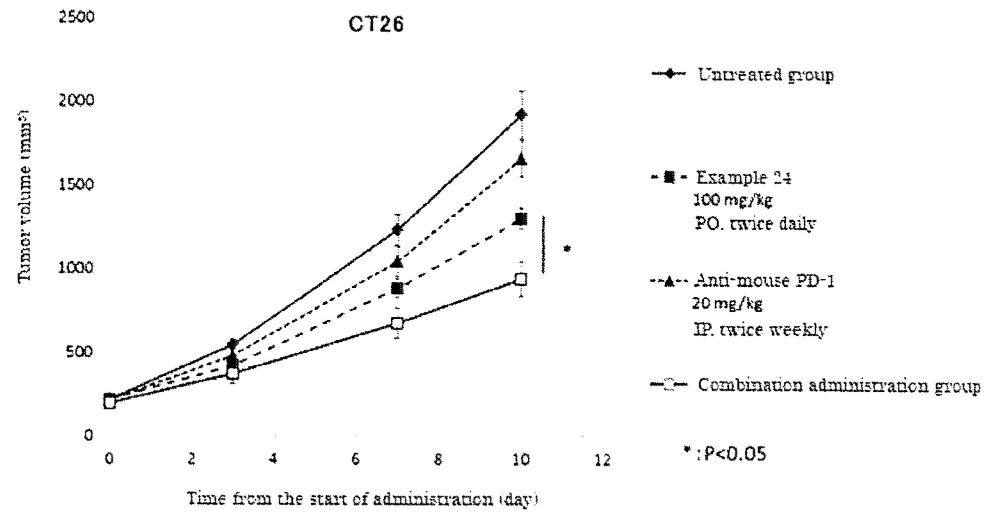


[Fig. 1-10]

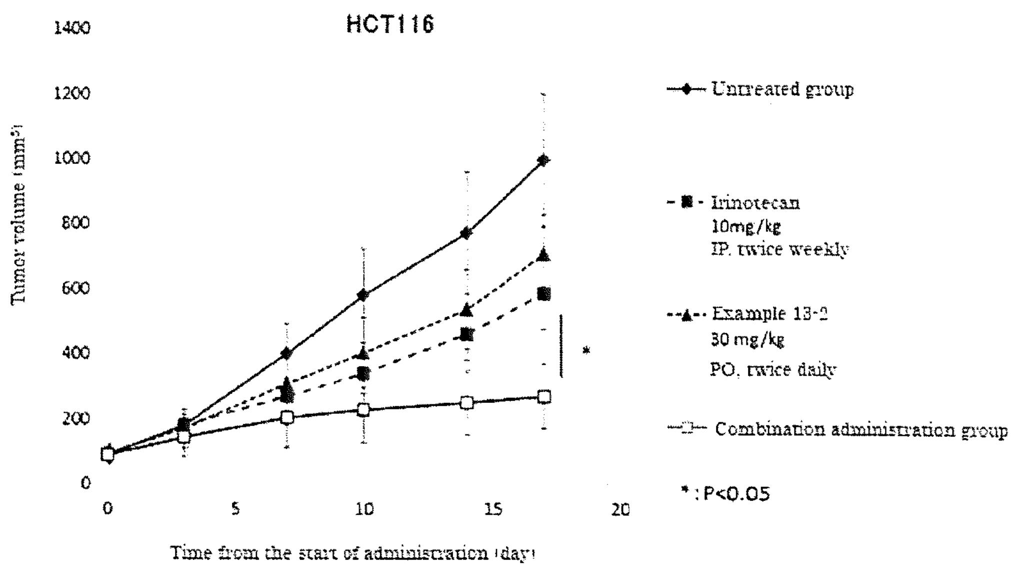

[Fig. 1-11]

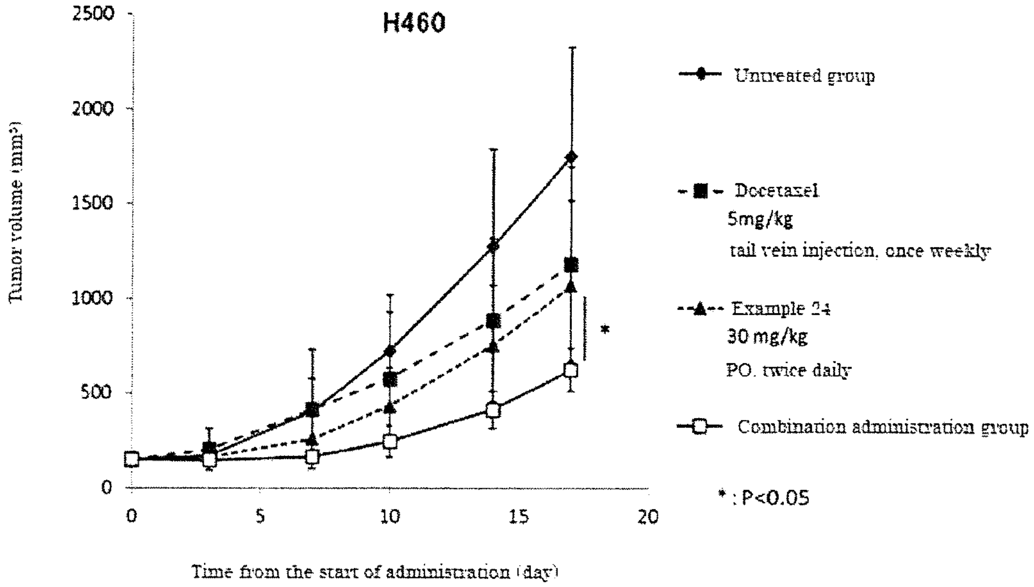


[Fig. 1-12]

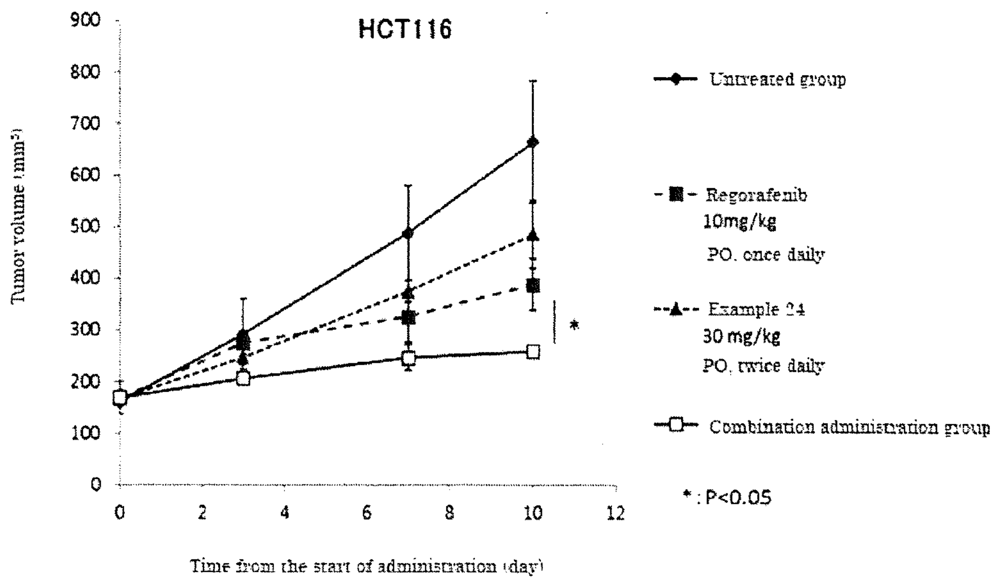

[Fig. 1-13]

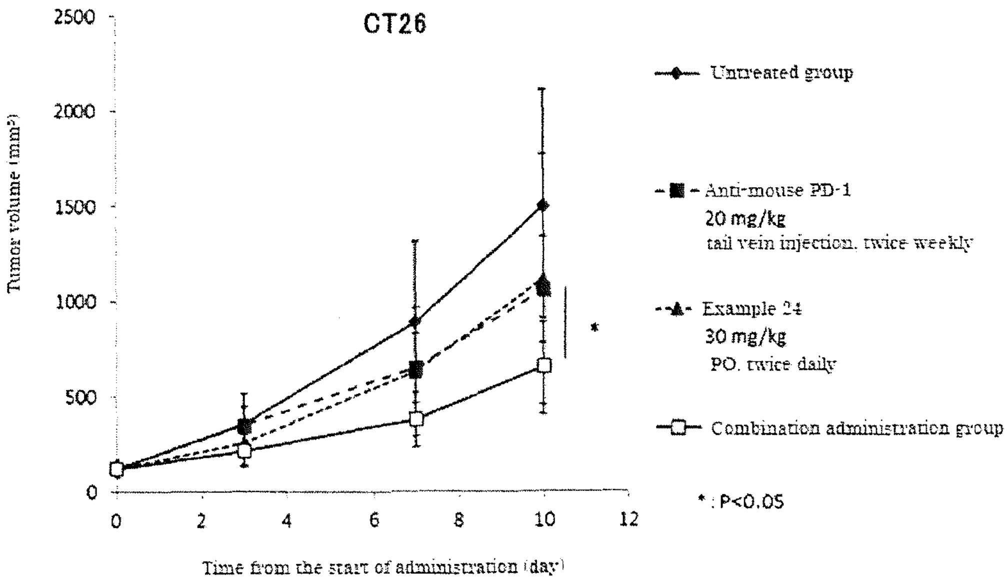
CT26
2500
2000
1500
1000
500
0
Tumor volume (mm³)
0
2
4
6
8
10
12
Time from the start of administration (day)
*
Untreated group
Anti-mouse PD-1
20 mg/kg
tail vein injection, twice weekly
Example 24
30 mg/kg
PO, twice daily
Combination administration group
*: P<0.05

**INTERNATIONAL SEARCH REPORT**

International application No.
PCT/JP2017/045836

A. CLASSIFICATION OF SUBJECT MATTER
See extra sheet
According to International Patent Classification (IPC) or to both national classification and IPC

B. FIELDS SEARCHED
Minimum documentation searched (classification system followed by classification symbols)
Int.Cl. A61K31/4164, A61K31/4178, A61K31/422, A61K31/427, A61K31/437, A61K31/4375, A61K31/4439, A61K31/4709, A61K31/4725, A61K31/497, A61K31/5365, A61K45/00, A61P35/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| A | WO 2015/178426 A1 (NATIONAL INSTITUTE OF ADVANCED INDUSTRIAL SCIENCE AND TECHNOLOGY) 26 November 2015 & US 2017/0079942 A1 & EP 3146978 A4 | 1-34 |
| A | WO 2016/027253 A1 (GLAXOSMITHKLINE INTELLECTUAL PROPERTY DEVELOPMENT LIMITED) 25 February 2016 & JP 2017-524028 A & US 2017/0266199 A1 & EP 3182974 A1 | 1-34 |
| P, A | WO 2017/146128 A1 (SUMITOMO DAINIPPON PHARMA CO., LTD.) 31 August 2017 (Family: none) | 1-34 |
| P, A | WO 2016/208591 A1 (SUMITOMO DAINIPPON PHARMA CO., LTD.) 29 December 2016 & US 2017/0166552 A1 | 1-34 |
| P, A | WO 2016/208592 A1 (SUMITOMO DAINIPPON PHARMA CO., LTD.) 29 December 2016 (Family: none) | 1-34 |

☐ Further documents are listed in the continuation of Box C.
☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 March 2018 (07.03.2018) | 20 March 2018 (20.03.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2017/045836

CLASSIFICATION OF SUBJECT MATTER
A61K31/4164(2006.01)i, A61K31/4178(2006.01)i, A61K31/422(2006.01)i, A61K31/427(2006.01)i, A61K31/437(2006.01)i, A61K31/4375(2006.01)i, A61K31/4439(2006.01)i, A61K31/4709(2006.01)i, A61K31/4725(2006.01)i, A61K31/497(2006.01)i, A61K31/5365(2006.01)i, A61K45/00(2006.01)i, A61P35/00(2006.01)i

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014125444 A
- WO 2009056556 A
- WO 2006065215 A

### Non-patent literature cited in the description


- **BOMAN et al.** *Journal of Clinical Oncology,* 2008, vol. 26 (17), 2795-2799
- **LOBO et al.** *Annu Rev Cell Dev Biol,* 2007, vol. 23, 675-99
- **AL-HAJJ et al.** *Oncogene,* 2004, vol. 23 (43), 7274-82
- **PONTI et al.** *Cancer Res,* 2005, vol. 65 (13), 5506-11
- **CARMERO et al.** *Cancer Treatment reviews,* 2016, vol. 49, 25-36
- **ZHANG et al.** *Stem Cells Translational Medicine,* 2013, vol. 2, 233-242
- *The 27th medicinal chemistry symposium abstract,* 166-167
- *Monthly Fine Chemicals,* August 2009, vol. 38 (8), 12-24
- **T. W. GREENE ; P. G. M. WUTS.** Protective Groups in Organic Synthesis. John Wiley and Sons, inc, 1999
- Protective Groups in Organic Synthesis. John Wiley & Sons, Inc,
- **R. C. LAROCK.** Comprehensive Organic Transformation. VCH publisher Inc, 1989
- Protective Groups in Organic Synthesis. John Wiley & Sons, Inc
- **R. C. LAROCK et al.** Comprehensive Organic Transformation. VCH publisher Inc, 1989
- *Cancer Res,* 2005, vol. 65, 5506-5511
- Cancer Cell Line Encyclopedia
- *Proc. Natl. Acad. Sci. USA,* 1997, vol. 94, 10330-10334